# EUROPEAN PATENT SPECIFICATION

(11) **EP 0 715 618 B1**
(45) Date of publication and mention of the grant of the patent: **16.12.1998**
(21) Application number: 94927162.1
(22) Date of filing: 23.08.1994
(51) Int. Cl.: C07C 311/29, C07D 213/30, C07C 317/14, C07C 311/18, C07D 307/79, C07K 5/062, A61K 31/18, A61K 31/44

(54) **HYDROXYETHYLAMINO SULPHONAMIDES USEFUL AS RETROVIRAL PROTEASE INHIBITORS**
HYDROXYAMINOSULFONAMIDE VERWENDBAR ALS INHIBITOREN RETROVIRALER PROTEASEN
HYDROXYETHYLAMINO-SULFONAMIDES APTES A ETRE UTILISES COMME INHIBITEURS DE PROTEASE RETROVIRALE

(30) Priority: 24.08.1993 US 110911; 02.03.1994 US 204827
(43) Date of publication of application: 12.06.1996
(73) Proprietor: G.D. SEARLE & CO., Chicago IL 60680-5110 (US); THE MONSANTO COMPANY, St. Louis, MO 63166 (US)
(72) Inventor: VAZQUEZ, Michael, L., Gurnee, IL 60031 (US); MUELLER, Richard, A., Glencoe, IL 60022 (US); TALLEY, John, J., Brentwood, MO 63144 (US); GETMAN, Daniel, P., Chesterfield, MO 63017 (US); DECRESCENZO, Gary, A., St. Peters, MO 63376 (US); FRESKOS, John, N., Clayton, MO 63105 (US); BERTENSHAW, Deborah, E., Brentwood, MO 63144 (US); HEINTZ, Robert, M., Ballwin, MO 63021 (US)
(74) Representative: Beil, Hans Christoph, Dr.
(86) International application number: US9409139
(87) International publication number: WO9506030

(56) References cited:
- EP-A- 0 264 795
- EP-A- 0 468 641
- WO-A-94/04492

## Description

The present invention relates to retroviral protease inhibitors and, more particularly, relates to novel compounds and a composition and method for inhibiting retroviral proteases. This invention, in particular, relates to sulfonamide-containing hydroxyethylamine protease inhibitor compounds, a composition and the use thereof for preparing medicaments for inhibiting retroviral hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds.

### Related Art

During the replication cycle of retroviruses, gag and gag-pol gene transcription products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse cranscriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. It has also been shown through site-directed mutagenesis of an aspartic acid residue in the HIV protease active site that processing of the gag precursor protein is prevented. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit specific processing of structural proteins and the release of retroviral protease itself. In this manner, retroviral replication proteases can be effectively inhibited.

Several classes of compounds have been proposed, particularly for inhibition of proteases, such as for inhibition of HIV protease. Such compounds include hydroxyethylamine isosteres and reduced amide isosteres. See, for example, EP O 346 847; EP O 342,541; Roberts et al, "Rational Design of Peptide-Based Proteinase Inhibitors, "Science, 248, 358 (1990); and Erickson et al, "Design Activity, and 2.8Å Crystal Structure of a C₂ Symmetric Inhibitor Complexed to HIV-1 Protease," Science, 249, 527 (1990).

Several classes of compounds are known to be useful as inhibitors of the proteolytic enzyme renin. See, for example, U.S. No. 4,599,198; U.K. 2,184,730; G.B. 2,209,752; EP O 264 795; G.B. 2,200,115 and U.S. SIR H725. of these, G.B. 2,200,115, GB 2,209,752, EP O 264,795, U.S. SIR H725 and U.S. 4,599,198 disclose urea-containing hydroxyethylamine renin inhibitors. EP 468 641 discloses renin inhibitors and intermediates for the preparation of the inhibitors, which include sulfonamide-containing hydroxyethylamine compounds, such as 3-(t-butoxycarbonyl)amino-cyclohexyl-1-(phenylsulfonyl)amino-2(5)-butanol. G.B. 2,200,115 also discloses sulfamoyl-containing hydroxyethylamine renin inhibitors, and EP 0264 795 discloses certain sulfonamide-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and compositions, to the use thereof for preparing medicaments for inhibiting retroviral proteases, the processes for preparing the compounds. The subject compounds are characterized as sulfon-amide-containing hydroxyethylamine inhibitor compounds.

### Detailed description of the invention

In accorance with the present invention there is provided a retroviral protease inhibiting compound of the formula I: 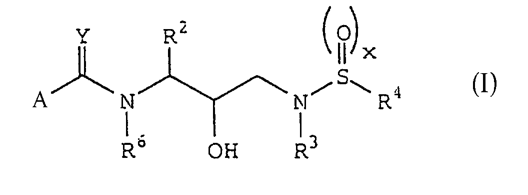 or a pharmaceutically acceptable salt, prodrug or ester thereof, wherein
R² is an alkyl, aryl, cycloalkyl, cycloalkylalkyl or aralkyl radical, which radical is optionally substituted with a radical selected from the group consisting of alkyl, halo, nitro, cyano, CF₃, -OR⁹, and -SR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or disubstituted aminoalkyl radicals, wherein said substituents are selected from the group consisting of alkyl, aryl, aralkyl, cycloalkyl, cycloalkylalkyl, heteroaryl, heteroaralkyl, heterocycloalkyl and heterocycloalkylalkyl radicals; or where said aminoalkyl radical is disubstituted, said substituents along with the nitrogen atom to which they are attached, form a heterocycloalkyl or a heteroaryl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl, heteroaralkyl, aminoalkyl or mono- or disubstituted aminoalkyl radical, wherein said substituents are selected from the group consisting of alkyl, aryl, aralkyl, cycloalkyl, cycloalkylalkyl, heteroaryl, heteroaralkyl, heterocycloalkyl and heterocycloalkylalkyl radicals; or where said aminoalkyl radical is disubstituted, said substituents along with the nitrogen atom to which they are attached, form a heterocycloalkyl or a heteroaryl radical;
R⁶ is a hydrogen or alkyl radical;
x is 1 or 2;
Y is O or S; and
A is an alkoxy, alkenoxy, aralkoxy, alkyl, cycloalkyl, cycloalkylalkoxy, cycloalkylalkyl, aralkyl, aryl, aryloxy, heterocycloalkyl, heterocycloalkoxy, heterocycloalkylalkyl, heterocycloalkylalkoxy, heteroaralkyl, heteroaralkoxy, heteroaryloxy, heteroaryl, alkenyl, aryloxyalkyl, heteroaryloxyalkyl, hydroxyalkyl, amino, or mono- or disubstituted amino radical, wherein the substituents are selected from the group consisting of alkyl, aryl, aralkyl, cycloalkyl, cycloalkylalkyl, heteroaryl, heteroaralkyl, heterocycloalkyl and heterocycloalkylalkyl radicals; or where said amino radical is disubstituted, said substituents along with the nitrogen atom to which they are attached form a heterocycloalkyl or heteroaryl radical, except the compounds of the formula 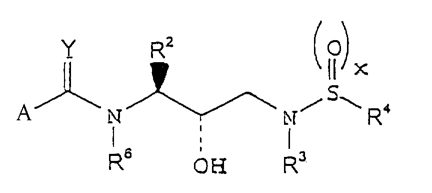 wherein

R³ and R⁶ are both hydrogen
R² is cyclohexylmethyl
x is 2
Y is oxygen
R⁴ is C₁-C₁₂-alkyl, alkylamino, alkylaminoalkyl, heteroaryl, heterocycloalkyl, C₃-C₁₀-cycloalkyl, aryl, C₂-C₆-alkenyl, C₂-C₆-alkynyl,
A is alkoxy, aralkoxy, aryloxy, cycloalkylalkoxy,

Such excluded compounds are disclosed in the EPA 0 468 641 and are said to be useful as renin inhibitors.

Preferred compounds are those of the above formula or a pharma-ceutically acceptable salt, prodrug or ester thereof, wherein
R² is an alkyl, aryl, cycloalkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or disubstituted aminoalkyl radicals, wherein said substituents are selected from the group consisting of alkyl, aralkyl, cycloalkyl and cycloalkylalkyl radicals; or where said aminoalkyl radical is disubstituted, said substituents along with the nitrogen atom to which they are attached, form a heterocycloalkyl or a heteroaryl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, cycloalkylalkoxy, cycloalkylalkyl, aralkyl, aryl, aryloxy, heterocycloalkyl, heterocycloalkoxy, heterocycloalkylalkyl, heterocycloalkylalkoxy, heteroaralkyl, heteroaralkoxy, heteroaryloxy, heteroaryl, hydroxyalkyl, amino, or mono- or disubstituted amino radical, wherein the substituents are selected from the group consisting of alkyl, aralkyl, heteroaryl, heteroaralkyl, heterocycloalkyl and heterocycloalkyalkyl radicals; or where said amino radical is disubstituted, said substituents along with the nitrogen atom to which they are attached form a heterocycloalkyl radical.

Further preferred are compounds of the above formula wherein
R² is an alkyl, aryl, cycloalkyl, cycloalkylalkyl or aralkyl radical, which radical is optionally substituted with a'radical selected from the group consisting of alkyl, halo and -OR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals.

Another group of preferred compounds are those wherein
R² is an alkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, alkenyl, alkynyl, hydroxyalkyl, alkoxyalkyl, alkylthioalkyl, alkylsulfonylalkyl, cycloalkyl, cycloalkylalkyl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals;
with the proviso that alkyl, alone or in combination, is a straight-chain or branched-chain hydrocarbon radical containing from one to eight carbon atoms; alkenyl, alone or in combination, is a straight-chain or branched-chain hydrocarbon radical having at least one double bond and containing from two to eight carbon atoms; alkynyl, alone or in combination, is a straight-chain or branched-chain hydrocarbon radical having at least one triple bond and containing from two to ten carbon atoms; and cycloalkyl, alone or in combination, is a hydrocarbon ring containing from three to eight carbon atoms.

More preferred are compounds wherein
R² is an alkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, alkenyl, alkynyl, hydroxyalkyl, alkoxyalkyl, alkylthioalkyl, alkylsulfonylalkyl, cycloalkyl, cycloalkylalkyl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals;
with the proviso that alkyl, alone or in combination, is a straight-chain or branched-chain hydrocarbon radical containing from one to five carbon atoms; alkenyl, alone or in combination, is a straight-chain or branched-chain hydrocarbon radical having at least one double bond and containing from two to five carbon atoms; alkynyl, alone or in combination, is a straight-chain or branched-chain hydrocarbon radical having at least one triple bond and containing from two to five carbon atoms; and cycloalkyl, alone or in combination, is a hydrocarbon ring containing from three to eight carbon atoms.

Especially preferred are compounds wherein
R² is butyl, cyclohexylmethyl, benzyl, 4-fluorobenzyl or naphthylmethyl;
R³ is methyl, ethyl, propyl, butyl, pentyl, hexyl, iso-butyl, iso-amyl, 3-methoxypropyl, 3-methylthiopropyl, 4-methylthiobutyl, 4-methylsulfonylbutyl, 2-dimethylaminoethyl, 2-(1-morpholino)ethyl, 4-hydroxybutyl, allyl, propargyl, cyclohexylmethyl, cyclopropylmethyl, phenyl, benzyl, 4-fluorobenzyl, 4-methoxybenzyl, 1-phenylethyl, 2-phenylethyl, naphthylmethyl, 3-pyridylmethyl or 4-pyridylmethyl;
R⁴ is methyl, ethyl, propyl, butyl, ethenyl, chloromethyl, cyclopropyl, cyclobutyl, cyclopentyl, cyclohexyl, phenyl, naphthyl, chlorophenyl, fluorophenyl, hydroxyphenyl, methylphenyl, methoxyphenyl, ethoxyphenyl, methylthiophenyl, methylsulfoxyphenyl, methylsulfonylphenyl, acetamidophenyl, methoxycarbonylphenyl, dimethylaminophenyl, nitrophenyl, trifluoromethylphenyl, benzyl, 2-phenylethenyl or thienyl;
R⁶ is hydrogen;
x is 2; and
Y is O; and
A is methyl, cyclohexyl, cyclopentyl, cycloheptyl, 1,2,3,4-tetrahydronaphthyl, naphthyl, quinolinyl, indolyl, pyridyl, methylpyridyl, furanyl, thiophenyl, oxazolyl, thiazolyl, phenyl, methylphenyl, ethylphenyl, dimethylphenyl, iso-propylphenyl, chlorophenyl, hydroxyphenyl, methoxyphenyl, methylsulfonylphenyl, methylsulfonylmethylphenyl, carboxyphenyl, aminocarbonylphenyl, methylhydroxyphenyl, methylnitrophenyl, methylaminophenyl, methyl-N,N-dimethylaminophenyl, t-butoxy, benzyloxy, pyridylmethoxy, 3-propenoxy, hydroxypyridylmethoxy, aminopyridylmethoxy, pyrimidinylmethoxy, N-oxo-pyrimidinylmethoxy, thiazolylmethoxy, tetrahydrothiophenoxy, 1,1-dioxotetrahydrothiophenoxy, tetrahydrofuranoxy, methylamino, benzylamino or isopropylamino,

Specific compounds according to the present invention are the following ones:
Phenylmethyl[2R-hydroxy-3-[(3-methylbutyl) (methylsulfonyl)amino]-1S-(phenylmethyl)propyl]carbamate;
Phenylmethyl[2R-hydroxy-3-[(3-methylbutyl) (phenylsulfonyl)amino]-1S-(phenylmethyl)propyl]carbamate;
N1-[2R-hydroxy-3[(3-methylbutyl)(phenylsulfonyl)amino]-1S-(phenylmethyl)propyl]-2S-[(phenylmethyloxycarbonyl) amino]butanediamide;
2S-[[(dimethylamino)acetyl]amino]-N-[2R-hydroxy-3-[(3-methyl- butyl)(phenylsulfonyl)amino]-1S-(phenylmethyl) propyl]-3,3-dimethylbutaneamide;
2S-[[(methylamino)acetyl]amino]-N-[2R-hydroxy-3-[(3-methyl-butyl)(phenylsulfonyl)amino)-1S-(phenylmethyl) propyl]-3,3-dimethylbutaneamide;
N1-[2R-hydroxy-3-[(3-methylbutyl)(phenyl-sulfonyl)amino]-N4-methyl-1S-(phenylmethyl)propyl]-2S-[(2-quinolinylcarbonyl)amino]butanediamide;
[3-[[2-hydroxy-3-[N-(3-methylbutyl)-N-(phenylsufonyl)amino]-1-(phenylmethyl)propyl]amino]-2-methyl-3-oxopropyl]-, (4-methoxyphenyl)methyl ester, [1S-[1R*(S*),2S*]]-;
Carbamic acid, [2R-hydroxy-3-[(4-hydroxyphenylsulfonyl) (2-methylpropyl)amino]-1S-(phenylmethyl)propyl-, 3-(S)-1,1-dioxotetrahydrothiophen-3-yl-ester;
Carbamic acid, [2R-hydroxy-3-[(4-methoxyphenylsulfonyl) (2-methylpropyl)amino]-1S-(phenylmethyl)propyl-, 3-(S)-1,1-dioxotetrahydrothiophen-3-yl-ester;
Carbamic acid, [2R-hydroxy-3-[(4-methoxyphenylsulfonyl) (2-methylpropyl)amino] -1S-(phenylmethyl)propyl-, 3-S-tetrahydrothiophen-3-yl-ester;
Carbamic acid, [2R-hydroxy-3-[(4-hydroxyphenylsulfonyl) (2-methylpropyl)amino]-1S-(phenylmethyl)propyl-, 3-S-tetrahydrothiophen-3-yl-ester;
Carbamic acid, [2R-hydroxy-3-[[(4-methoxyphenyl)sulfonyl](2-methylpropyl)amino]-1S-(phenylmethyl)propyl]-, 3-(6-aminopyridyl)methyl ester;
Carbamic acid, [2R-hydroxy-3-[[(4-hydroxyphenyl)sulfonyl](2-methylpropyl)amino]-1S-(phenylmethyl)propyl]-, 3-(6-aminopyridyl)methyl ester;
Carbamic acid, [2R-hydroxy-3-[[(4-methoxyphenyl)sulfonyl] (2-methylpropyl)amino]-1S-(phenylmethyl)propyl]-, 3-(6-hydroxypyridyl)methyl ester;
Carbamic acid, [2R-hydroxy-3-[[(4-hydroxyphenyl)sulfonyl] (2-methylpropyl)amino]-1S-(phenylmethyl)propyl]-, 5-pyrimidylmethyl ester; or
Benzamide, N-[2R-hydroxy-3-[[(4-methoxyphenyl)sulfonyl] (2-methylpropyl)amino]-1S-(phenylmethyl)propyl]-2-methyl.

As utilized herein, the term "alkyl", alone or in combination, means a straight-chain or branched-chain alkyl radical containing from 1 to about 10 carbon atoms, preferably from 1 to about 8 carbon atoms, more preferably 1-5 carbon atoms. Examples of such radicals include methyl, ethyl, n-propyl, isopropyl n-butyl, isobutyl, set-butyl, tert-butyl, pentyl, iso-amyl, hexyl, octyl and the like. The term "alkenyl", alone or in combination, means a straight-chain or branched-chain hydrocarbon radial having one or more double bonds and containing from 2 to about 18 carbon atoms, preferably from 2 to about 8 carbon atoms, more preferably from 2 to about 5 carbon atoms. Examples of suitable alkenyl radicals include ethenyl, propenyl, alkyl, 1,4-butadienyl and the like. The term "alkynyl", alone or in combination, means a straight-chain or branched chain hydrocarbon radical having one or more triple bonds and containing from 2 to about 10 carbon atoms, more preferably from 2 to about 5 carbon atoms. Examples of alkynyl radicals include ethynyl, propynyl, (propargyl), butynyl and the like. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy and the like. The term "cycloalkyl", alone or in combination, means a saturated or partially saturated monocyclic, bicyclic or tricyclic alkyl radical wherein each cyclic moiety contains from about 3 to about 8 carbon atoms, more preferably from about 3 to about 6 carbon atoms. Examples of such cycloalkyl radicals include cyclopropyl, cyclobutyl, cyclopentyl, cyclohexyl and the like. The term "cycloalkylalkyl" means an alkyl radical as defined above which is substituted by a cycloalkyl radical as defined above. Examples of such cycloalkylalkyl radicals include cyclopropylmethyl, cyclobutylmethyl, cyclopentylmethyl, cyclohexylmethyl, 1-cyclopentylethyl, 1-cyclohexylethyl, 2-cyclopentylethyl, 2-cyclohexylethyl, cyclobutylpropyl, cyclopentylpropyl, cyclohexylbutyl and the like. The term "aryl", alone or in combination, means a phenyl or naphthyl radical which optionally carries one or more substituents selected from alkyl, alkoxy, halogen, hydroxy, amino, nitro, cyano, haloalkyl, carboxy, alkoxycarbonyl, cycloalkyl, heterocycloalkyl, amido, mono and dialkyl substituted amino, mono and dialkyl substituted amido and the like, such as phenyl, p-tolyl, 4-methoxyphenyl, 4-(tert-butoxy)phenyl, 3-methyl-4-methoxyphenyl, 4-fluorophenyl, 4-chlorophenyl, 3-nitrophenyl, 3-aminophenyl, 3-acetamidophenyl, 4-acetamidophenyl, 2-methyl-3-acetamidophenyl, 2-methyl-3-aminophenyl, 3-methyl-4-aminophenyl, 2-amino-3-methylphenyl, 2,4-dimethyl-3-aminophenyl, 4-hydroxyphenyl, 3-methyl-4-hydroxyphenyl, 1-naphthyl, 2-naphthyl, 3-amino-1-naphthyl, 2-methyl-3-amino-1-naphthyl, 6-amino-2-naphthyl, 4,6-dimethoxy-2-naphthyl and the like. The terms "aralkyl" and "aralkoxy", alone or in combination, means an alkyl or alkoxy radical as defined above in which at least one hydrogen atom is replaced by an aryl radical as defined above, such as benzyl, benzyloxy, 2-phenylethyl, dibenzylmethyl, hydroxyphenylmethyl, methylphenylmethyl, and the like. The term "aralkoxycarbonyl", alone or in combination, means a radical of the formula aralkyl-O-C(O)- in which the term "aralkyl" has the significance given above. Examples of an aralkoxycarbonyl radical are benzyloxycarbonyl and 4-methoxyphenylmethoxycarbonyl. The term "aryloxy" means a radical of the formula aryl-O-in which the term aryl has the significance given above. The term "alkanoyl", alone or in combination, means an acyl radical derived from an alkanecarboxylic acid, examples of which include acetyl, propionyl, butyryl, valeryl, 4-methylvaleryl, and the like. The term "cycloalkylcarbonyl" means an acyl group derived from a monocyclic or bridged cycloalkanecarboxylic acid such as cyclopropylcarbonyl, cyclohexylcarbonyl, adamantylcarbonyl, and the like, or from a benz-fused monocyclic cycloalkanecarboxylic acid which is optionally substituted by one or more substituents selected from alkyl, alkoxy, halogen, hydroxy, amino, nitro, cyano, haloalkyl, carboxy, alkoxycarbonyl, cycloalkyl, heterocycloalkyl, alkanoylamino, amido, mono and dialkyl substituted amino, mono and dialkyl substituted amido and the like, such as 1,2,3,4-tetrahydro-2-naphthoyl, 2-acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydrocinnamoyl, 4-methoxyhydrocinnamoyl, and the like. The term "aroyl" means an acvl radical derived from an arylcarboxylic acid, aryl having the meaning given above. Examples of such arylcarboxylic acid radicals include substituted and unsubstituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl, 3-(benzyloxyformamidol-2-naphthoyl, and the like. The terms "heterocyclyl" and "heterocycloalkyl," alone or in combination, mean a saturated or partially unsaturated monocyclic, bicyclic or tricyclic heterocycle having preferably 3 to 12 ring members, more preferably 5 to 10 ring members and most preferably 5 to 6 ring members, which contains one or more heteroatom ring members selected from nitrogen, oxygen and sulphur, and which is optionally substituted on one or more carbon atoms by halogen, alkyl, alkoxy, hydroxy, oxo, aryl, aralkyl and the like, and/or on a secondary nitrogen atom (i.e., -NH-) by hydroxy, alkyl, aralkoxycarbonyl, alkanoyl, phenyl or phenylalkyl and/or on a tertiary nitrogen atom (i.e., =N-) by oxido. Heterocycloalkyl and heterocyclyl also includes benz-fused monocyclic cycloalkyl groups having at least one such heteroatom. Heterocycloalkyl and heterocyclyl in addition to sulfur and nitrogen also includes sulfones, sulfoxides and N-oxides of tertiary nitrogen containing heterocycloalkyl groups. The term "heteroaryl", alone or in combination, means an aromatic monocyclic, bicyclic, or tricyclic heterocyclyl (heterocycloalkyl) radical as defined above and is optionally substituted as defined above with respect to the definitions of aryl and heterocyclyl (heterocycloalkyl). Examples of such heterocyclyl (heterocycloalkyl) and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, (e.g., 2-(1-piperidinyl)pyridyl and 2-(4-benzyl piperazin-1-yl-1-pyridinyl), pyrazinyl, pyrimidinyl, furyl, tetrahydrofuryl, thienyl, triazolyl, oxazolyl, thiazolyl, indolyl (e.g., 2-indolyl, etc.), quinolinyl, (e.g., 2-quinolinyl, 3-quinolinyl, 1-oxido-2-quinolinyl, etc.), isoquinolinyl (e.g., 1-isoquinolinyl, 3-isoquinolinyl, etc.), tecrahydroquinolinyl (e.g., 1,2,3,4-tetrahydro-2-quinolyl, etc.), 1,2,3,4-tetrahydroisoquinolinyl (e.g., 1,2,3,4-tetrahydro-1-oxo-isoquinolinyl, etc.), quinoxalinyl, β-carbolinyl, 2-benzofurancarbonyl, 1-, 2-,4- or 5-benzimidazolyl, and the like. The term "cycloalkylalkoxycarbonyl" means an acyl group derived from a cycloalkylalkoxycarboxylic acid of the formula cycloalkylalkyl-O-COOH wherein cycloalkylalkyl has the meaning given above. The term "aryloxyalkanoyl" means an acyl radical of the formula aryl-O-alkanoyl wherein aryl and alkanoyl have the meaning given above. The term "heterorycloalkoxycarbonyl" means an acyl group derived from heterocyclyl-O-COOH wherein heterocyclyl is as defined above. The term "heterocycloalkylalkanoyl" is an acyl radical derived from a heterocycloalkyl-substituted alkylcarboxylic acid wherein heterocycloalkyl has the meaning given above. The term "heterocycloalkylalkoxycarbonyl" means an acyl radical derived from a heterocycloalkyl-substituted alkyl-O-COOH wherein heterocycloalkyl has the meaning given above. The term "heteroaryloxycarbonyl" means an acyl radical derived from a carboxylic acid represented by heteroaryl-O-COOH wherein heteroaryl has the meaning given above. The term "aminocarbonyl" alone or in combination, means an amino-substituted carbonyl (carbamoyl) group wherein the amino group can be a primary, secondary or tertiary amino group containing substituents selected from alkyl, aryl, aralkyl, cycloalkyl, cycloalkylalkyl radicals and the like. The term "aminoalkanoyl" means an acyl group derived from an amino-substituted alkylcarboxylic acid wherein the amino group can be a primary, secondary or tertiary amino group containing substituents selected from alkyl, aryl, aralkyl, cycloalkyl, cycloalkylalkyl radicals and the like. The term "halogen" means fluorine, chlorine, bromine or iodine. The term "haloalkyl" means an alkyl radical having the meaning as defined above wherein one or more hydrogens are replaced with a halogen. Examples of such haloalkyl radicals include chloromethyl, 1-bromoethyl, fluoromethyl, difluoromethyl, trifluoromethyl, 1,1,1-trifluoroethyl and the like. The term "leaving group" generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate. The term "amino acid side chain" means the side chain group, including the stereochemistry of the carbon to which it is attached, attached to the naturally occurring amino acid which distinguishes the amino acid from glycine. For example, the amino acid side chain of alanine is methyl, of histidine is imidazolylmethyl and phenylalanine is benzyl, and the attachment of such side chains to the compound of this invention retain the naturally occurring stereochemistry of the carbon to which it is attached. The following example illustrates the definition:

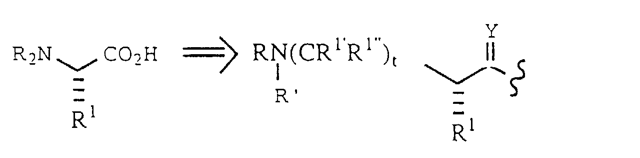

Procedures for preparing the compounds of Formula I are set forth below. It should be noted that the general procedure is shown as it relates to preparation of compounds having the specified stereochemistry, for example, wherein the absolute stereochemistry about the hydroxyl group is designated as (R). However, such procedures are generally applicable to those compounds of opposite configuration, e.g., where the stereochemistry about the hydroxyl group is (S). In addition, the compounds having the (R) stereochemistry can be utilized to produce those having the (S) stereochemistry. For example, a compound having the (R) stereochemistry can be inverted to the (S) stereochemistry using well-known methods.

### Preparation of Compounds of Formula I

The compounds of the present invention represented by Formula I above can be prepared utilizing the following general procedures. An N-protected amino expoxide of the formula 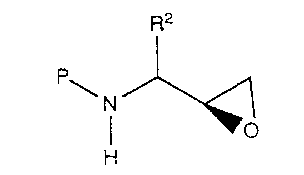 wherein P is an amino protecting group and R² is as defined above is prepared from the corresponding chloro ketone. Suitable solvent wherein P and R² are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

Alternatively, a protected amino epoxide can be prepared, such as in co-owned and co-pending PCT Patent Application Serial No. PCT/US93/04804 which is incorporated herein by reference, starting with an L-amino acid which is reacted with a suitable amino-protecting group in a suitable solvent to produce an amino-protected L-amino acid ester of the formula: 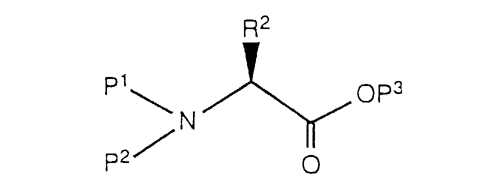 wherein P³ represents carboxyl-protecting group, e.g., methyl, ethyl, benzyl, tertiary-butyl and the like; R² is as defined above; and P¹ and P² independently are selected from amine protecting groups, including but not limited to, arylalkyl, substituted arylalkyl, cycloalkenylalkyl and substituted cycloalkenylalkyl, allyl, substituted allyl, acyl, alkoxycarbonyl, aralkoxycarbonyl and silyl. Examples of arylalkyl include, but are not limited to benzyl, ortho-methylbenzyl, trityl and benzhydryl, which can be optionally substituted with halogen, alkyl of C₁-C₈, alkoxy, hydroxy, nitro, alkylene, amino, alkylamino, acylamino and acyl, or their salts, such as phosphonium and ammonium salts. Examples of aryl groups include phenyl, naphthalenyl, indanyl, anthracenyl, durenyl, 9-(9-phenylfluorenyl) and phenanthrenyl, cycloalkenylalkyl or substituted cycloalkylenylalkyl radicals containing cycloalkyls of C₆-C₁₀. Suitable acyl groups include carbobenzoxy, t-butoxycarbonyl, iso-butoxycarbonyl, benzoyl, substituted benzoyl, butyryl, acetyl, trifluoroacetyl, tri-chloroacetyl, phthaloyl and the like.

Additionally, the P¹ and/or P² protecting groups can form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituced, e.g., nitrophthalimidyl. The term silyl refers to a silicon atom optionally substituted by one or more alkyl, aryl and aralkyl groups.

Suitable silyl protecting groups include, but are not limited to, trimethylsilyl, triethylsilyl, tri-isopropylsilyl, tert-butyldimethylsilyl, dimethylphenylsilyl, 1,2-bis(dimethylsilyl)benzene, 1,2-bis(dimethylsilyl)ethane and diphenylmethylsilyl. Silylation of the amine functions to provide mono- or bis-disilylamine can provide derivatives of the aminoalcohol, amino acid, amino acid esters and amino acid amide. In the case of amino acids, amino acid esters and amino acid amides, reduction of the carbonyl function provides the required mono- or bis-silyl aminoalcohol. Silylation of the aminoalcohol can lead to the N,N,O-tri-silyl derivative. Removal of the silyl function from the silyl ether function is readily accomplished by treatment with, for example, a metal hydroxide or ammonium flouride reagent, either as a discrete reaction step or in situ during the preparation of the amino aldehyde reagent. Suitable silylating agents are, for example, trimethylsilyl chloride, tert-buty-dimethylsilyl chloride, phenyldimethylsilyl chlorie, diphenylmethylsilyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Preferably P¹ and P² are independently selected from aralkyl and substituted aralkyl. More preferably, each of P¹ and P² is benzyl. As illustrated in the Examples below, P, P¹ and P² may serve as a nitrogen protecting group which is later removed in the preparation of compounds of this invention or may form a part of the final inhibitor structure. For example, benzoyl, benzyloxycarbonyl, t-butoxycarbonyl, pyridylmethoxycarbonyl, tetrahydrofuryloxycarbonyl, pyridylcarbonyl and the like can used to both protect a nitrogen from undergoing an undesired reaction and also be part of the structure of an active enzyme inhibitor.

The amino-protected L-amino acid ester is then reduced, to the corresponding alcohol. For example, the amino-protected L-amino acid ester can be reduced with diisobutylaluminum hydride at -78° C in a suitable solvent such as toluene. Preferred reducing agents include lithium aluminium hydride, lithium borohydride, sodium borohydride, borane, lithium tri-ter-butoxyaluminum hydride, borane/THF complex. Most preferably, the reducing agent is diisobutylaluminum hydride (DiBAL-H) in toluene. The resulting alcohol is then converted, for example, by way of a Swern oxidation, to the corresponding aldehyde of the formula: 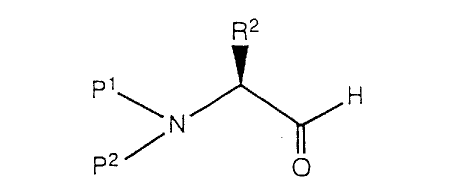 wherein P¹, P² and R² are as defined above. Thus, a dichloromethane solution of the alcohol is added to a cooled (-75 to -68° C) solution of oxalyl chloride in dichloromethane and DMSO in dichloromethane and stirred for 35 minutes.

Acceptable oxidizing reagents include, for example, sulfur trioxide-pyridine complex and DMSO, oxalyl chloride and DMSO, acetyl chloride or anhydride and DMSO, trifluoroacetyl chloride or anhydride and DMSO, methanesulfonyl chloride and DMSO or tetrahydro thiaphene-S-oxide, toluenesulfonyl bromide and DMSO, trifluoromethanesulfonyl anhydride (triflic anhydride) and DMSO, phosphorus pentachloride and DMSO, dimethylphosphoryl chloride and DMSO and isobutyl chloroformate and DMSO. The oxidation conditions reported by Reetz et al [Angew Chem., 99, p. 1186, (1987)], Angew Chem. Int. Ed. Engl., 26, p. 1141, 1987) employed oxalyl chloride and DMSO at -78°C.

The preferred oxidation method described in this invention is sulfur trioxide pyridine complex, triethylamine and DMSO at room temperature. This system provides excellent yields of the desired chiral protected amino aldehyde usable without the need for purification i.e., the need to purify kilograms of intermediates by chromatography is eliminated and large scale operations are made less hazardous. Reaction at room temperature also eliminated the need for the use of low temperature reactor which makes the process more suitable for commercial production.

The reaction may be carried out under and inert atmosphere such as nitrogen or argon, or normal or dry air, under atmospheric pressure or in a sealed reaction vessel under positive pressure. Preferred is a nitrogen atmosphere. Alternative amine bases include, for example, tri-butyl amine, tri-isopropyl amine, N-methylpiperidine, N-methyl morpholine, azabicyclononane, diisopropylethylamine, 2,2,6,6-tetramethylpiperidine, N,N-dimethylaminopyridine, or mixtures of these bases. Triethylamine is a preferred base. Alternatives to pure DMSO as solvent include mixtures of DMSO with non-protic or halogenated solvents such as tetrahydrofuran, ethyl acetate, toluene, xylene, dichloromethane, ethylene dichloride and the like. Dipolar aprotic to-solvents include acetonitrile, dimethylformamide, dimethylacetamide, acetamide, tetramethyl urea and its cyclic analog, N-methylpyrrolidone, sulfolane and the like. Rather than N,N-dibenzylphenylalaninol as the aldehyde precursor, the phenylalaninol derivatives discussed above can be used to provide the corresponding N-monosubstituted [either P¹ or P² = H] or N,N-disubstituted aldehyde.

In addition, hydride reduction of an amide or ester derivative of the corresponding alkyl, benzyl or cycloalkenyl nitrogen protected phenylalanine, substituted phenylalanine or cycloalkyl analog of phenyalanine derivative can be carried out to provide the aldehydes. Hydride transfer is an additional method of aldehyde synthesis under conditions where aldehyde condensations are avoided, cf, Oppenauer Oxidation.

The aldehydes of this process can also be prepared by methods of reducing protected phenylalanine and phenylalanine analogs or their amide or ester derivatives by, e.g., sodium amalgam with HCl in ethanol or lithium or sodium or potassium or calcium in ammonia. The reaction temperature may be from about -20°C to about 45°C, and preferably from abut 5°C to about 25°C. Two additional methods of obtaining the nitrogen protected aldehyde include oxidation of the corresponding alcohol with bleach in the presence of a catalytic amount of 2,2,6,6-tetramethyl-1-pyridyloxy free radical. In a second method, oxidation of the alcohol to the aldehyde is accomplished by a catalytic amount of tetrapropylammonium perruthenate in the presence of N-methylmorpholine oxide.

Alternatively, an acid chloride derivative of a protected phenylalanine or phenylalanine derivative as disclosed above can be reduced with hydrogen and a catalyst such as Pd on barium carbonate or barium sulphate, with or without an additional catalyst moderating agent such as sulfur or a thiol (Rosenmund Reduction).

The aldehyde resulting from the Swern oxidation is then reacted with a halomethyllithium reagent, which reagent is generated in situ by reacting an alkyllithium or arylithium compound with a dihalomethane represented by the formula X¹CH₂X² wherein X¹ and X² independently represent I, Br or Cl. For example, a solution of the aldehyde and chloroiodomethane in THF is cooled to -78° C and a solution of n-butyllithium in hexane is added. The resulting product is a mixture of diastereomers of the corresponding amino-protected epoxides of the formulas: 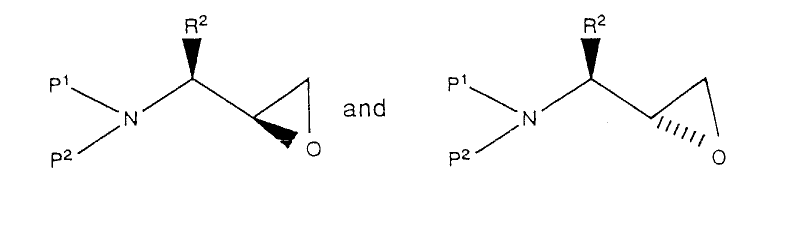 The diastereomers can be separated e.g., by chromatography, or, alternatively, once reacted in subsequent steps the diastereomeric products can be separated. For compounds having the (S) stereochemistry, a D-amino acid can be utilized in place of the L-amino acid.

The addition of chloromethylithium or bromomethylithium to a chiral amino aldehyde is highly diastereoselective. Preferably, the chloromethyllithium or bromomethylithium is generated in-situ from the reaction of the dihalomethane and n-butyllithium. Acceptable methyleneating halomethanes include chloroiodomethane, bromochloromethane, dibromomethane, diiodomethane, bromofluoromethane and the like. The sulfonate ester of the addition product of, for example, hydrogen bromide to formaldehyde is also a methyleneating agent.
Tetrahydrofuran is the preferred solvent, however alternative solvents such as toluene, dimethoxyethane, ethylene dichloride, methylene chloride can be used as pure solvents or as a mixture. Dipolar aprotic solvents such as acetonitrile, DMF, N-methylpyrrolidone are useful as solvents or as part of a solvent mixture. The reaction can be carried out under an inert atmosphere such as nitrogen or argon. For n-butyl lithium can be substituted other organometalic reagents reagents such as methyllithium, tert-butyl lithium, set-butyl lithium, phenyllithium, phenyl sodium and the like. The reaction can be carried out at temperatures of between about -80°C to 0°C but preferably between about -80°C to -20°C. The most preferred reaction temperatures are between -40°C to -15°C. Reagents can be added singly but multiple additions are preferred in certain conditions. The preferred pressure of the reaction is atmospheric however a positive pressure is valuable under certain conditions such as a high humidity environment.

Alternative methods of conversion to the eooxides of this invention include substitution of other charged methylenation precurser species followed by their treatment with base to form the analogous anion. Examples of these species include trimethylsulfoxonium tosylate or triflate, tetramethylammonium halide, methyldiphenylsulfoxonium halide wherein halide is chloride, bromide or iodide.

The conversion of the aldehydes of this invention into their epoxide derivative can also be carried out in multiple steps. For example, the addition of the anion of thioanisole prepared from, for example, a butyl or aryl lithium reagent, to the protected aminoaldehyde, oxidation of the resulting protected aminosulfide alcohol with well known oxidizing agents such as hydrogen peroxide, cert-butyl hypochlorite, bleach or sodium periodate to give a sulfoxide. Alkylation of the sulfoxide with, for example, methyl iodide or bromide, methyl tosylate, methyl mesylate, methyl triflate, ethyl bromide, isopropyl bromide, benzyl chloride or the like, in the presence of an organic or inorganic base Alternatively, the protected aminosulfide alcohol can be alkylated with, for example, the alkylating agents above, to provide a sulfonium salts that are subsequently converted into the subject epoxides with tert-amine or mineral bases.

The desired epoxides formed, using most preferred conditions, diastereoselectively in ratio amounts of at least about an 85:15 ratio (S:R). The product can be purified by chromatography to give the diastereomerically and enantiomerically pure product but it is more conveniently used directly without purification to prepare retroviral protease inhibitors. The foregoing process is applicable to mixtures of optical isomers as well as resolved compounds. If a particular optical isomer is desired, it can be selected by the choice of starting material, e.g., L-phenylalanine, D-phenylalanine, L-phenylalaninol, D-phenylalaninol, D-hexahydrophenylalaninol and the like, or resolution can occur at intermediate or final steps. Chiral auxiliaries such as one or two equivilants of camphor sulfonic acid, citric acid, camphoric acid, 2-methoxyphenylacetic acid and the like can be used to form salts, esters or amides of the compounds of this invention. These compounds or derivatives can be crystallized or separated chromatographically using either a chiral or achiral column as is well known to those skilled in the art.

The amino epoxide is then reacted, in a suitable solvent system, with an equal amount, or preferably an excess of, a desired amine of the formula R³NH₂, wherein R³ is hydrogen or is as defined above. The reaction can be conducted over a wide range of temperatures, e.g., from about 10°C to about 100°C, but is preferably, but not necessarily, conducted at a temperature at which the solvent begins to reflux. Suitable solvent systems include protic, non-protic and dipolar aprotic organic solvents such as, for example, those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R²)-1-(NHR³)-propan-2-ol derivative (hereinafter referred to as an amino alcohol) can be represented by the formulas: 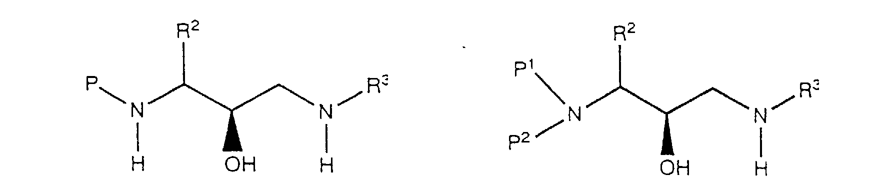 wherein P, P¹, P², R² and R³ are as described above. Alternatively, a haloalcohol can be utilized in place of the amino epoxide.

The amino alcohol defined above is then reacted in a suitable solvent with a sulfonyl chloride (R⁴SO₂Cl) or sulfonyl anhydride in the presence of an acid scavenger. Suitable solvents in which the reaction can be conducted include methylene chloride, tetrahydrofuran. Suitable acid scavengers include triethylamine, pyridine. Preferred sulfonyl chlorides are methanesulfonyl chloride and benzenesulfonyl chloride. The resulting sulfonamide derivative can be represented, depending on the epoxide utilized by the formulas 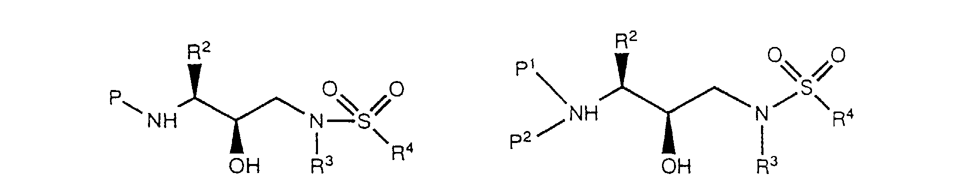 wherein P, P¹, P², R², R³ and R⁴ are as defined above. These intermediates are useful for preparing inhibitor compounds of the present invention and are also active inhibitors of retroviral proteases.

The sulfonyl halides of the formula R⁴SO₂X can be prepared by the reaction of a suitable Grignard or alkyl lithium reagent with sulfuryl chloride, or sulfur dioxide followed by oxidation with a halogen, preferably chlorine. Also, thiols may be oxidized to sulfonyl chlorides using chlorine in the presence of water under carefully controlled conditions. Additionally, sulfonic acids may be converted to sulfonyl halides using reagents such as PCl₅, and also to anhydrides using suitable dehydrating reagents. The sulfonic acids may in turn be prepared using procedures well known in the art. Such sulfonic acids are also commercially available. In place of the sulfonyl halides, sulfinyl halides (R⁴SOX) or sulfenyl halides (R⁴SX) can be utilized to prepare compounds wherein the -SO₂- moiety is replaced by an -SO- or -S- moiety, respectively.

Following preparation of the sulfonamide derivative, the amino protecting group P or P¹ and P² amino protecting groups are removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative of the formula

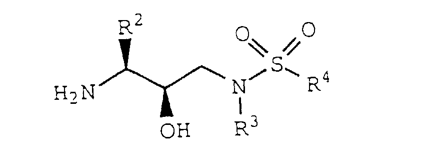

This amine can be coupled to a carboxylate represented by the formula 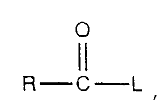 wherein R is as defined above and L is an appropriate leaving group such as a halide. Preferably, where R¹ is a side chain of a naturally occurring α-amino acid, R is a 2-quinoline carbonyl group derived from N-hydroxysuccinimide-2-quinoline carboxylate, i.e., L is hydroxy succinimide. A solution of the free amine (or amine acetate salt) and about 1.0 equivalent of the carboxylate are mixed in an appropriate solvent system and optionally treated with up to five equivalents of a base such as, for example, N-methylmorpholine, at about room temperature. Appropriate solvent systems include tetrahydrofuran, methylene chloride or N,N-dimethyl formamide, and the like, including mixtures thereof.

Alternatively, the protected amino alcohol from the epoxide opening can be further protected at the newly introduced amino group with a protecting group P' which is not removed when the first protecting P is removed. One skilled in the art can choose appropriate combinations of P

The thiocarbonyl compounds of this invention are really prepared by methods well known to those skilled in the art, for example, by treatment of a carbonyl compound with Lawesson's reagent (2,4-bis(4-methoxyphenyl) -1,3-dithia-2,4-diphosphetane-2,4-disulfide) which is an article of commerce. Phosphorus pentasulfide may also be used or one can treat an amine of this invention with a pre-formed thiocarbonyl reagent such as thiocarbonylchlorid in the presence of base.

In place of the sulfonyl halides, sulfinyl halides (RSOCl) and sulfenyl halides (RSCl) can be utilized to prepare compounds wherein the -SO₂- moiey is replaced by -SO- or -S-, respectively.

It is contemplated that where R³ of the amino alcohol intermediate is hydrogen, the inhibitor compounds of the present invention wherein R³ is alkyl, or other substituents wherein the α-C contains at least one hydrogen, can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties, such as tautomers thereof as well as compounds, wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, or other reactions disclosed herein or otherwise conventional, will be applicable to the preparation of the corresponding compounds of this invention. In all preparative methods, all starting materials are known or readily preparable from known starting materials.

All reagents were used as received without purification. All proton and carbon NMR spectra were obtained on either a Varian VXR-300 or VXR-400 nuclear magnetic resonance spectrometer.

The following Examples 1 through 10 illustrate preparation of the compounds of the present invention.

### Example 1A

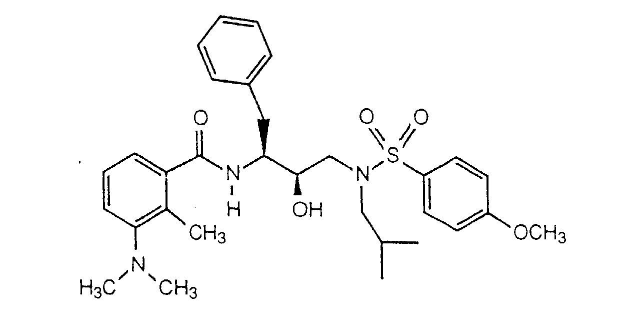

### Preparation of Benzamide, N-[2R-hydroxy-3-[[(4-methoxyphenyl)sulfonyl](2-methylpropyl)amino]-1S-(phenylmethyl)propyl]-2-methyl.

Part A. Preparation of 4-Nitro-2-methylbenzoic Acid.
   A mixture of 1.0 g (3.8 mmol) of 2-iodo-nitrotoluene, 2.1 g (15.2 mmol) potassium carbonate and 27 mg (0.038 mmol) of palladium(II) dichloride bis(triphenylphosphine) in a mixture of 5mL of water and 10 mL of N,N-dimethylformamide. This was placed in a Fisher/Porter bottle under 15 psig of carbon monoxideand heated at 70°C for 16 hours. The solution became homogeneous when heated. The reaction was cooled, diethyl ether and water was added, the organic layer separated and discarded. The aqueous layer was acidified with 1N hydrohloric acid, extracted with ethyl acetate, washed with water, brine, dried over magnesium sulfate, filtered and concentrated to yield 0.5g of crude material. This dissolved in ethyl acetate, hexane added and the resulting brown solid discarded. The filtrate was concentrated, and then recrystallized fom diethyl ether/hexane to afford 215mg of 4-nitro-2-methylbenzoic acid, m/e=182(M+H).
Part B. Preparation of Benzamide, N-[2R-hydroxy-3-[[(4-methoxyphenyl)sulfonyl](2-methylpropyl)amino]-1S-(phenylmethyl)propyl]-2-methyl-4-nitro.
   To a solution of 181mg (1.0mmol) of 4-nitro-2-methylbenzoic acid and 230mg(1.5mmol) N-hydroxybenzotriazole in 3mL of anhydrous N,Ndimethylformamide at 0°C, was added 211mg(1.1mmol) of 1-(3-dimethylaminopropyl)-3-ethylcarbodiimide hydrochloride. After stirring at 0 C for 1 hour, 406mg (1mmol) of 2R-hydroxy-3-[(2-methylpropyl)(4-methoxyphenyl)sulfonyl]amino-1S-(phenylmethyl)propylamine was added. After 17 hours at room temperature, the solvent was removed under reduced pressure, ethyl acetate added, washed with 5% citric acid, saturated sodium bicarbonate, brine, dried with magnesium sulfate, filtered and concentrated to yield 0.55g of crude product. This was chromatographed on silica gel using 20-50% ethyl acetate/hexane as eluent to afford 0.49g of the desired benzamide, N-[2R-hydroxy-3-[[(4-methoxyphenyl)sulfonyl](2-methylpropyl)amino]-1S-(phenylmethyl)propyl]-2-methyl-4-nitro, m/e=570 (M+H).
Part C. Preparation of Benzamide, N-[2R-hydroxy-3-[[(4-methoxyphenyl)sulfonyl](2-methylpropyl)amino]-1S-(phenylmethyl)propyl]-2-methyl-4-amino.
   A solution of 400mg (0.70mmol) of benzamide, N-[2R-hydroxy-3-[[(4-methoxyphenyl)sulfonyl](2-methylpropyl)amino]-1S-(phenylmethyl)propyl]-2-methyl-4-nitro from part B in 20mL of methanol was hydrogenated over 0.2 g of 10% palladium on carbon catalyst under 50 psig of hydrogen for 2.5 hours. The catalyst was removed by filtration and the solution concentrated to afford 370mg of the desired benzamide, N-[2R-hydroxy-3-[[(4-methoxyphenyl)sulfonyl](2-methylpropyl)amino]-1S-(phenylmethyl)propyl]-2-methyl-4-amino, m/e=540(M+H).
Part D. Preparation of Benzamide, N-[2R-hydroxy-3-[[(4-methoxyphenyl)sulfonyl](2-methylpropyl)amino]-1S-(phenylmethyl)propyl]-2-methyl-4-dimethylamino.
   A solution of 0.17g (0.31mmol) of benzamide, N-[2R-hydroxy-3-[[(4-methoxyphenyl)sulfonyl](2-methylpropyl)amino]-1S-(phenylmethyl)propyl]-2-methyl-4-amino from part C in 5mL of methanol and 0.20mL of 37% aqueous formaldehyde was hydrogenated over 90mg of 10% palladium on carbon under 15psig of hydrogen for 16 hours. The catalyst was removed by filtration, the solvents removed under reduced pressure to afford 0.16g of crude material. Chromatography on silica gel using 50% ethyl acetate as eluent afforded 0.12g of the desired benzamide, N-[2R-hydroxy-3-[[(4-methoxyphenyl)sulfonyl] (2-methylpropyl)amino]-1S-(phenylmethyl)propyl]-2-methyl-4-dimethylamino, m/e=568 (M+H).

### Example 1B

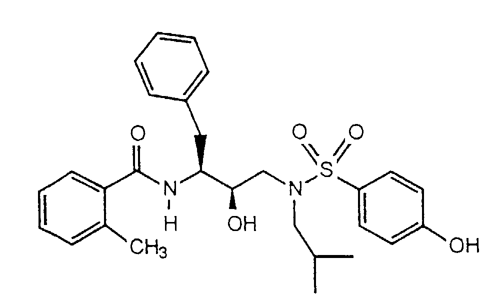

### Preparation of Benzamide, N-[2R-hydroxy-3-[[(4-hydroxyphenyl)sulfonyl](2-methylpropyl)amino]-1S-(phenylmethyl)propyl]-2-methyl.

To a solution of 500 mg (1mmol) of 2R-hydroxy-3-[(2-methylpropyl)(4-hydroxyphenyl)sulfonyl]amino-1S-(phenylmethyl)propylamine in 2 mL of methylene chloride and 2 mL of N,N-dimethylformamide, was added 0.42 mL of triethylamine, followed by 0.12 mL of ortho-toluoyl chloride. After 17 hours, the solvent was removed under reduced pressure, the residue dissolved in ethyl acetate, was with 5% citric acid, saturated sodium bicarbonate and brine, dried over anhydrous magnesium sulfate, filtered and concentrated to afford 490 mg of crude material. This was chromatographed over 100g of silica gel using 20-50% ethyl acetate/hexane as eluent to afford 232 mg of the desired product, m/e=511 (M+H).

### Example 1C

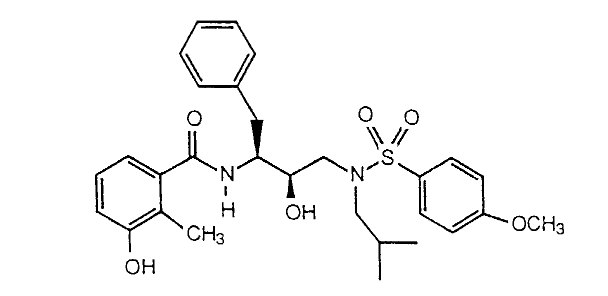

### Preparation of Benzamide, N-[2R-hydroxy-3-[[(4-methoxyphenyl]sulfonyl](2-methylpropyl)amino]-1S-(phenylmethyl)propyl]-3-hydroxy-2-methyl

To a solution of 131 mg (0.86 mmol) of 3-hydroxy-2-methylbenzoic acid and 305 mg (0.75 mmol) of N-hydroxybenzotriazole in 4 mL of anhydrous N,N-dimethylformamide at 0°C, was added 165 mg (0.86 mmol) of EDC. After 20 minutes of activation at 0°C and 1 hour at room temperature, 305 mg (0.75 mmol) of 2R-hydroxy-3-[(2-methylpropyl)(4-methoxyphenyl)sulfonyl]amino-1S(phenylmethyl)propylamine was added. After 15 hours at room temperature, ethyl acetate was added, washed with 5% citric acid, saturated sodium bicarbonate, brine, dried, filtered and concentrated to afford 460 mg of crude material. This was chromatographed on silica gel using 0-35% ethyl acetate/methylene chloride as eluent to afford 250 mg of pure benzamide, N-[2R-hydroxy-3-[[(4-methoxyphenyl)sulfonyl](2-methylpropyl)amino]-1S-(phenylmethyl)propyl]-3-hydroxy-2-methyl, m/e = 547 (M+Li).

### EXAMPLE 1D

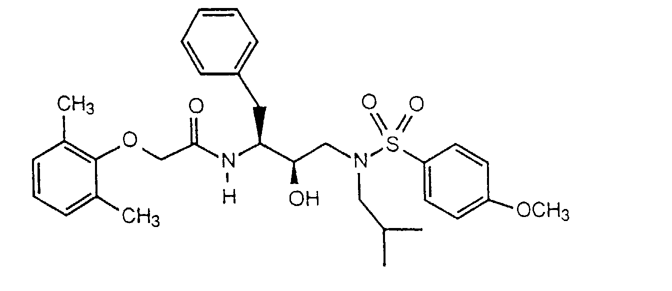

### Preparation of N-[2R-hydroxy-3-[[(4-methoxyphenyl) sulfonyl](2-methylpropyl)amino]-1S-(phenylmethyl)propyl]-(2,6-dimethylphenoxy)acetamide

### Part A: Preparation of 2,6-Dimethylphenoxyacetic acid

2,6-Dimethylphenol (6.1 g, 50.0 mmol), bromoacetic acid (6.9 g, 50.0 mmol) and 2.5 N aqueous sodium hyroxide (50.0 mL, 125.0 mmol) were refluxed in water (125 mL) for 4 hrs. Bromoacetic acid (6.9 g, 50.0 mmol) and 2.5 N aqueous sodium hydroxide (20.0 mL, 62.5 mmol) were added and the solution refluxed for an additional 16 hrs. The solution was cooled to room temperature and water (200 mL) was added. The pH of the solution was adjusted to 1.0 with concentrated aqueous hydrochloric acid. The resulting precipitate was collected and recrystallized from ethyl acetate/hexanes (1:9, 700 mL). 2,6-Dimethylphenoxyacetic acid (4.53 g, 25.1 mmol, 50%) was collected as a white crystalline solid. ¹H NMR (CD₃OD) d 2.26 (s, 6H), 4.38 (s, 2H), 6.90-7.00 (m, 3H).

### Part B: Preparation of N-[2R-hydroxy-3-[[(4-methoxyphenyl)sulfonyl](2-methylpropyl)amino]-1S-(phenylmethyl)propyl]-(2,6-dimethylphenoxy)acetamide

To a solution of 180.1 mg (0.83 mmol) of 2,6-(dimethylphenoxy)acetic acid in 10 mL of anhydrous methylene chloride at room temperature, was added 114 mg (0.60 mmol) of EDC. After 15 minutes of activation, 203 mg of 2R-hydroxy-3-[[(2-methylpropyl)(4-methoxybenzene) sulfonyl]amino]-1S-(phenylmethyl)propylamine was added. After stirring at room temperature for 16 hours the solution was extracted with 5% citric acid, sodium bicarbonate, brine, dried over magnesium sulfate, filtered and concentrated to afford 244 mg of crude product. A quantity of this (15 mg) was chromatographed on silica gel using 25% ethyl acetate/hexane to afford 8 mg of the desired compound, m/e=575(M+Li).

### Example 1E

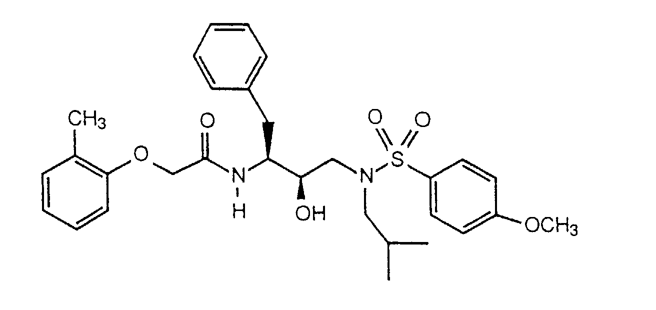

### Preparation of N-[2R-hydroxy-3-[[(4-methoxyphenyl)sulfonyl](2-methylpropyl)amino]-1S-(phenylmethyl)propyl]-(2-methylphenoxy)acetamide

### Part A: Preparation of 2-Methylphenoxyacetic Acid.

2-Methylphenol (2.0 g, 18.4 mmol), bromoacetic acid (2.5 g, 18.4 mmol) and 2.5 N aqueous sodium hyroxide (25.0 mL, 62.55.0 mmol) refluxed for 16 hrs. The pH of the solution was adjusted to 1 with concentrated aqueous hydrochloric acid. The resulting precipitate was collected triturated with hexanes. The 2-methylphenoxyacetic acid (720 mg, 4.33 mmol, 25%) was collected as a white crystalline solid. ¹HNMR (CD₃OD) d 2.43 (s, 3H), 4.65 (s, 2H) 6.70-7.10 (m, 4H).

### Part B: Preparation of N-[2R-hydroxy-3-[[(4-methoxyphenyl)sulfonyl](2-methylpropyl)amino]-1S-(phenylmethyl)propyl]-(2-methylphenoxy)acetamide

To a solution of 97 mg (0.59 mmol) of 2-(methylphenoxy) acetic acid in 5 mL of anhydrous methylene chloride at room temperature, was added 89.1 mg (0.55 mmol) of carbonyl diimidazole. After 15 minutes of activation, 200 mg (0.49 mmol) of 2R-hydroxy-3-[[(2-methylpropyl)(4-methoxybenzene)sulfonyl]amino]-1S-(phenylmethyl) propylamine was added. After stirring at room temperature for 15 hours the solution was extracted with 5% citric acid, sodium bicarbonate, brine, dried over magnesium sulfate, filtered and concentrated to afford crude product. This was chromatographed on silica gel using 25% ethyl acetate/hexane to afford 198 mg of the desired compound.

### Example 1F

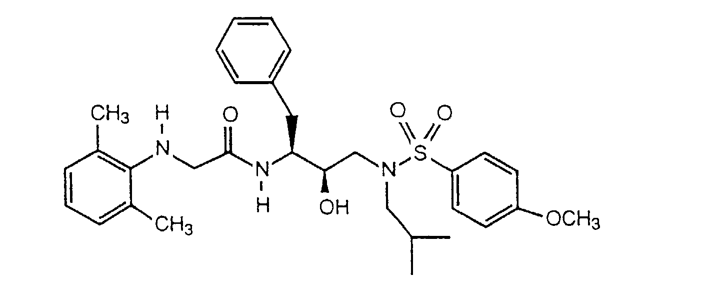

### Preparation of N-[2R-hydroxy-3-[[(4-methoxyphenyl) sulfonyl](2-methylpropyl)amino]-1S-(phenylmethyl)propyl]-2 -(2,6-dimethylphenylamino)acetamide

Part A: Preparation of N-(2,6 Dimethylphenyl)glycine.
   2,6-Dimethylaniline (6.1 g, 50.4 mmol), and ethyl bromoacetate (8.4 g, 50.4 mmol) were refluxed neat for 10 min. The reaction mixture was cooled to room temperature and poured into dichloromethane (75 mL). A precipitated formed which was collected and triturated with dichloromethane (25 mL). N-(2,6-Dimethylphenyl)glycine hydrobromide salt (1.21 g, 4.6 mmol, 9.0%) was collected as a white crystalline solid. ¹H NMR (CD₃OD) d 2.48 (s, 6H), 4.29 (s, 2H), 7.00-7.10 (m, 3H).
Part B: Preparation of N-[2R-hydroxy-3-[[(4-methoxyphenyl)sulfonyl](2-methylpropyl)amino]-1S-(phenylmethyl)propyl]-2-(2,6-dimethylphenylamino) acetamide
   To a solution of 100 mg (0.39 mmol) of N-(2,6-dimethylphenyl)glycine hydrobromide and 100 mg of triethylamine in 5 mL of anhydrous methylene chloride at room temperature, was added 74 mg (0.39 mmol) of EDC. After 15 minutes of activation, 157 mg (0.39 mmol) of 2R-hydroxy-3-[[(2-methylpropyl)(4-methoxybenzene)sulfonyl] amino]-1S-(phenylmethyl)propylamine was added. After stirring at room temperature for 4 hours, an additional 100 mg of N-(2,6-dimethylphenyl)glycine and 74 mg of EDC was added. After stirring at room temperature for 16 hours, the solution was extracted with 5% citric acid, sodium bicarbonate, brine, dried over magnesium sulfate, filtered and concentrated to afford 206 mg of crude product. This was purified by chromtaograpghy on reverse phase using 20-90% acetonitrile/water (0.05% trifluoroacetic acid) to afford 75 mg of the desired compound, m/e = 568 (M+H).

### Example 1G

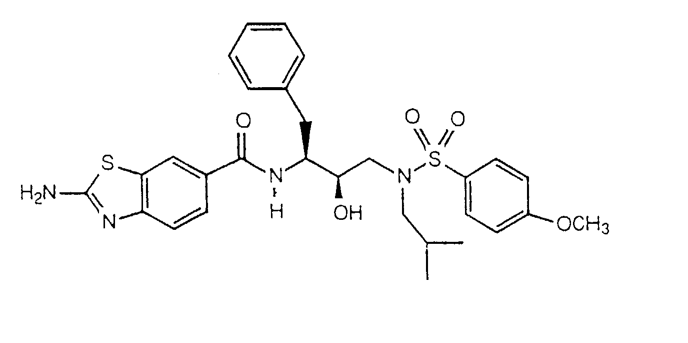

### Preparation of N-[2R-hydroxy-3-[[(4-methoxyphenyl) sulfonyl](2-methylpropyl)amino]-1S-(phenylmethyl)propyl]-2-amino-benzothiazole-6-carboxamide

### Part A: Preparation of 2-Amino-6-Carboxy-Benzothiazole Ethyl Ester

A 100 ml round bottom flask equipped with magnetic stir bar and N₂ inlet was charged with 1.0 g of methyl p-aminobenzoate in 35 mL methanol. The solution was heated to reflux and 4.0 g of Cu^{II}SO₄ and 5.0 g of KSCN were added. The reaction mixture was refluxed 2 hours and then filtered. The filtrate was diluted with 60 mL of water and 20 mL of ethanol and heated to boiling. Upon cooling 1.15 g (78%) of 2-Amino-6-Carboxy-Benzothiazole Ethyl Ester was isolated, m/e=223(M+H).

### Part B: Preparation of 2-Amino-6-Carboxy-Benzothiazole.

A 50 mL round bottom flask equipped with magnetic stir bar was charged with 250 mg 2-Amino-6-Carboxy-Benzothiazole Ethyl Ester, 190 mg (4 eq.) LiOH in 3 mL dioxane and 3 mL water. The slurry was heated to 60°C for 2 hours. After 2 hours the solution was acidified with 1N HCl and concentrated in vacuo to a light grey solid which was identified as 2-amino-6-carboxy-benzothiazole, m/e = 195(M+H). It was used without further purification.

### Part C: Preparation of N-[2R-hydroxy-3-[[(4-methoxyphenyl)sulfonyl](2-methylpropyl)amino]-1S-(phenylmethyl)propyl]-2-amino-benzothiazole-6-carboxamide

A 100 mL round bottom flask equipped with magnetic stir bar and N₂ inlet was charged with 110 mg 2-amino-6-carboxybenzothiazole, 110 mg EDC, and 100 mg HOBt in 4 mL dry DMF. After 30 minutes activation 203 mg amine (A) and 5 mL of triethylamine were added and the reaction was stirred overnight. The reaction was partioned between ethyl acetate and saturated aqueous sodium bicarbonate. The combined organics were washed with 10 % aqueous Citric Aacid, water, saturated aqueous sodium bicarbonate, brine and concentrated in vacuo to 210 mg white foam, idenitifed as the desired product, m/e=589 (M+Li)

### Example 2A

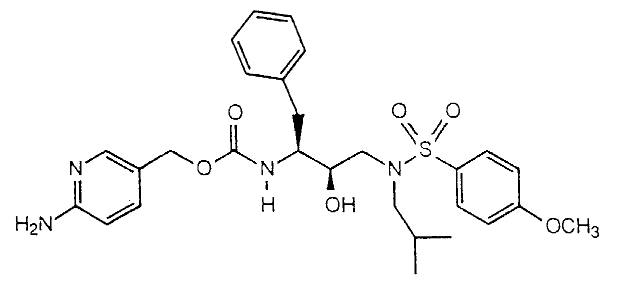

### Preparation of Carbamic acid, [2R-hydroxy-3-[[(4-methoxyphenyl)sulfonyl](2-methylpropyl)amino]-1S-(phenylmethyl)propyl]-, 3-(6-aminopyridyl)methyl ester.

### Part A: Prepartion of Ethyl 6-Aminonicotinate

To a suspension of 1.3g (9.4 mmol) 6-aminonicotinic acid in 100 mL of ethanol, was bubbled in dry hydrochloric acid at 0°C, then the solution was refluxed until all the solids dissolved. The solvents were removed under reduced pressure, the residue dissolved in ethyl acetate, washed with saturated sodium bicarbonate, brine and concentrated to afford 1.37g of a white solid, m/e=166(M+H).

### Part B: Preparation of Ethyl 6-(tert-Butyloxycarbonylamino)nicotinate

A mixture of 848 mg(5.1 mmol) of ethyl 6-aminonicotinate from part A, 1.11g(5.1 mmol) of di-tert-butylpyrocarbonate and 0.71 mL (5.1 mmol) of triethylamine in 10mL of anhydrous toluene was refluxed for 15 hours. The solution was cooled, ethyl acetate added, washed with saturated sodium bicarbonate, brine, dried over anhydrous magnesium sulfate, filtered and concentrated to afford 1.28g of the desired ethyl 6-(tert-butyloxycarbonylamino)nicotinate, m/e=267(M+H), which was used directly in the next step.

### Part C: Preparation of 6-(tert-Butyloxycarbonylamino)-3-pyridylmethanol

To 4.6 mL (4.6 mmol) of a 1M solution of lithium aluminum hydride in diethyl ether at -40°C under a nitrogen atmosphere, was added a slution of 618mg (2.3 mmol) of ethyl 6-(tert-butyloxycarbonylamino)nicotinate from part B in 40 mL of anhydrous tetrahydrofuran. After the addition, this was warmed to room temperature, stirred for 3 hours, cooled to 0°C, and 145 µL of water, 145µL of 20% sodium hydroxide solution and 290µL of water were successively added. To the resulting mixture was added 50mL of tetrahydrofuran and stirring continued for 30 minutes. Anhydrous magnesium sulfate was added, the solids removed via filtration and the filtrate concentrated under reduced pressure to afford 460 mg of the desired product, m/e=224(M+), which was used directly in the next step.

### Part D: Preparation of Carbamic acid, [2R-hydroxy-3-[[(4-methoxyphenyl)sulfonyl](2-methylpropyl)amino]-1S-(phenylmethyl)propyl]-, 3-[(6-tert-butyloxycarbonylamino)pyridyl]methyl ester

To a solution of 336 mg (1.5mmol) of 6-(tert-butyloxycarbonylamino)-3-pyridylmethanol from part C in 14 mL of anhydrous acetonitrile at room temperature under a nitrogen atmosphere, was added 384 mg(1.5mmol) of N,N'-disuccinimidyl carbonate and 364 µL (4.5mmol) of anhydrous pyridine. After 4 hours, 406mg (1mmol) of 2R-hydroxy-3-[(2-methylpropyl)(4-methoxyphenyl)sulfonyl)amino-1S-(phenylmethyl)propylamine was added and stirring continued for 19 hours. The solvent was removed under reduced pressure, ethyl acetate added, washed with saturated sodium bicarbonate, brine, dried over magnesium sulfate, filtered and concentrated to afford 702 mg of crude product. Chromatography on silica gel using 1% methanol/methylene chloride as eluent afforded 170 mg of the desired carbamic acid, [2R-hydroxy-3-[[(4-methoxyphenyl)sulfonyl](2-methylpropyl)amino]-1S-(phenylmethyl)propyl]-, 3-[(6-tert-butyloxycarbonylamino)pyridyl]methyl ester, m/e=663(M+Li).

### Part E: Preparation of Carbamic acid, [2R-hydroxy-3-[[(4-methoxyphenyl)sulfonyl](2-methylpropyl)amino]-1S-(phenylmethyl)propyl]-, 3-(6-aminopyridyl)methyl ester

To 5mL of 4N hydrochloric acid in dioxane at room temperature, was added 150mg (0.23mmol) of carbamic acid, [2R-hydroxy-3-[[(4-methoxyphenyl)sulfonyl](2-methylpropyl)amino]-1S-(phenylmethyl)propyl]-, 3-[(6-tert-butyloxycarbonylamino)pyridyl]methyl ester from part D. After stirring at room temperature for 28 hours, the solvent was removed under reduced pressure, the resulting solids triturated with diethyl ether, then dissolved in ethyl acetate and saturated sodium bicarbonate slution, separated, the organic layer washed with brine, dried with magnesium sulfate, filtered and concentrated. The residue was chromatographed on silica gel using 2.5% methanol/methylene chloride to yield 59mg of the desired carbamic acid, [2R-hydroxy-3-[[(4-methoxyphenyl)sulfonyl](2-methylpropyl) amino]-1S-(phenylmethyl)propyl]-, 3-(6-aminopyridyl)methyl ester, m/e=557(M+H).

### EXAMPLE 2B

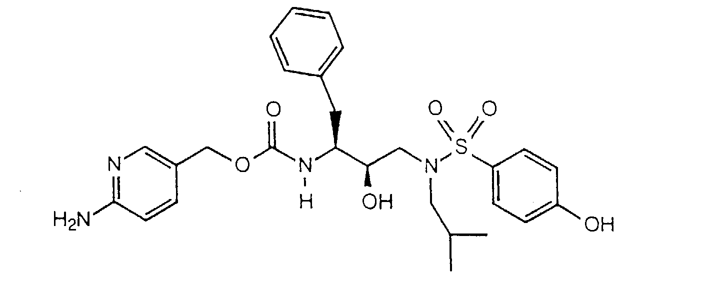

### Preparation of Carbamic acid, [2R-hydroxy-3-[[(4-hydroxyphenyl)sulfonyl](2-methylpropyl)amino]-1S-(phenylmethyl)propyl]-, 3-(6-aminopyridyl)methyl ester.

### Part A: Preparation of Carbamic acid, [2R-hydroxy-3-[[(4-methoxyphenyl)sulfonyl](2-methylpropyl)amino]-1S-(phenylmethyl)propyl]-, 3-[(6-tert-butyloxycarbonylamino)pyridyl]methyl ester

To a solution of 505 mg (2.25mmol) of 6-(tert-butyloxycarbonylamino)-3-pyridylmethanol from in 20 mL of anhydrous acetonitrile at room temperature under a nitrogen atmosphere, was added 576 mg (2.25mmol) of N,N'-disuccinimidyl carbonate and 546 µL (6.75mmol) of anhydrous pyridine. After 1 hour, 837mg (1.87mmol) of 2R-hydroxy-3-[(2-methylpropyl)(4-hydroxyphenyl)sulfonyl]amino-1S-(phenylmethyl)propylamine was added and stirring continued for 3 hours. The solvent was removed under reduced pressure, ethyl acetate added, washed with saturated sodium bicarbonate, brine, dried over magnesium sulfate, filtered and concentrated to afford 1.37g of crude product. Chromatography on silica gel using 1% methanol/methylene chloride as eluent afforded 830 mg of material which was identified as a mixture of the desired carbamic acid, [2R-hydroxy-3-[[(4-hydroxyphenyl)sulfonyl](2-methylpropyl) amino]-1S-(phenylmethyl)propyl]-, 3-[(6-tert-butyloxycarbonylamino)pyridyl]methyl ester and the cyclic carbamate derived from the 2R-hydroxy-3-[(2-methylpropyl)(4-hydroxyphenyl)sulfonyl]amino-1S-(phenylmethyl)propylamine. The mixture was very difficult to separate, so was used as is in the next step.

### Part B: Preparation of Carbamic acid, [2R-hydroxy-3-[[(4-hydroxyphenyl)sulfonyl](2-methylpropyl)amino]-1S-(phenylmethyl)propyl]-, 3-(6-aminopyridyl)methyl ester

To 830mg of the mixture from part A, was added 50mL of a 1:1 mixture of trifluoroacetic acid and methylene chloride. After 2.5 hours at room temperature, the solvent was removed under reduced pressure, ethyl acetate added, washed with saturated sodium bicarbonate, dried over magnesium sulfate, filtered and concentrated to afford 720 mg of crude material. This was chromatgraphed on silica gel using 5% methanol/ethyl acetate as eluent to yield 220mg of product, which was recrystallized from methylene chloride/diethyl ether to afford 108mg of the desired carbamic acid, [2R-hydroxy-3-[[(4-hydroxyphenyl)sulfonyl](2-methylpropyl) amino]-1S-(phenylmethyl)propyl]-, 3-(6-aminopyridyl)methyl ester, m/e=549(M+Li).

### Example 2C

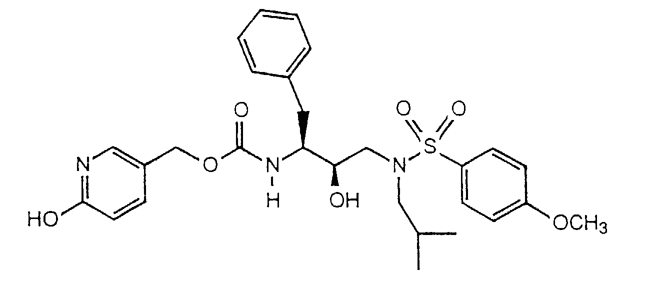

### Preparation of Carbamic acid, [2R-hydroxy-3-[[(4-methoxyphenyl)sulfonyl](2-methylpropyl)amino]-1S-(phenylmethyl)propyl]-, 3-(6-hydroxypyridyl)methyl ester.

### Part A: Preparation of tert-Butyldimethylsilyl 6-(tert-butyldimethylsiloxy)nicotinate

To a solution of 5.0g (35.9mmol) of 6-hydroxynicotinic acid in 200mL of anhydrous N,N-dimethylformamide at room temperature, was added 8.56g (125mmol) of imidazole and then 13.5g (89mmol) of tert-butyldimethylsilyl chloride. After 20 hours, the solvent was removed under reduced pressure, ethyl acetate added, washed with water, 5% citric acid, saturated sodium bicarbonate, brine, dried over anhydrous magnesium sulfate, filtered and concentrated to afford 10.5 g of crude material, m/e=368(M+H).

### Part B: Preparation of 3-(6-tert-butyldimethylsiloxy) pyridylcarbinol

To 11mL of 1M solution of lithium aluminum hydride in diethyl ether at -35°C under a nitrogen atmosphere, was added a solution of 2.0g (5.46mmol) of product from part A in 20mL of anhydrous diethyl ether. After 30 minutes, the reaction was warmed to 0°C and stirred for 40 minutes. The solution was then quenched by the careful addition of 0.42mL of water, 0.42mL of 20% sodium hydroxide solution, and 0.84mL of ater. Ethyl acetate was added, the precipate filtered and the organic phase concentrated to yield 0.93 g of crude 3-(6-tert-butyldimethylsiloxy)pyridylcarbinol, which was used directly in the next step.

### Part C: Preparation of Carbamic acid, [2R-hydroxy-3-[[(4-methoxyphenyl)sulfonyl](2-methylpropyl)amino]-1S-(phenylmethyl)propyl]-, 3-(6-hydroxypyridyl)methyl ester

To a solution of 860mg (3.6mmol) of material from part B in 15mL of anhydrous acetonitrile, was added 919mg (3.6mmol) of N,N'-disuccinimidyl carbonate and 0.87mL of pyridine. After 1 hour, 1.42g(3.5mmol) of 2R-hydroxy-3-[(2-methylpropyl)(4-methoxyphenyl)sulfonyl]amino-1S-(phenylmethyl)propylamine was added. After 14 hours at room temperature, the solvent was removed under reduced pressure, the residue disslved in ethyl acetate, washed with 5% citric acid, saturated sodium bicarbonate, brine, dried over magnesium sukfate, filtered and concentrated to afford 2.1 g of crude material. This was directly deprotected by dissolving in 40mL of 80% acetic acid/water and stirring for 2 hours. The solvents were removed under reduced pressure, the residue dissolved in ethyl acetate, washed with saturated sodium bicarbonate, brine, dried over magnesium sulfate, filtered and concentrated to afford 1.7g of crude product. This was chromatographed on silica gel using 50-100% ethyl acetate/hexane to provide a fraction of 0.19g of fairly pure material, which was further purified by reverse phase chromatography using 15-40% acetonitrile/water (0.05% trifluoroacetic acid) to provide 120mg of the desired carbamic acid, [2R-hydroxy-3-[[(4-methoxyphenyl)sulfonyl](2-methylpropyl)amino]-1S-(phenylmethyl)propyl]-, 3-(6-hydroxypyridyl)methyl ester, m/e=558 (M+H).

### Example 2D

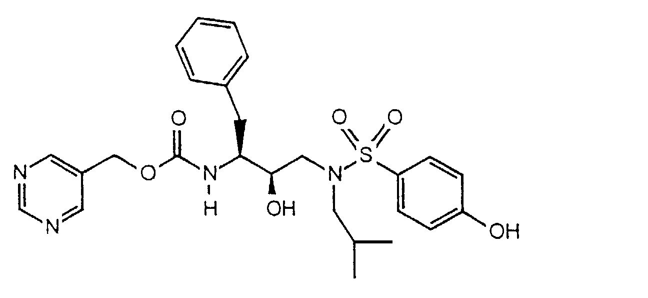

### Preparation of Carbamic acid, [2R-hydroxy-3-[[(4-hydroxyphenyl)sulfonyl](2-methylpropyl)amino]-1S-(phenylmethyl)propyl], 5-pyrimidylmethyl ester.

To a solution of 237mg (2.15mmol) of 5-pyrimidylcarbinol in 24mL of anhydrous acetonitrile , was added 602mg (2.35mmol) of N,N'-disuccinimidyl carbonate and then 0.47mL of pyridine. After stirring for 4.5 hours, 766mg (1.96mmoll of 2R-hydroxy-3-[(2-methylpropyl)(4-hydroxyphenyl)sulfonyl]amino-1S-(phenylmethyl)propylamine was added. After stirring for 19 hours, the solvent was removed under reduced pressure, ethyl acetate added, washed with 5% citric acid, saturated sodium bicarbonate, brine, dried over anhydrous magnesium sulfate, filtered and concentrated to afford 1.0 g of crude material. Chromatography on silica gel using 50-100% ethyl acetate/hexane as eluent afforded 450 mg of the desired carbamic acid, [2R-hydroxy-3-[[(4-hydroxyphenyl) sulfonyl](2-methylpropyl)amino]-1S-(phenylmethyl)propyl]-, 5-pyrimidylmethyl ester, m/e=529 (M+H).

### EXAMPLE 2E

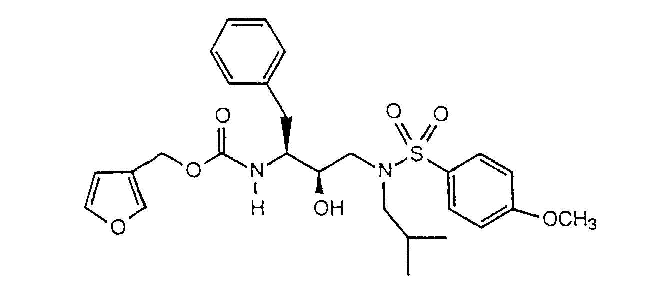

### Preparation of Carbamic acid, [2R-hydroxy-3-[[(4-methoxyphenyl)sulfonyl](2-methylpropyl)amino]-1S-(phenylmethyl)propyl-, 3-furanylmethyl ester

To a solution of 98mg (1 mmol) of 3-(hydroxymethyl)furan in 3 mL of anhydrous acetonitrile, was added 242 µL of pyridine and then 256 mg of N,N'-disuccinimidyl carbonate at room temperature under nitrogen. After 45 minutes, 406 mg (1 mmol) of 2R-hydroxy-3-[(2-methylpropyl)(4-methoxyphenyl)sulfonyl]amino-1S-(phenylmethyl)propylamine was added. After stirring at room temperature for 16 hours, ethyl acetate was added, washed with 5% citric acid, saturated sodium bicarbonate and brine, dried over magnesium sulfate, filtered and concentrated to afford 565 mg of crude product. This was chromatographed on silica gel using 50% ethyl acetate/hexane as eluent to afford 305mg of a white foam, which was recrystallized from diethyl ether/hexane to yield 245 mg of pure carbamic acid, [2R-hydroxy-3-[[(4-methoxyphenyl)sulfonyl) (2-methylpropyl)amino]-1S-(phenylmethyl)propyl-, 3-furanylmethyl ester, m/e = 537(M+Li).

### Example 3A

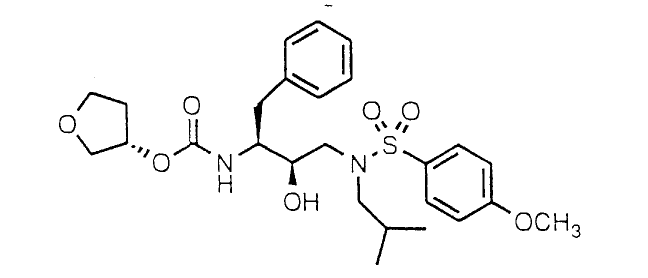

### Preparation of Carbamic acid, [2R-hydroxy-3-[(4-methoxyphenylsulfonyl)(2-methylpropyl)amino]-1S-(phenylmethyl)propyl-, tetrahydrofuran-3S-yl ester,

To a solution of 406 mg (1.0 mmol) of [2R-hydroxy-3-[[(4-methoxyphenyl)sulfonyl](2-methylpropyl)amino]-1S-(phenylmethyl)propylamine in 5.0 mL of dichloromethane containing 150 mg (1.5mmol) of triethylamine was added 280mg (1.22 mmol) of N-succinimidyl-3S-tetrahydrofuranyl carbonate and the reacton mixture was stirred for 2 hours, an additonal 136 mg (0.3mmol) of amine was added to the mixture and the solution stirred another 2 hours. The contents were diluted with 50 mL of ethyl acetate and washed with 5% aqueous citric acid, saturated sodium bicarbonate, and brine, then dried over magnesium sulfate, filtered and concentrated to yield 330 mg of crude product. Purification by silica gel chromatography using an eluant of 1:1 to 2:1 ethyl acetate/hexanes gradient provided Carbamic acid, [2R-hydroxy-3-[(4-methoxyphenylsulfonyl)(2-methylpropyl)amino]-1S-(phenylmethyl)propyl-, tetrahydrofuran-3S-yl ester as a white solid. m/z = 521 (M+H) calc. 521.2311 obs. 521.2311.

### Example 3B

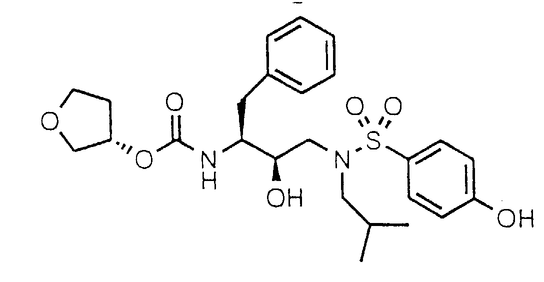

### Preparation of Carbamic acid, [2R-hydroxy-3-[(4-hydroxyphenylsulfonyl)(2-methylpropyl)amino]-1S-(phenylmethyl)propyl-, tetrahydrofuran-3S-yl ester,

To a solution of 435 mg (1.0 mmol) of [2R-hydroxy-3-[[(4-hydroxyphenyl)sulfonyl](2-methylpropyl)amino]-1S-(phenylmethyl)propylamine in 3.0 mL of dimethylformamide was added 225mg (0.98 mmol) of N-succinimidyl-3S-tetrahydrofuranylcarbonate and the solution was stirred overnight. The mixture was diluted with 50 mL of ethyl acetate and washed with 5% aqueous citric acid, saturated sodium bicarbonate, and brine, dried over magnesium sulfate, filtered and concentrated to yield 515 mg of crude product. Purificaton by silica gel chromatography using and eluant of 1:1 ethyl acetate: hexanes provided 315 mg of Carbamic acid, [2R-hydroxy-3-[(4-hydroxyphenylsulfonyl)(2-methylpropyl)amino]-1S-(phenylmethyl)propyl-, tetrahydrofuran-3S-yl ester, as a white solid. HRMS talc. 507.2165, obs. 507.2155.

### Example 3C

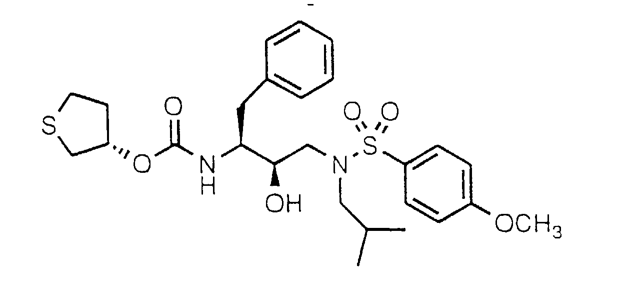

### Preparation of Carbamic acid, [2R-hydroxy-[(4-methoxyyphenylsulfonyl)(2-methylpropyl)amino]-1S-(phenylmethyl)propyl-, tetrahydrothiophen-3S-yl-ester,

To a solution of 215 mg (2.0mmol) of 35-hydroxythiophene, 415 µL of anhydrous pyridine,and 2 mL of dry acetonitrile was added 512 mg (2.0 mmol) of N,N'-Dimethylsuccinimidyl carbonate and this suspension was stirred for 45 minutes. To this clear solution was added a solution of 700 mg (1.7 mmol) of [2R-hydroxy- 3-[[(4-methoxyyphenyl) sulfonyl](2-methylpropyl)amino]-1S-(phenylmethyl) propylamine in 2.0 mL of acetonitrile and stirred for 12 hours. The contents were concentrated, and the residue was partitioned between ethyl acetate and 5% aqueous potassium hydrogen sulfate. The organic layer was washed with saturated sodium bicarbonate and then brine, dried over sodium sulfate, filtered and concentrated to yield 780 mg of crude material. Purificaton by silica gel chromatograpy using an eluant of 10:10:1 ethyl acetate: hexane:methanol provided 520 mg of Carbamic acid, [2R-hydroxy-3-[(4-methoxyphenylsulfonyl)(2-methylpropyl) amino]-1S-(phenylmethyl)propyl-, tetrahydrothiophen-3S-yl-ester, as a crystalline white solid. m.p.= 162-3°C, m/z= 553 (M+H).

### Example 3D

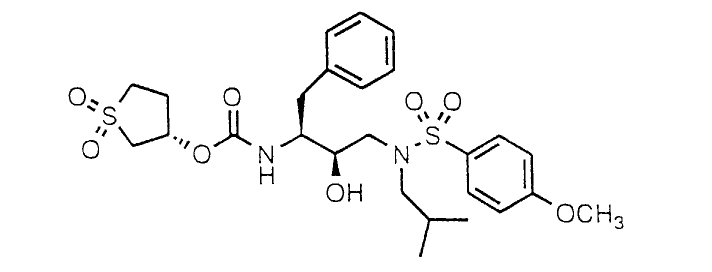

### Preparation of Carbamic acid, [2R-hydroxy-3-[(4-methoxyphenylsulfonyl)(2-methylpropyl)amino]-1S-(phenylmethyl)propyl-, 1,1-dioxotetrahydrothiophen-3S-yl ester

To a solution of 270 mg (0.5 mmol) of Carbamic acid, [2R-hydroxy-3-[(4-methoxyphenylsulfonyl)(2-methylpropyl) amino]-1S-(phenylmethyl)propyl-, tetrahydrothiophen-3S-yl ester in 30 mL of dichloromethane was added 400 mg (1.2 mmol) of m-chloroperbenzoic acid (50 wt%) and the mixture was stirred for 12 hours. The contents were diluted with 10 mL of 10% aqueous sodium metabisulfite and stirred for 30 minutes. The organic layer was washed with saturated sodium bicarbonate, dried over sodium sulfate, filtered and concentrated to yield 290 mg of crude product. Purification by silica gel chromatography using an eluant of 10:10:1 ethyl acetate:hexane:methanol provided 260 mg of Carbamic acid, [2R-hydroxy-3-[(4-methoxyphenyl sulfonyl)(2-methylpropyl)amino]-1S-(phenylmethyl)propyl-, 1,1-dioxotetrahydrothiophen-3S-yl ester, as a white crystalline solid. m.p.= 69 ° C, m/z = 569 (M+H).

### Example 3E

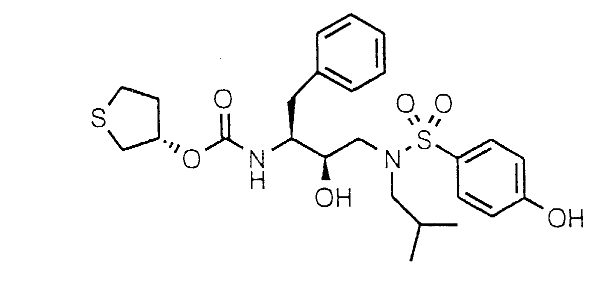

### Preparation of Carbamic acid, [2R-hydroxy-3-[(4-hydroxyphenylsulfonyl)(2-methylpropyl)amino]-1S-(phenylmethyl)propyl-, tetrahydrothiophen-3S-yl ester,

To a solution of 125 mg (1.2 mmol) of 3-S-hydroxythiophene, 250 µL of anhydrous pyridine,and 1 mL of dry acetonitrile was added 307 mg (1.2 mmol) of N,N'-dimethylsuccinimidyl carbonate and this suspension was stirred for 45 minutes. To this clear solution was added a solution of 445 mg (1.0 mmol) [2R-hydroxy-3-[[(4-hydroxyphenyl)sulfonyl](2-methylpropyl)amino]-1S-(phenylmethyl)propylamine in 1.0 mL of acetonitrile and stirred for 12 hours. The contents were concentrated, and the residue was partitioned between ethyl acetate and 5% aqueous potassium hydrogen sulfate. The organic layer was washed with saturated sodium bicarbonate and then brine, dried over sodium sulfate, filtered and concentrated to yield 460 mg of crude material. Purificaton by silica gel chromatograpy using an eluant of 10:10:1 ethyl acetate: hexane:methanol provided 235 mg of Carbamic acid, [2R-hydroxy-3-[(4-hydroxyphenyl sulfonyl)(2-methylpropyl)amino]-1S-(phenylmethyl)propyl-, tetrahydrothiophen-3S-yl ester, as a crystalline white solid. m.p.= 184-85°C , m/z = 529 (M+Li).

### Example 3F

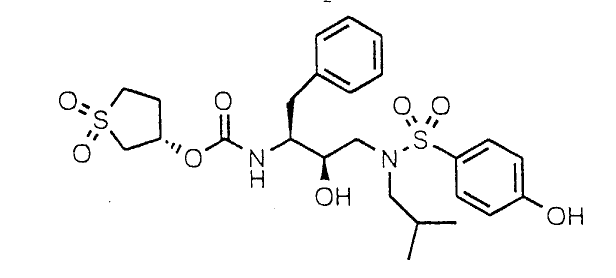

### Preparation of Carbamic acid, [2R-hydroxy-3-[(4-hydroxyphenylsulfonyl)(2-methylpropyl)amino]-1S-(phenylmethyl)propyl-, 1,1-dioxotetrahydrothiophen-3S-yl ester

To a solution of 125 mg (0.24 mmol) of carbamic acid, [2R-hydroxy-3-[(4-hydroxyphenylsulfonyl)(2-methylpropyl) amino]-1S-(phenylmethyl)propyl-, tetrahydrothiophen-3S-yl ester in 30 mL of dichloromethane was added 240 mg (0.7 mmol) of m-chloroperbenzoic acid (50 wt%) and the mixture was stirred for 12 hours. The contents were diluted with 5 mL of 10% aqueous sodium metabisulfite and stirred for 30 minutes. The organic layer was washed with saturated sodium bicarbonate, dried over sodium sulfate, filtered and concentrated to yield 110 mg of crude product. 0 Purification by silica gel chromatography using an eluant of 1:1 to 2:1 ethyl acetate:hexane:methanol provided 100 mg of carbamic acid, [2R-hydroxy-3-[(4-hydroxyphenyl sulfonyl)(2-methylpropyl)amino]-1S-(phenylmethyl)propyl-, 1,1-dioxotetrahydrothiophen-3S-yl-ester, as a white crystalline solid, m.p.= 190°C, m/z= 561 (M+Li).

### Example 4A

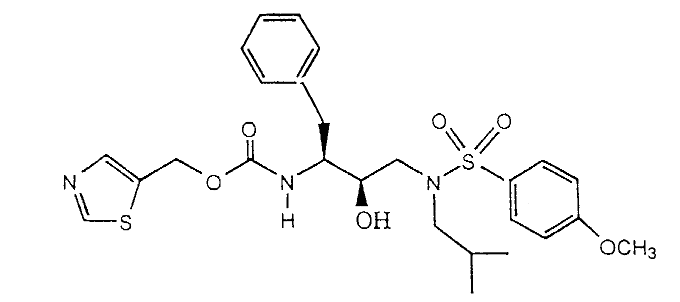

### Preparation of Carbamic acid, [2R-hydroxy-3-[[(4-methoxyphenyl)sulfonyl](methylpropyl)amino]-1S-(phenylmethyl)propyl]-, 5-thiazolylmethyl ester

### Part A: Preparation of Methyl 2-aminothiazole-5-carboxylate

Methyl chloroacetate 190 g (1.75 mol) and methyl formate 111 g (1.80 mol), were added dropwise to a suspension of 100 g (1.8 moL) of sodium methoxide in 450 mL of dry toluene at 5°C over 2 hours. After an additional 2.5 hours at 0°C. The contents were diluted with 600 mL of water and the layers separated. The aqueous phase was acidified with 113 mL of concentrated hydrochloric acid. The aqueous solution was placed in a 2 liter flask and 175 grams of thiourea was added and to solution was heated to reflux for 1.45 hours. To the cooled solution was added 25 g of DARCO activated charcoal and filtered through filter paper. The crude dark yellow solution was neutralized with 2.5 N sodium hydroxide upon which time an amber solid precipitated which was filtered and air dried to yield 147 g of desired methyl 2-aminothiazole-5-carboxylate. m/e = 159(M+H).

### Part B: Preparation of Methyl 5-thiazolecarboxylate

To a solution of 35 mL (30.5 g, 260 mmol) of isoamyl nitrite in 120 mL of dioxane at 80°C under nitrogen, was slowly added a slurry of 20.0 g (126 mmol) of methyl 2-amino-5-thiazolecarboxylate over a 45 minute period. After refluxing for a further 1 hour, the solution was cooled, concentrated, dissolved in ethyl acetate, washed with saturated sodium bicarbonate, brine, dried over magnesium sulfate, filtered and concentrated to afford 28 g of the crude product. This was chromatographed on 400 g of silica gel using 20% ethyl acetate/hexane to afford 9.07 g of purified material, which was crystallized from methylene chloride/hexane to yield 7.64g of pure methyl 5-thiazolylcarboxylate, m/e=144(M+H).

### Part C: Preparation of 5-thiazolemethanol

To a solution of 11.73 g (82 mmol) of methyl 5-thiazolylcarboxylate in 105 mL of anhydrous tetrahydrofuran at 0°C under nitrogen, was added 90 mL (90 mmol) of a 1.0M lithium aluminum hydride solution in diethyl ether over a 35 minute period. After stiiring at room temperature for 30 minutes, the solution was cooled to 0°C, and carefully quenched by the addition of 3 mL of water, 3 mL of 20% sodium hydroxide solution, and 6 mL of water, then 100mL of tetrahydrofuran was added. After stirring for 1 hour, the mixture was filtered, the solid was washed with tetrahydrofuran, and the filtrate concentrated to afford 7.56 g of 5-thiazolylmethanol.

### Part D: Preparation of Carbamic acid, [2R-hydroxy-3-[[(4-methoxyphenyl)sulfonyl](methylpropyl)amino-1S-(phenylmethyl)propyl]-, 5-(thiazolyl)methyl ester

To a solution of 115 mg (1.00 mmol) of 5-(hydroxymethyl) thiazole in 3 mL of anhydrous acetonitrile, was added 0.25 mL (0.25 g, 3.09 mmol) of pyridine and then 256 mg (1.03 mmol) of N,N'-disuccinimidyl carbonate at room temperature under nitrogen. After 45 minutes, 406 mg (1.00 mmol) of 2R-hydroxy-3-[(2-methylpropyl)(4-methoxyphenyl)sulfonyl]amino-1S-(phenylmethyl)propylamine was added. After stirring at room temperature for 15 hours, ethyl acetate was added, washed with water, saturated sodium bicarbonate and brine, dried over magnesium sulfate, filtered and concentrated to afford 500mg of crude product. This was chromatographed on silica gel using 80% ethyl acetate/hexane as eluent to afford 307 mg of a white solid, which was identified as the desired carbamic acid, [2R-hydroxy-3-[[(4-methoxyphenyl)sulfonyl](2-methylpropyl)amino]-1S-(phenylmethyl)propyl-, 5-thiazolylmethyl ester, m/e = 548 (M+H).

### Example 4B

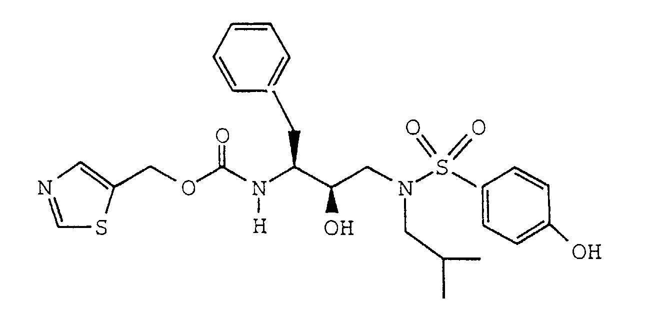

### Preparation of Carbamic acid, [2R-hydroxy-3-[[(4-hydroxyphenyl)sulfonyl](2-methylpropyl)amino]-1S-(phenylmethyl)propyl-, 5-thiazolylmethyl ester

To a solution of 115 mg (1.00 mmol) of 5-(hydroxymethyl) thiazole in 3 mL of anhydrous acetonitrile, was added 0.25 mL (0.25 g, 3.09 mmol) of pyridine and then 256 mg (1.03 mmol) of N,N'-disuccinimidyl carbonate at room temperature under nitrogen. After 45 minutes, 392 mg (1.00 mmol) of 2R-hydroxy-3-[(2-methylpropyl)(4-hydroxyphenyl)sulfonyl]amino-1S-(phenylmethyl)propylamine was added. After stirring at room temperature for 15 hours, ethyl acetate was added, washed with water, saturated sodium bicarbonate and brine, dried over magnesium sulfate, filtered and concentrated to afford 450 mg of crude product. This was chromatographed on silica gel using 80% ethyl acetate/hexane as eluent to afford 270 mg of a white solid, which was identified as the desired carbamic acid, [2R-hydroxy-3-[[(4-hydroxyphenyl) sulfonyl](2-methylpropyl)amino]-1S-(phenylmethyl)propyl-, 5-thiazolylmethyl ester, m/e =534 (M+H).

### Example 5A

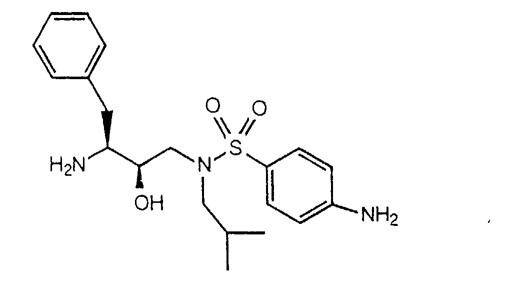

### Preparation of 2R-hydroxy-3-[[(4-aminophenyl)sulfonyl] (2-methylpropyl)amino]-1S-(phenylmethyl)propylamine

### Part A: Preparation of Carbamic acid, 2-hydroxy-3-[[(4-nitrophenyl)sulfonyl](2-methylpropyl)amino]-1S-(phenylmethyl)propyl-, phenylmethyl ester

To a solution of 4.0 g (10.8 mmol) of N-[3S-benzyloxy carbonylamino-2R-hydroxy-4-phenyl]-N-isobutylamine in 50mL of anhydrous methylene chloride, was added 4.5mL (3.27g, 32.4 mmol) of triethylamine. The solution was cooled to 0°C and 2.63g (11.9 mmol) of 4-nitrobenzene sulfonyl chloride was added, stirred for 30 minutes at 0°C, then for 1 hour at room temperature. Ethyl acetate was added, washed with 5% citric acid, saturated sodium bicarbonate, brine, dried and concentrated to yield 5.9 g of crude material. This was recrystallized from ethyl acetate/hexane to afford 4.7 g of pure carbamic acid, [2R-hydroxy-3-[[(4-nitrophenyl)sulfonyl](2-methylpropyl) amino]-1S-(phenylmethyl)propyl-, phenylmethyl ester, m/e=556(M+H).

### Part B: Preparation of 2R-hydroxy-3-[[(4-aminophenyl)sulfonyl](2-methylpropyl)amino]-1S-(phenylmethyl) propylamine

A solution of 3.0g (5.4 mmol) of carbamic acid, 2R-hydroxy-3-[[(4-nitrophenyl)sulfonyl](2-methylpropyl) amino]-1S-(phenylmethyl)propyl-, phenylmethyl ester in 20 mL of ethyl acetate was hydrogenated over 1.5 g of 10% palladium-on-carbon catalyst under 35 psig of hydrogen for 3.5 hours. The catalyst was removed by filtration and the solution concentrated to afford 2.05 g of the desired 2R-hydroxy-3-[[(4-aminophenyl)sulfonyl](2-methylpropyl)amino]-1S-(phenylmethyl)propylamine, m/e=392(M+H).

### Example 5B

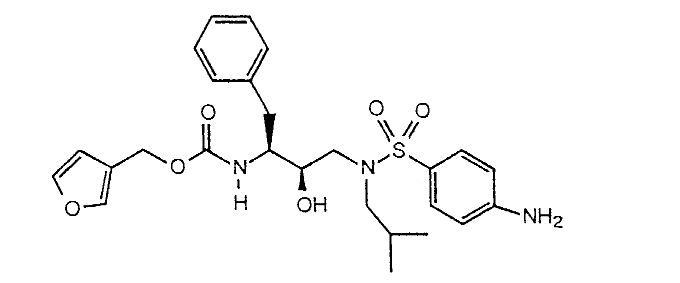

### Preparation of Carbamic acid, 2R-hydroxy-3-[[(4-aminophenyl)sulfonyl](2-methylpropyl)amino]-1S-(phenylmethyl)propyl-, 3-furanylmethyl ester

To a solution of 104mg (1.06 mmol) of 3-(hydroxymethyl) furan in 2 mL of anhydrous acetonitrile, was added 0.26 mL (0.25g, 3.18 mmol) of pyridine and then 277 mg (1.06 mmol) of N,N'-disuccinimidyl carbonate at room temperature under nitrogen. After 45 minutes, 415 mg (1.06 mmol) of 2R-hydroxy-3-[(2-methylpropyl)(4-aminophenyl)sulfonyl]amino-1S-(phenylmethyl)propylamine was added. After stirring at-room temperature for 72 hours, ethyl acetate was added, washed with 5% citric acid, saturated sodium bicarbonate and brine, dried over magnesium sulfate, filtered and concentrated to afford 550 mg of crude product. This was chromatographed on silica gel using 50% ethyl acetate/hexane as eluent to afford 230 mg of a white foam, which was identified as the desired carbamic acid, 2R-hydroxy-3-[[(4-aminophenyl)sulfonyl](2-methylpropyl)amino]-1S-(phenylmethyl)propyl-, 3-furanylmethyl ester, m/e = 522 (M+Li).

### Example 5C

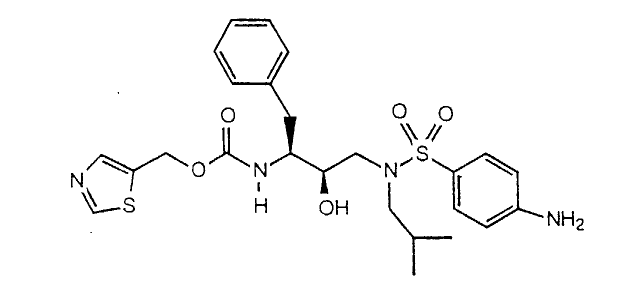

### Preparation of Carbamic acid, 2R-hydroxy-3-[[(4-aminophenyl)sulfonyl](2-methylpropyl)amino]-1S-(phenylmethyl)propyl-, 5-thiazolylmethyl ester

To a solution of 118mg (1.03 mmol) of 5-(hydroxymethyl) thiazole in 3 mL of anhydrous acetonitrile, was added 0.25 mL (0.24g, 3.09 mmol) of pyridine and then 264 mg (1.03 mmol) of N,N'-disuccinimidyl carbonate at room temperature under nitrogen. After 45 minutes, 403 mg (1.03 mmol) of 2R-hydroxy-3-[(2-methylpropyl)(4-aminophenyl)sulfonyl]amino-1S-(phenylmethyl)propylamine was added. After stirring at room temperature for 15 hours, ethyl acetate was added, washed with 5% citric acid, saturated sodium bicarbonate and brine, dried over magnesium sulfate, filtered and concentrated to afford 350 mg of crude product. This was chromatographed on silica gel using 80% ethyl acetate/hexane as eluent to afford 290 mg of a white solid, which was identified as the desired carbamic acid, 2R-hydroxy-3-[[(4-aminophenyl)sulfonyl](2-methylpropyl)amino]-1S-(phenylmethyl)propyl-, 5-thiazolylmethyl ester, m/e = 539 (M+Li).

### Example 5D

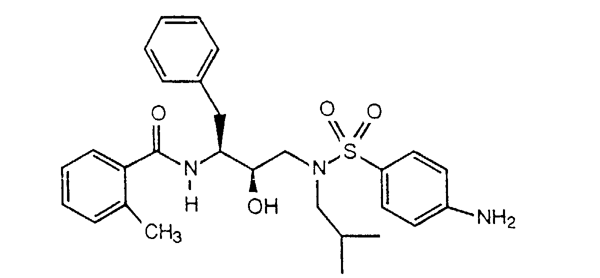

### Preparation of Benzamide, N-[2R-hydroxy-3-[[(4-aminophenyl)sulfonyl](2-methylpropyl)amino]-1S-(phenylmethyl)propyl]-2-methyl

To a solution of 391 mg (1 mmol) of 2R-hydroxy-3-[(2-methylpropyl)(4-aminophenyl)sulfonyl]amino-1S-(phenylmethyl)propylamine in 3 mL of anhydrous methylene chloride, was added 0.42 mL (3 mmol) of triethylamine, then at room temperature, 0.12 mL (0.9 mmol) of ortho-toluoyl chloride was added. After 15 hours at room temp ethyl acetate was added, washed with 5% citric acid, saturated sodium bicarbonate, brine, dried, filtered and concentrated to afford 420 mg of crude material. This was chromatographed on 40 g of silica gel using 50% ethyl acetate/hexane to afford 368 mg of pure benzamide, N-[2R-hydroxy-3-[[(4-aminophenyl)sulfonyl](2-methylpropyl) amino]-1S-(phenylmethyl)propyl]-2-methyl, m/e=516(M+LI).

### Example 5E

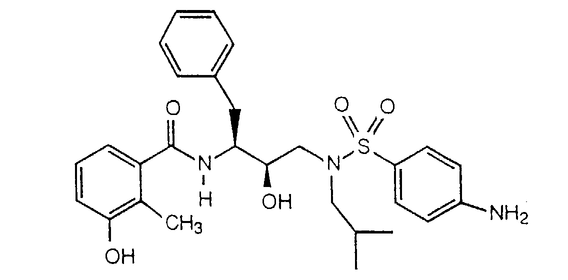

### Preparation of Benzamide, N-[2R-hydroxy-3-[[(4-aminophenyl)sulfonyl](2-methylpropyl)amino]-1S-(phenylmethyl)propyl]-3-hydroxy-2-methyl

### Part A: Preparation of 3-Hydroxy-2-methylbenzoic Acid

A one-necked 100 mL round-bottomed flask (magnetic stirring) was charged with 1.0 gram (6.6 mM) 3-amino-2-methylbenzoic acid. A warm mixture of 2.3 mL conc. sulfuric acid in 4.3 mL water was added to the flask, the resulting slurry was cooled below 15°C in an ice bath, and 6.6 grams of ice was added. The reaction mixture was treated via subsurface addition with a solution of 0.6 gram (8.6 mM) sodium nitrite in 6.6 mL ice water with the reaction temperature maintained at 0-5°C during the addition. After stirring at 0-5°C for 30 min., a few crystals of urea were added to decompose the excess nitrite. The reaction mixture was then poured into a room temperature solution of 23.8 grams (102.3 mM) copper (II) nitrate hemipentahydrate in 200 mL water. With vigorous stirring, the reaction mixture was treated with 0.9 gram (6.0 mM) copper (I) oxide. The reaction mixture foamed and changed from turquoise blue to dark green in color. Reaction was left stirring for 30 min. The reaction mixture was extracted with diethyl ether (3X), and the organic extracts were combined. The organic extracts were concentrated to approximately one-fourth the original volume, then extracted with 25 mL 1N sodium hydroxide solution. The layers were separated, and the dark-red aqueous layer was acidified to pH=2 using 1N hydrochloric acid solution. The acidified aqueous layer was then extracted with diethyl ether (3X), and the ether extracts were combined, dried (MgSO4), and concentrated to yield a reddish-colored oil. Purification by flash chromatography on silica gel using a gradient of 0-7% methanol/methylene chloride afforded 0.39 grams (36%) of a yellow solid.

### Part B: Preparation of Benzamide, N-[2R-hydroxy-3-[[(4-aminophenyl)sulfonyl](2-methylpropyl)amino]-1S-(phenylmethyl)propyl]-3-hydroxy-2-methyl

To a solution of 175 mg (1.15 mmol) of 3-hydroxy-2-methylbenzoic acid and 203 mg (1.5 mmol) of N-hydroxybenzotriazole in 6 mL of anhydrous N,N-dimethylformamide at 0°C, was added 220 mg (1.15 mmol) of EDC. After 20 minutes of activation at 0°C and 1 hour at room temperature, 392 mg (1.0 mmol) of 2R-hydroxy-3-[(2-methylpropyl)(4-aminophenyl)sulfonyl]amino-1S-(phenylmethyl)propylamine was added. After 15 hours at room temperature, ethyl acetate was added, washed with 5% citric acid, saturated sodium bicarbonate, brine, dried, filtered and concentrated to afford 590 mg of crude material. This was chromatographed on silica gel using 50-80% ethyl acetate/methylene chloride as eluent to afford 255 mg of pure benzamide, N-[2R-hydroxy-3-[[(4-aminophenyl)sulfonyl](2-methylpropyl)amino]-1S-(phenylmethyl)propyl]-3-hydroxy-2-methyl, m/e = 526(M+H).

### Example 5F

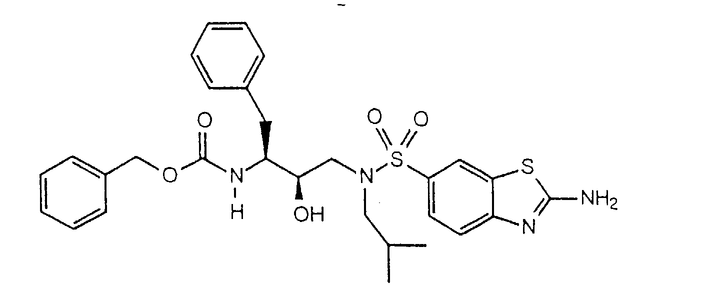

### Preparation of Carbamic acid, 2R-hydroxy-3-[[(2-aminobenzothiazol-6-yl)sulfonyl](2-methylpropyl)amino]-1S-(phenylmethyl)propyl-, phenylmethyl ester

Carbamic acid, 2R-hydroxy-3-[[(4-aminophenyl)sulfonyl](2-methylpropyl)amino]-1S-(phenylmethyl)propyl-, phenylmethyl ester 0.30 g (0.571 mmol) was added to a well mixed powder of anhydrous copper sulfate (1.20 g) and potassium thiocyanate (1.50 g) followed by dry methanol (6 mL) and the resulting black-brown suspension was heated at reflux for 2 hrs. The reaction mixture was filtered and the filtrate was diluted with water (5 mL) and heated at reflux. Ethanol was added to the reaction mixture, cooled and filtered. The filtrate upon concentration afforded a residue which was chromatographed (ethyl acetate:hexane 80:20) to afford 0.26 g (78%) of the desired compound as a solid.

### Example 5G

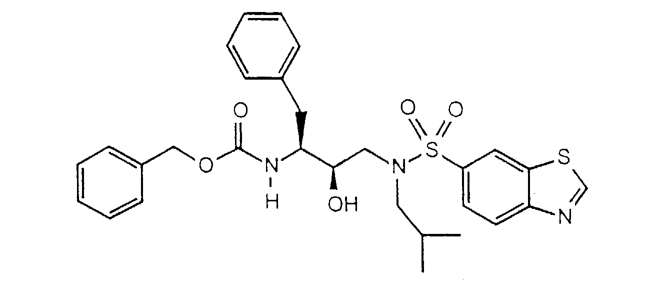

### Preparation of Carbamic acid, 2R-hydroxy-3-[[(benzothiazol-6-yl)sulfonyl](2-methylpropyl)amino]-1S-(phenylmethyl)propyl-, phenylmethyl ester

Carbamic acid, 2R-hydroxy-3-[[(2-aminobenzothiazol-6-yl)sulfonyl](2-methylpropyl)amino]-1S-(phenylmethyl) propyl-, phenylmethyl ester (0.25 g, 0.429 mmol) was added to a solution of isoamylnitrite (0.116 mL, 0.858 mmol) in dioxane (5 mL) and the mixture was heated at 85°C. After the cessation of evolution of nitrogen, the reaction mixture was concentrated and the residue was purified by chromatography (hexane:ethyl acetate 5:3) to afford 0.130 g (53%) of the desired product as a solid.

### Example 6A

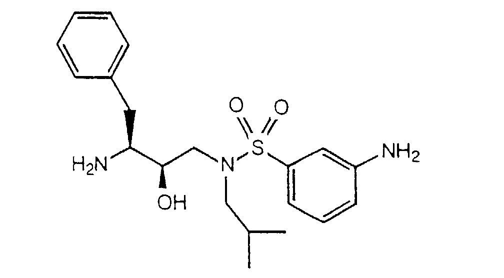

### Preparation of 2R-hydroxy-3-[[(3-aminophenyl)sulfonyl](2-methylpropyl)amino]-1S-(phenylmethyl)propylamine

### Part A: Preparation of Carbamic acid, [2R-hydroxy-3-[(3-nitrophenylsulfonyl)(2-methylpropyl)amino]-1S-(phenylmethyl)propyl-, phenylmethyl ester

To a solution of 1.1 g (3.0 mmol) of N-[3S-benzyloxy carbonylamino-2R-hydroxy-4-phenyl]-N-isobutylamine in 15mL of anhydrous methylene chloride, was added 1.3mL (0.94g, 9.3 mmol) of triethylamine. The solution was cooled to 0°C and 0.67 g (3.0 mmol) of 3-nitrobenzene sulfonyl chloride was added, stirred for 30 minutes at 0°C, then for 1 hour at room temperature. Ethyl acetate was added, washed with 5% citric acid, saturated sodium bicarbonate, brine, dried and concentrated to yield 1.74 g of crude material. This was recrystallized from ethyl acetate/hexane to afford 1.40 g of pure carbamic acid, [2R-hydroxy-3-[(3-nitrophenylsulfonyl)(2-methylpropyl) amino]-1S-(phenylmethyl)propyl-, phenylmethyl ester, m/e=562 (M+Li).

### Part B: Preparation of [2R-hydroxy-3-[[(3-aminophenyl)sulfonyl](2-methylpropyl)amino]-1S-(phenylmethyl)propylamine

A solution of 1.33g (2.5 mmol) of carbamic acid, [2R-hydroxy-3-[(3-nitrophenylsulfonyl)(2-methylpropyl)amino]-1S-(phenylmethyl)propyl-, phenylmethyl ester in 40 mL of 1:1 methanol/tetrahydrofuran was hydrogenated over 0.70 g of 10% palladium-on-carbon catalyst under 40 psig of hydrogen for 1.5 hours. The catalyst was removed by filtration and the solution concentrated to afford 0.87 g of the desired [2R-hydroxy-3-[[(3-aminophenyl)sulfonyl] (2-methylpropyl)amino]-1S-(phenylmethyl)propylamine.

### Example 6B

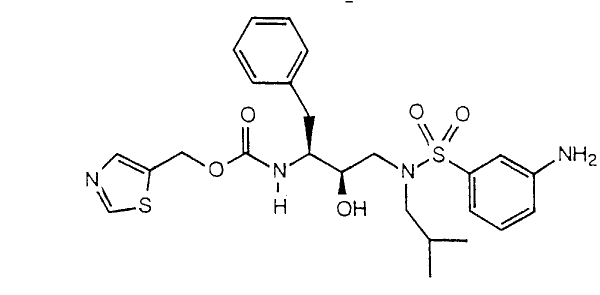

### Preparation of Carbamic acid, 2R-hydroxy-3-[[(3-aminophenyl)sulfonyl](2-methylpropyl)amino]-1S-(phenylmethyl)propyl-, 5-thiazolylmethyl ester

To a solution of 133mg (1.15 mmol) of 5-(hydroxymethyl) thiazole in 3 mL of anhydrous acetonitrile, was added 0.30 mL (0.29g, 3.7 mmol) of pyridine and then 296 mg (1.15 mmol) of N,N'-disuccinimidyl carbonate at room temperature under nitrogen. After 60 minutes, 460 mg (1.18 mmol) of 2R-hydroxy-3-[(2-methylpropyl)(3-aminophenyl)sulfonyl]amino-1S-(phenylmethyl)propylamine was added. After stirring at room temperature for 15 hours, ethyl acetate was added, washed with 5% citric acid, saturated sodium bicarbonate and brine, dried over magnesium sulfate, filtered and concentrated to afford 480 mg of crude product. This was chromatographed on silica gel using 50-80% ethyl acetate/hexane as eluent to afford 422 mg of a white solid, which was identified as the desired carbamic acid, 2R-hydroxy-3-[(3-aminophenyl sulfonyl)(2-methylpropyl)amino]-1S-(phenylmethyl)propyl-, 5-thiazolylmethyl ester, m/e = 539 (M+Li).

### Example 6C

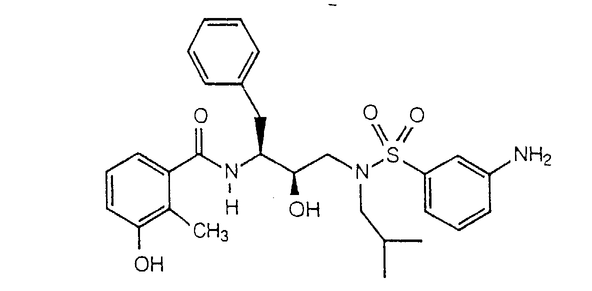

### Preparation of Benzamide, N-[2R-hydroxy-3-[[(3-aminophenyl)sulfonyl](2-methylpropyl)amino]-1S-(phenylmethyl)propyl]-3-hydroxy-2-methyl

To a solution of 134 mg (0.88 mmol) of 3-hydroxy-2-methylbenzoic acid and 155 mg (1.15 mmol) of N-hydroxybenzotriazole in 5 mL of anhydrous N,N-dimethylformamide at 0°C, was added 167 mg (0.88 mmol) of EDC. After 20 minutes of activation at 0°C and 1 hour at room temperature, 300 mg (1.0 mmol) of 2R-hydroxy-3-[(2-methylpropyl)(3-aminophenyl)sulfonyl]amino-1S-(phenylmethyl)propylamine was added. After 15 hours at room temperature, ethyl acetate was added, washed with 5% citric acid, saturated sodium bicarbonate, brine, dried, filtered and concentrated to afford 330 mg of crude material. This was chromatographed on silica gel using 30-70% ethyl acetate/methylene chloride as eluent to afford 230 mg of pure benzamide, N-[2R-hydroxy-3-[[(3-aminophenyl)sulfonyl](2-methylpropyl)amino]-1S-(phenylmethyl)propyl]-3-hydroxy-2-methyl.

### Example 6D

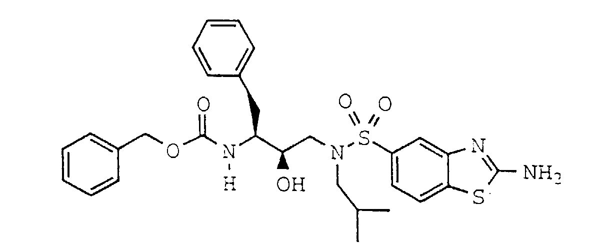

and

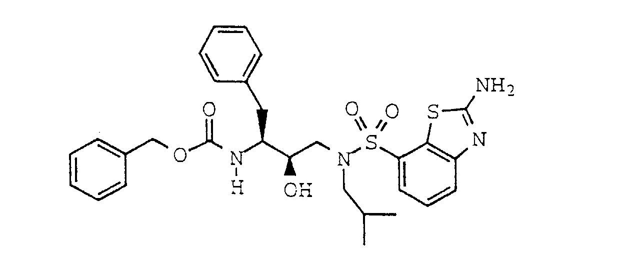

### Preparation of Carbamic acid, 2R-hydroxy-3-[[(2-amino benzothiazol-5-yl)sulfonyl](2-methylpropyl)amino]-1S-(phenylmethyl)propyl-, phenylmethyl ester; and Carbamic acid, 2R-hydroxy-3-[[(2-aminobenzothiazol-7-yl)sulfonyl](2-methylpropyl)amino]-1S-(phenylmethyl)propyl-, phenylmethyl ester

The carbamic acid, 2R-hydroxy-3-[(3-aminophenylsulfonyl) (2-methylpropyl)amino]-1S-(phenylmethyl) propyl-, phenylmethyl ester 0.36 g (0.685 mmol) was added to a well mixed powder of anhydrous copper sulfate (1.44 g) and potassium thiocyanate (1.80 g) followed by dry methanol (10 mL) and the rsulting black-brown suspension was heated at reflux for 2 hrs. The reaction mixture was filtered and the filtrate was diluted with water (5 mL) and heated at reflux. Ethanol was added to the reaction mixture, cooled and filtered. The filtrate upon concentration afforded a rseidue which was chromatographed (ethyl acetate:hexane 1:1) to afford 0.18 g (45%) of the 7-isomer as a solid. Further elution of the column with (ethyl acetate:hexane 3:2) afforded 0.80 g (20%) afforded the 5-isomer-as a solid.

### Example 7A

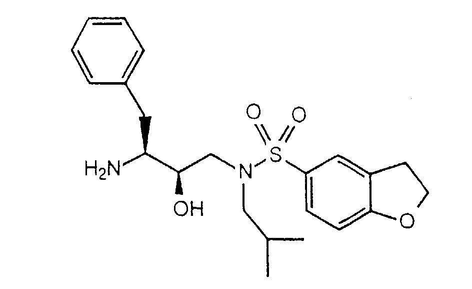

### Preparation of 2R-hydroxy-3-[[(2,3-dihydrobenzofuran-5-yl)sulfonyl](2-methylpropyl)amino]-1S-(phenylmethyl) propylamine

### Part A: Preparation of 5-(2,3-dihydrobenzofuranyl) sulfonyl chloride

To a solution of 3.35g of anhydrous N,N-dimethylformamide at 0°C under nitrogen was added 6.18 g of sulfuryl chloride, whereupon a solid formed. After stirring for 15 minutes, 4.69 g of 2,3-dihydrobenzofuran was added, and the mixture heated at 100°C for 2 hours. The reaction was cooled, poured into ice water, extracted with methylene chloride, dried over magnesium sulfate, filtered and concentrated the crude material. This was recrystallized from ethyl acetate to afford 2.45 g of 5-(2,3-dihydrobenzofuranyl)sulfonyl chloride.

### Part B: Preparation of Carbamic acid, 2R-hydroxy-3-[[(2,3-dihydrobenzofuran-5-yl)sulfonyl](2-methylpropyl) amino]-1S-(phenylmethyl)propyl-, phenylmethyl ester

To a solution of 1.11 g (3.0 mmol) of N-[3S-benzyloxy carbonylamino-2R,-hydroxy-4-phenyl]-N-isobutylamine in 20mL of anhydrous methylene chloride, was added 1.3mL (0.94 g, 9.3 mmoll of triethylamine. The solution was cooled to 0°C and 0.66 g of 5=(2,3-dihydrobenzofuranyl) sulfonyl chloride was added, stirred for 15 minutes at 0°C, then for 2 hour at room temperature. Ethyl acetate was added, washed with 5% citric acid, saturated sodium bicarbonate, brine, dried and concentrated to yield 1.62 g of crude material. This was recrystallized from diethyl ether to afford 1.17 g of pure carbamic acid, [2R-hydroxy-3-[[(2,3-dihydrobenzofuran-5-yl)sulfonyl](2-methylpropyl)amino]-1S-(phenylmethyl)propyl-, phenylmethyl ester.

### Part C: Preparation of [2R-hydroxy-3-[[(2,3-dihydro benzofuran-5-yl)sulfonyl](2-methylpropyl)amino]-1S-(phenylmethyl)propylamine

A solution of 2.86 g of carbamic acid, [2R-hydroxy-3-[[(2,3-dihydrobenzofuran-5-yl)sulfonyl](2-methylpropyl) amino]-1S-(phenylmethyl)propyl-, phenylmethyl ester in 30 mL of tetrahydrofuran was hydrogenated 0.99g of 10% palladium-on-carbon under 50 psig of hydrogen for 16 hours. The catalyst was removed by filtration and the filtrate concentrated to afford 1.99 g of the desired [2R-hydroxy-3-[[(2,3-dihydrobenzofuran-5-yl)sulfonyl](2-methylpropyl)amino]-1S-(phenylmethyl)propylamine.

### Example 7B

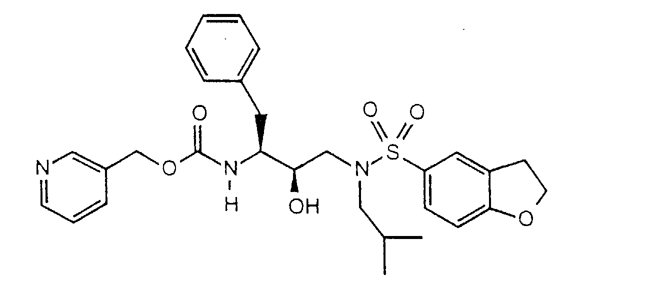

### Preparation of Carbamic acid, [2R-hydroxy-3-[[(2,3-dihydrobenzofuran-5-yl)sulfonyl](2-methylpropyl)amino]-1S-(phenylmethyl)propyl-, 3-pyridylmethyl ester

To a solution of 110 mg of 3-pyridylcarbinol in 3 mL of anhydrous acetonitrile, was added 0.24g of anhydrous pyridine and then 260 mg of N,N'disuccinimidyl carbonate at room temperature under nitrogen. After 45 minutes, 420 mg of 2R-hydroxy-3-[(2-methylpropyl)(2,3-dihydrobenzofuran-5-yl)sulfonyl]amino-1S-(phenylmethyl) propylamine was added. After stirring at room temperature for 20 hours, ethyl acetate was added, washed with 5% citric acid, saturated sodium bicarbonate and brine, dried over magnesium sulfate, filtered and concentrated to afford 320 mg of crude product. This was chromatographed on silica gel using 50% ethyl acetate/hexane as eluent to afford 260 mg of a white solid, which was identified as the desired carbamic acid, [2R-hydroxy-3-[[(2,3-dihydrobenzofuran-5-yl)sulfonyl](2-methylpropyl)amino]-1S-(phenylmethyl)propyl-, 3-pyridylmethyl ester.

### Example 7C

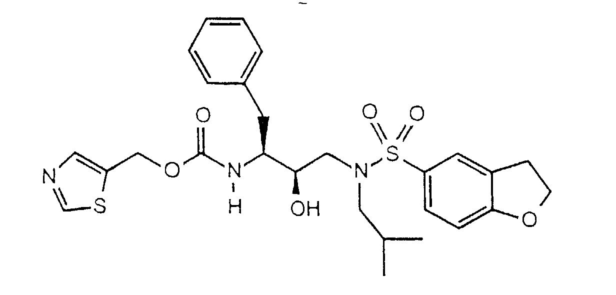

### Preparation of Carbamic acid, [2R-hydroxy-3-[[(2,3-dihydrobenzofuran-5-yl)sulfonyl](2-methylpropyl)amino]-1S-(phenylmethyl)propyl-, 5-thiazolylmethyl ester

To a solution of 66 mg of 5-(hydroxymethyl)thiazole in 3 mL of anhydrous acetonitrile, was added 0.14g of anhydrous pyridine and then 150 mg of N,N'-disuccinimidyl carbonate at room temperature under nitrogen. After 45 minutes, 240 mg of 2R-hydroxy-3-[(2-methylpropyl)(2,3-dihydrobenzofuran-5-yl)sulfonyl]amino-1S-(phenylmethyl) propylamine was added. After stirring at room temperature for 20 hours, ethyl acetate was added, washed with 5% citric acid, saturated sodium bicarbonate and brine, dried over magnesium sulfate, filtered and concentrated to afford 220 mg of crude product. This was chromatographed on silica gel using 50% ethyl acetate/hexane as eluent to afford 120 mg of a white solid, which was identified as the desired carbamic acid, [2R-hydroxy-3-[[(2,3-dihydrobenzofuran-5-yl)sulfonyl](2-methylpropyl)amino]-1S-(phenylmethyl)propyl-, 5-thiazolylmethyl ester.

### Example 7D

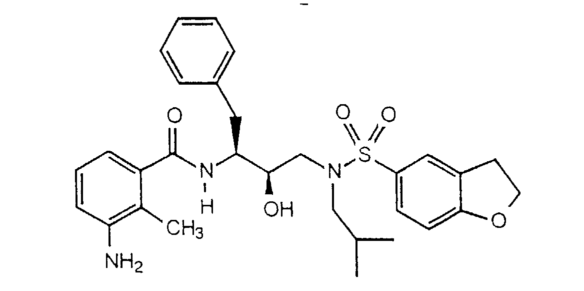

### Preparation of Benzamide, N-[2R-hydroxy-3-[[(2,3-dihydrobenzofuran-5-yl)sulfonyl](2-methylpropyl)amino]-1S-(phenylmethyl)propyl]-3-amino-2-methyl-

To a solution of 175 mg of 3-amino-2-methylbenzoic acid in 2 mL of anhydrous N,N-dimethylformamide at 0°C, was added 200 mg of N-hydroxybenzotriazole and then 210 mg of EDC. After 20 minutes of activation, 405 mg of 2R-hydroxy-3-[[(2,3-dihydrobenzofuran-5-yl)sulfonyl](2-methylpropyl)amino]-1S-(phenylmethyl)propylamine was added. After stirring at room temperature for 16 hours, ethyl acetate was added, wshed with 5% citric acid, sodium bicarbonate, brine, dried over magnesium sulfate, filtered and concentrated to afford 225 mg of crude product. This was chromatographed on silica gel using 50% ethyl acetate/hexane to afford 140 mg of the desired benzamide, N-[2R-hydroxy-3-[[(2,3-dihydrobenzofuran-5-yl)sulfonyl](2-methylpropyl)amino]-1S-(phenylmethyl) propyl]-3-amino-2-methyl, m/e=552 (M+H).

### Example 8A

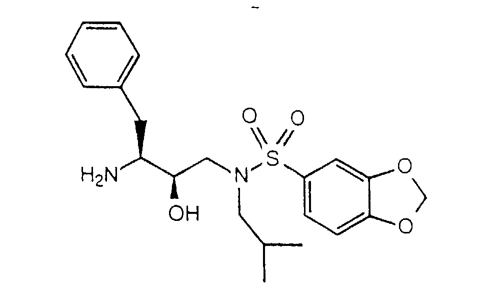

### Preparation of 2R-hydroxy-3-[[(1,3-benzodioxol-5-yl)sulfonyl](2-methylpropyl)amino]-1S-(phenylmethyl) propylamine

### Part A: Preparation of (1,3-Benzodioxol-5-yl)sulfonyl chloride

To a solution of 4.25 g of anhydrous N,N-dimethylformamide at 0°C under nitrogen was added 7.84g of sulfuryl chloride, whereupon a solid formed. After stirring for 15 minutes, 6.45 g of 1,3-benzodioxole was added, and the mixture heated at 100°C for 2 hours. The reaction was cooled, poured into ice water, extracted with methylene chloride, dried over magnesium sulfate, filtered and concentrated to give 7.32 g of crude material as a black oil. This was chromatographed on silica gel using 20% methylene chloride/hexane to afford 1.9 g of (1,3-benzodioxol-5-yl)sulfonyl chloride.

### Part B: Preparation of Carbamic acid, 2R-hydroxy-3-[[(1,3-benzodioxol-5-yl)sulfonyl](2-methylpropyl)amino]-1S-(phenylmethyl)propyl-, phenylmethyl ester

To a solution of 3.19 g(8.6 mmol) of N-[3S-benzyloxy carbonylamino-2R-hydroxy-4-phenyl]-N-isobutylamine in 40mL of anhydrous methylene chloride, was added 0.87g of triethylamine. The solution was cooled to 0°c and 1.90g of (1,3-benzodioxol-5-yl)sulfonyl chloride was added, stirred for 15 minutes an 0°C, then for 17 hours at room temperature. Ethyl acetate was added, washed with 5% citric acid, saturated sodium bicarbonate, brine, dried and concentrated to yield crude material. This was recrystallized from diethyl ether/hexane to afford 4.77 g of pure carbamic acid, 2R-hydroxy-3-[[(1,3-benzodioxol-5-yl)sulfonyl](2-methylpropyl)amino]-1S-(phenylmethyl)propyl-, phenylmethyl ester.

### Part C: Preparation of 2R-hydroxy-3-[[(1,3-benzodioxol-5-yl)sulfonyl](2-methylpropyl)amino]-1S-(phenylmethyl) propylamine

A solution of 4.11 g of carbamic acid, 2R-hydroxy-3-[[(1,3-benzodioxol-5-yl)sulfonyl](2-methylpropyl)amino]-1S-(phenylmethyl)propyl-, phenylmethyl ester in 45 mL of tetrahydrofuran and 25 mL of methanol was hydrogenated over 1.1 g of 10% palladium-on-carbon under 50 psig of hydrogen for 16 hours. The catalyst was removed by filtration and the filtrate concentrated to afford 1.82g of the desired 2R-hydroxy-3-[[(1,3-benzodioxol-5-yl)sulfonyl](2-methylpropyl)amino]-1S(phenylmethyl)propylamine.

### Example 8B

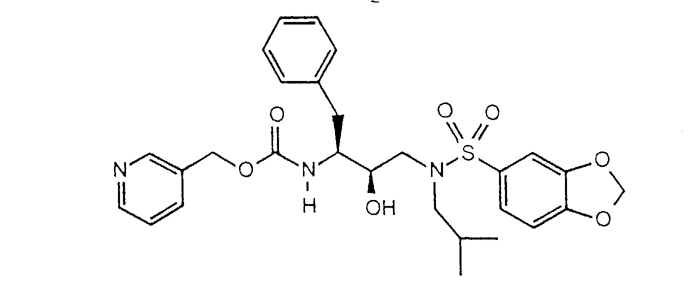

### Preparation of Carbamic acid, 2R-hydroxy-3-[[(1,3-benzodioxol-5-yl)sulfonyl](2-methylpropyl)amino]-1S-(phenylmethyl)propyl-, 3-pyridylmethyl ester

To a solution of 110 mg of 3-pyridylcarbinol in 3 mL of anhydrous acetonitrile, was added 0.24 g of anhydrous pyridine and then 260 mg of N,N'disuccinimidyl carbonate at room temperature under nitrogen. After 45 minutes, 410 mg of 2R-hydroxy-3-[[(1,3-benzodioxol-5-yl)sulfonyl](2-methylpropyl)amino]-1S-(phenylmethyl)propylamine was added. After stirring at room temperature for 20 hours, ethyl acetate was added, washed with 5% citric acid, saturated sodium bicarbonate and brine, dried over magnesium sulfate, filtered and concentrated to afford 330 mg of crude product. This was chromatographed on silica gel using 50% ethyl acetate/hexane as eluent to afford 160 mg of a white solid, which was identified as the desired carbamic acid, [2R-hydroxy-3-[[(1,3-benzodioxol-5-yl) sulfonyl](2-methylpropyl)amino]-1S-(phenylmethyl)propyl-, 3-pyridylmethyl ester, m/e=562(M+Li).

### Example 8C

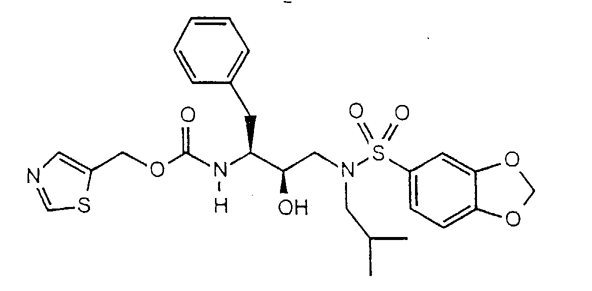

### Preparation of Carbamic acid, 2R-hydroxy-3-[[(1,3-benzodioxol-5-yl)sulfonyl](2-methylpropyl)amino]-1S-(phenylmethyl)propyl-, 5-thiazolylmethyl ester

To a solution of 85 mg (0.8 mmol) of 5-(hydroxymethyl) thiazole in 2.2 mL of anhydrous acetonitrile, was added 0.18 mL (2.2 mmol) of anhydrous pyridine and then 189 mg (0.74 mmol) of N,N'-disuccinimidyl carbonate at room temperature under nitrogen. After 45 minutes, 310 mg of 2R-hydroxy-3-[[(1,3-benzodioxol-5-yl)sulfonyl](2-methylpropyl)amino]-1S-(phenylmethyl)propylamine was added. After stirring at room temperature for 20 hours, ethyl acetate was added, washed with 5% citric acid, saturated sodium bicarbonate and brine, dried over magnesium sulfate, filtered and concentrated to afford 300 mg of crude product. This was chromatographed on silica gel using 50% ethyl acetate/hexane as eluent to afford 150 mg of a white solid, which was identified as the desired carbamic acid, 2R-hydroxy-3-[[(1,3-benzodioxol-5-yl)sulfonyl](2-methylpropyl)amino]-1S-(phenylmethyl)propyl-, 5-thiazolylmethyl ester, m/e=568(M+Li).

### Example 8D

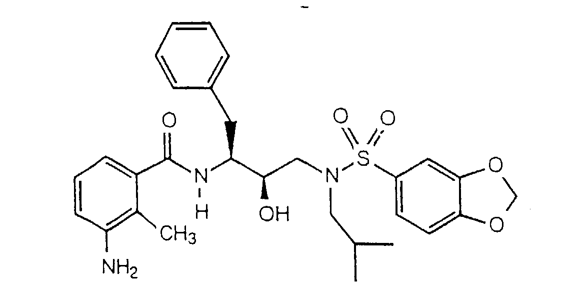

### Preparation of Benzamide, N-[2R-hydroxy-3-[[(1,3-benzodioxol-5-yl)sulfonyl](2-methylpropyl)amino]-1S-(phenylmethyl)propyl]-3-amino-2-methyl

To a solution of 175 mg of 3-amino-2-methylbenzoic acid in 2 mL of anhydrous N,N-dimethylformamide at 0°C, was added 200 mg of N-hydroxybenzotriazole and then 210 mg of EDC. After 20 minutes of activation, 410 mg of 2R-hydroxy-3-[[(1,3-benzodioxol-5-yl)sulfonyl](2-methylpropyl)amino]-1S-(phenylmethyl)propylamine was added. After stirring at room temperature for 16 hours, ethyl acetate was added, washed with 5% citric acid, sodium bicarbonate, brine, dried over magnesium sulfate, filtered and concentrated to afford 500 mg of crude product. This was chromatographed on silica gel using 50% ethyl acetate/hexane to afford 310 mg of the desired benzamide, N-[2R-hydroxy-3-[[(1,3-benzodioxol-5-yl)sulfonyl](2-methylpropyl)amino]-1S-(phenylmethyl)propyl]-3-amino-2-methyl, m/e=560 (M+Li).

### Example 8 E

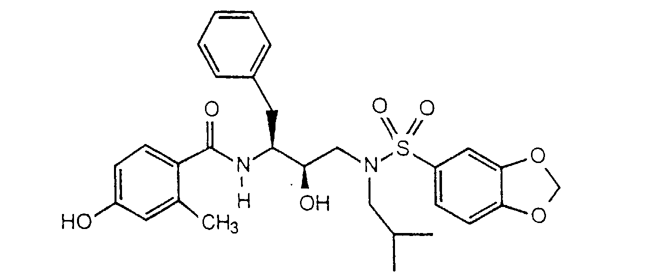

### Preparation of Benzamide, N-[2R-hydroxy-3-[[(1,3-benzodioxol-5-yl)sulfonyl](2-methylpropyl)amino]-1S-(phenylmethyl)propyl]-4-hydroxy-2-methyl

### Part A: Preparation of 2-Trimethylsilyloxy-1,3-cyclohexadiene

A 100 mL round bottom flask equipped with magnetic stir bar, addition funnel, and N₂ inlet was charged with 40 mL dry THF and 8.3 mL diisopropyl amine. The solution was cooled to -78°C and charged with 23.8 mL of 2.5M nBuLi in Hexane. After 10 minutes a solution of 5.2 g cyclohexenone in 10 mL THF was added dropwise. The reaction was stirred 10 minutes at -78°C and quenched with 7.5 mL trimethylsilyl chloride. The reaction was stirred 15 minutes and then partitioned between diethyl ether and cold saturated aqueous sodium bicarbonate. The combined organic layers were dried over sodium sulfate and concentrated in vacuo to a yellow oil. Short path distillation (BP 27-29°C/0.5mm) afforded 6.0 g (66%) of 2-Trimethylsilyloxy-1,3-Cyclohexadiene.

### Part B: Preparation of Methyl (2-methyl-4-trimethylsilyoxy)benzoate

A 50 mL round bottom flask equipped with magnetic stir bar and condenser was charged with 6.0 g of 2-trimethylsilyloxy-1,3-cyclohexadiene, 3.5 g methyl tetrolate in 3 mL dry toluene. The reaction was heated to 150°C for 50 hours at which point 1H-NMR indicated no starting diene. The raction was concentrated in vacuo to provide 5.7 g (67%) methyl 2-methyl-4-trimethylsilyloxybenzoate.

### Part C: Preparation of 4-Hydroxy-2-methylbenzoic acid

A 100mL round bottom flask equipped with magnetic stir bar was charged with 5.7 g methyl 2-methyl-4-trimethylsilyloxybenzoate and 2.0 g LiOH in 40 mL methanol and 10 mL water. After 2 hours at reflux the reaction was poured into 10 mL concentrated HCl and then 100 g ice. Extraction with ethyl acetate followed by concentration in vacuo gave a crude solid (70:30) product:starting material. Flash Chromatography using 50-50 ethyl acetate/hexanes as an eluent gave 1.15 g 2-methyl-4-hydroxybenzoic acid, m/e=193 (M+H).

### Part D: Preparation of Benzamide, N-[2R-hydroxy-3-[[(1,3-benzodioxol-5-yl)sulfonyl](2-methylpropyl)amino]-1S-(phenylmethyl) propyl]-4-hydroxy-2-methyl

To a solution of 175 mg of 4-hydroxy-2-methylbenzoic acid in 2 mL of anhydrous N,N-dimethylformamide at 0°C, was added 200 mg of N-hydroxybenzotriazole and then 220 mg of EDC. After 20 minutes of activation, 450 mg of 2R-hydroxy-3-[[(1,3-benzodioxol-5-yl)sulfonyl](2-methylpropyl)amino]-1S-(phenylmethyl)propylamine was added. After stirring at room temperature for 16 hours, ethyl acetate was added, wshed with 5% citric acid, sodium bicarbonate, brine, dried over magnesium sulfate, filtered and concentrated to afford crude product. This was chromatographed on silica gel using 50% ethyl acetate/hexane to afford 102 mg of the desired benzamide, N-[2R-hydroxy-3-[[(1,3-benzodioxol-5-yl)sulfonyl](2-methylpropyl)amino]-1S-(phenylmethyl)propyl]-4-hydroxy-2-methyl.

### Example 8F

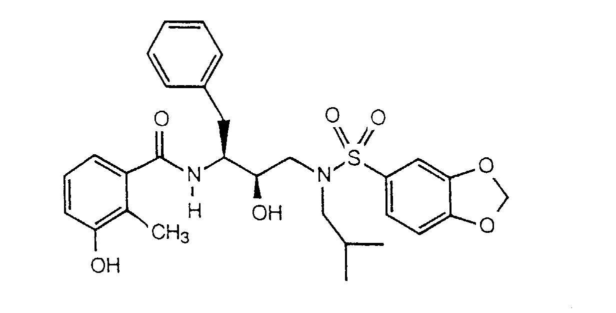

### Preparation of Benzamide, N-[2R-hydroxy-3-[[(1,3-benzodioxol-5-yl)sulfonyl](2-methylpropyl)amino]-1S-(phenylmethyl)propyl]-3-hydroxy-2-methyl

To a solution of 187 mg (1.23 mmol) of 3-hydroxy-2-methylbenzoic acid and 217 mg (1.61 mmol) of N-hydroxybenzotriazole in 6 mL of anhydrous N,N-dimethylformamide at 0°C, was added 236 mg (1.23 mmol) of EDC. After 20 minutes of activation at 0°C and 1 hour at room temperature, 450 mg (1.07 mmol) of 2R-hydroxy-3-[[(1,3-benzodioxol-5-yl)sulfonyl](2-methylpropyl)amino]-1S-(phenylmethyl)propylamine was added. After 15 hours at room temperature, ethyl acetate was added, washed with 5% citric acid, saturated sodium bicarbonate, brine, dried, filtered and concentrated to afford 650 mg of crude material. This was chromatographed on silica gel using 0-25% ethyl acetate/methylene chloride as eluent to afford 390 mg of pure benzamide, N-[2R-hydroxy-3-[[(1,3-benzodioxol-5-yl)sulfonyl](2-methylpropyl) amino]-1S-(phenylmethyl)propyl]-3-hydroxy-2-methyl, m/e=561 (M+Li).

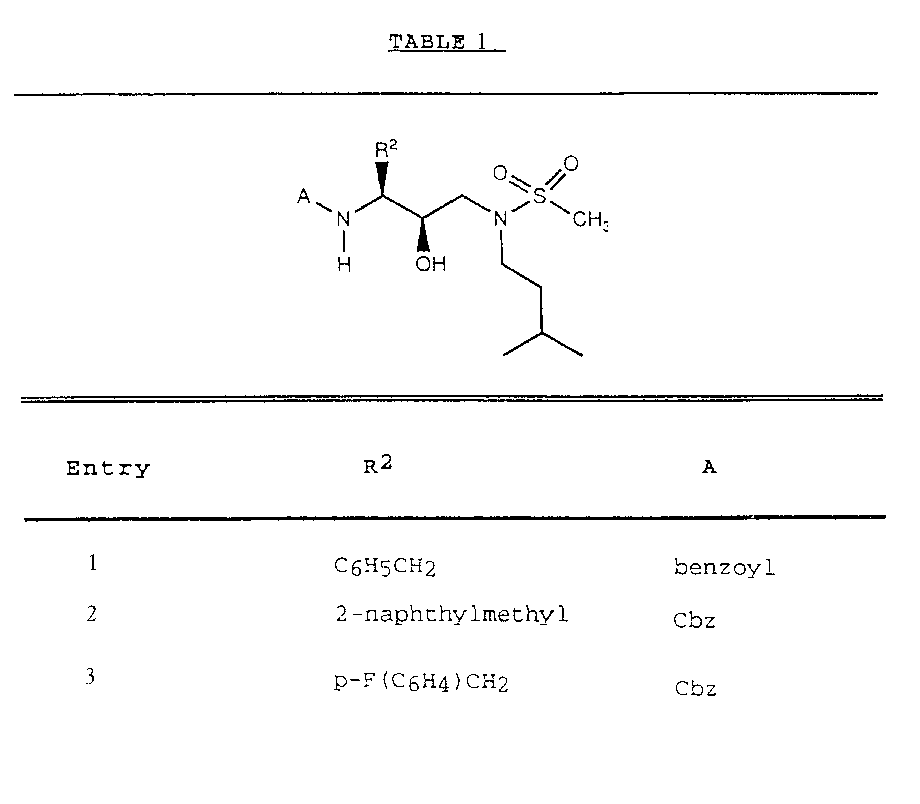

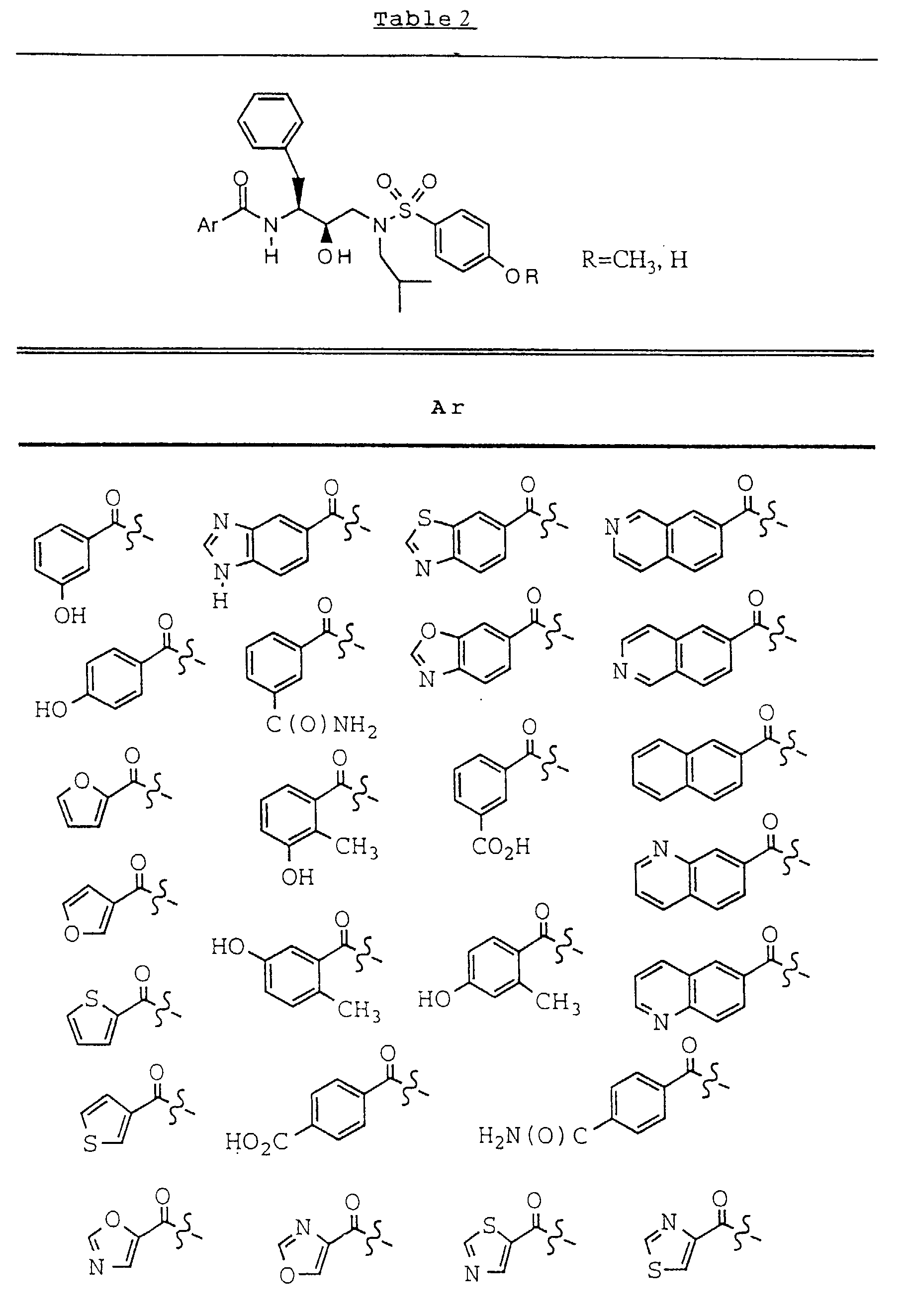

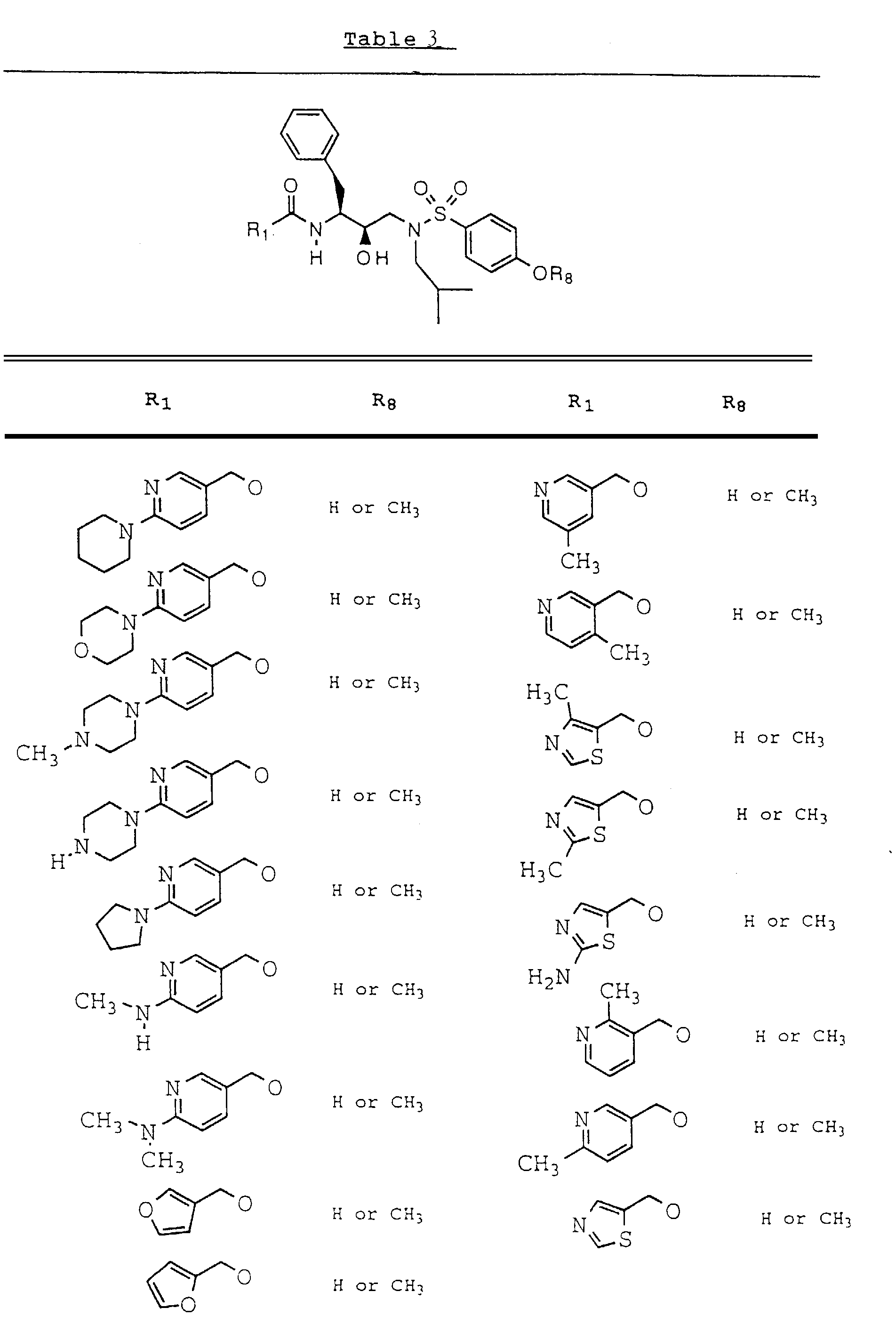

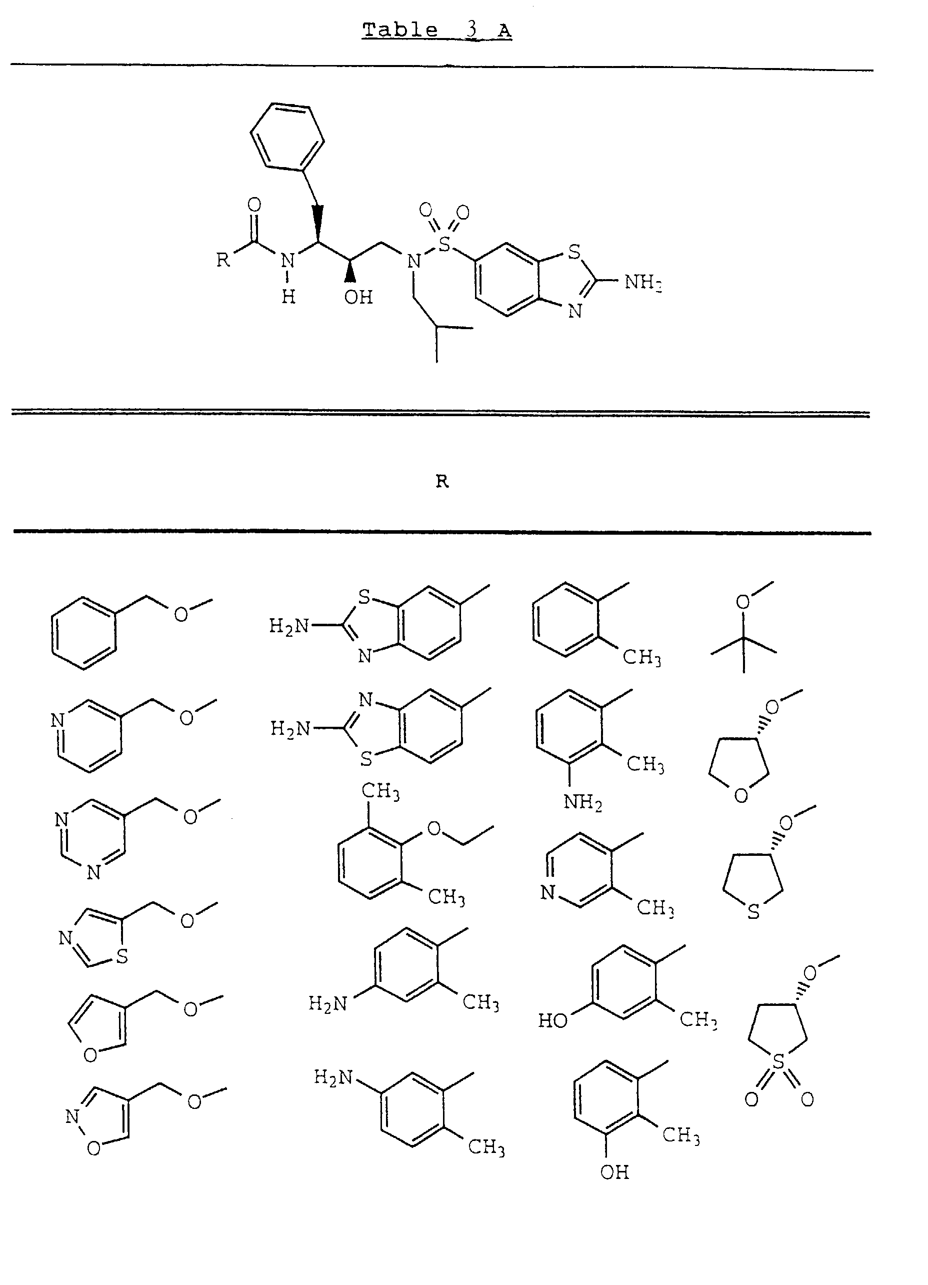

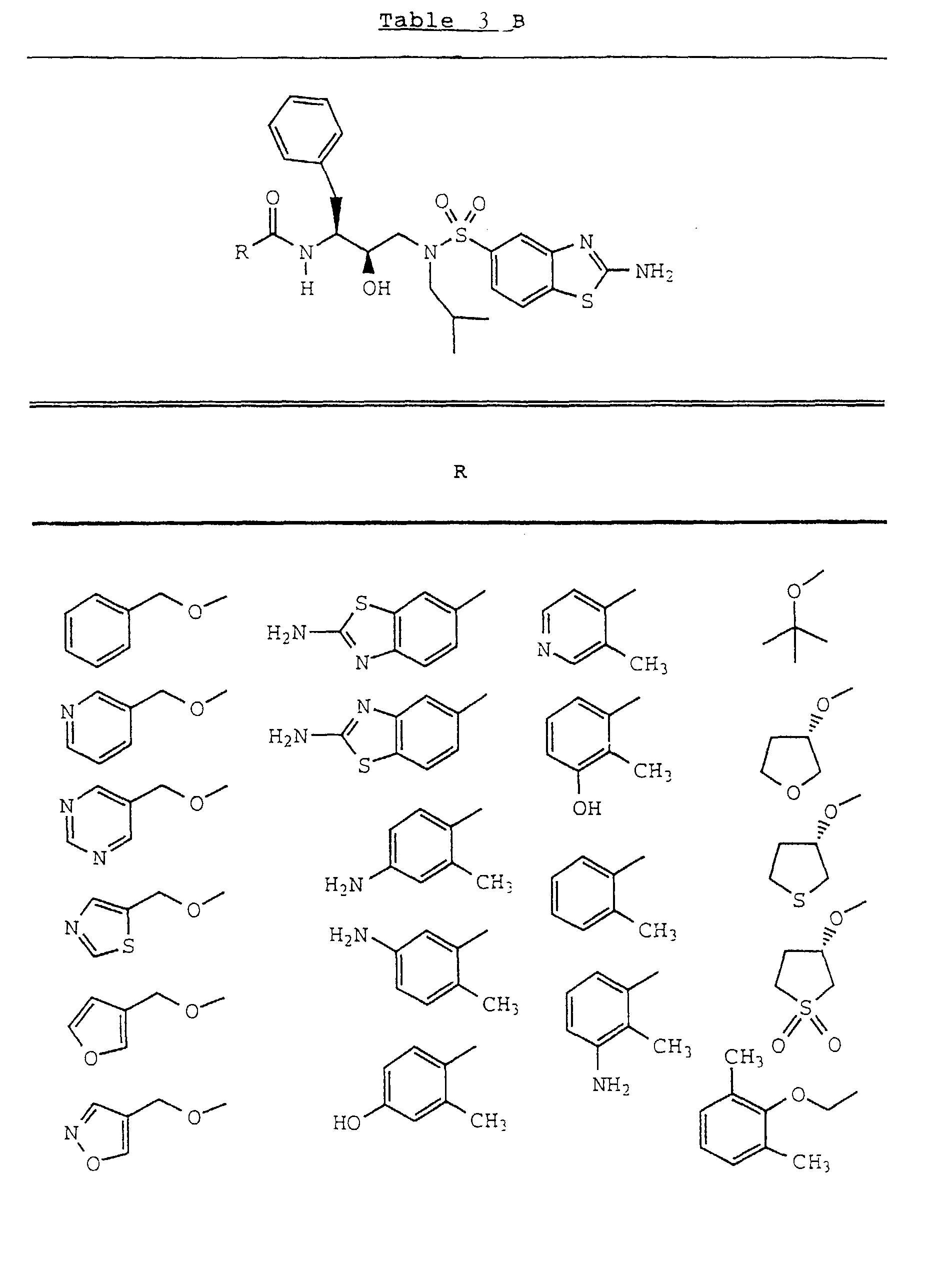

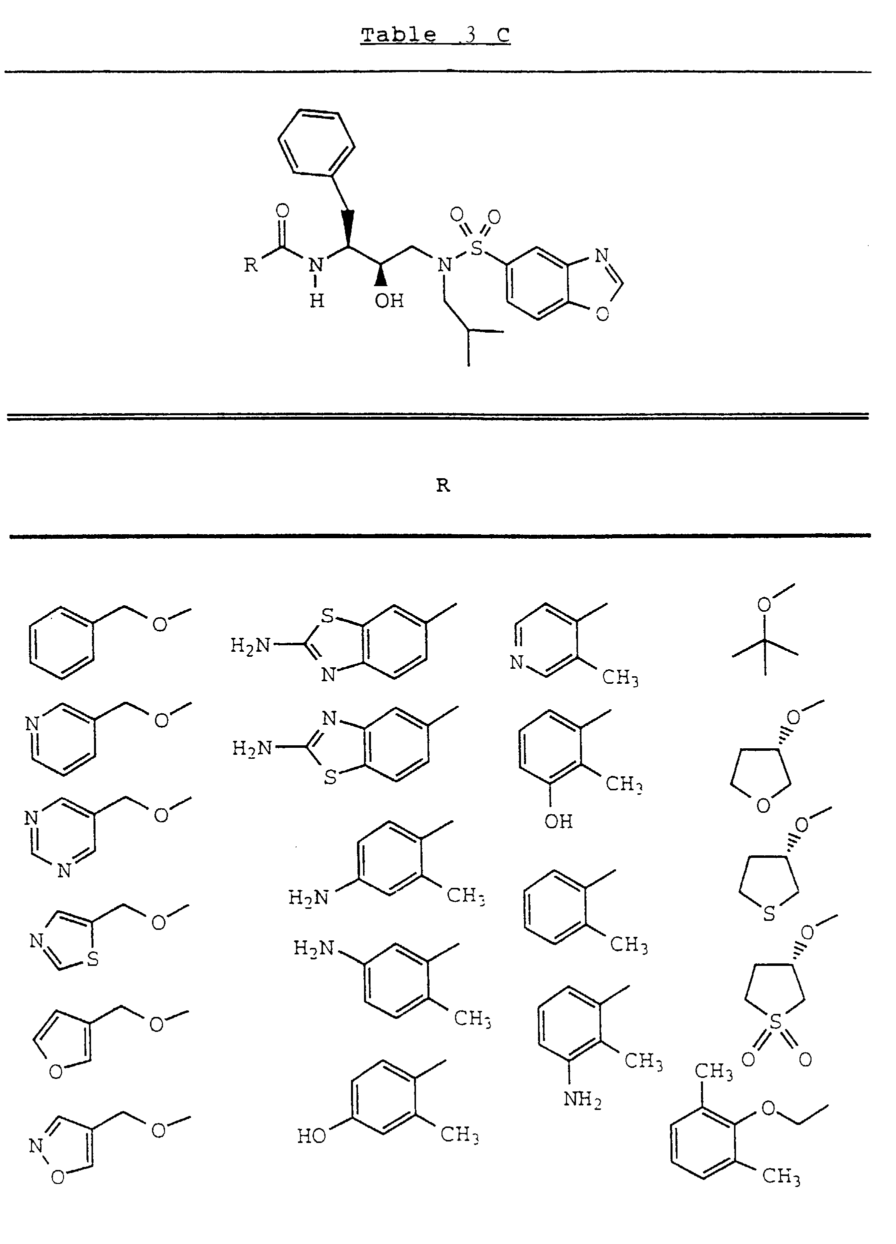

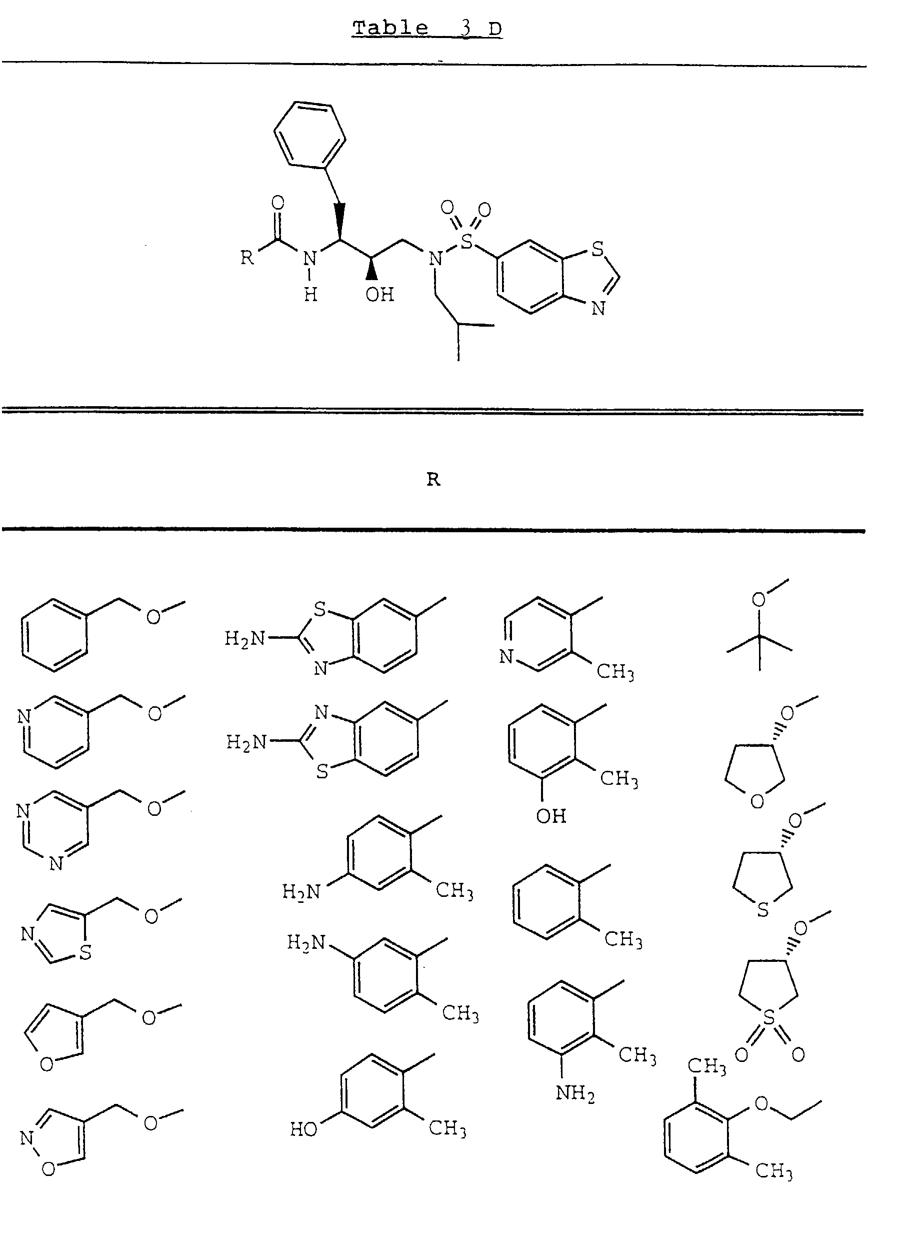

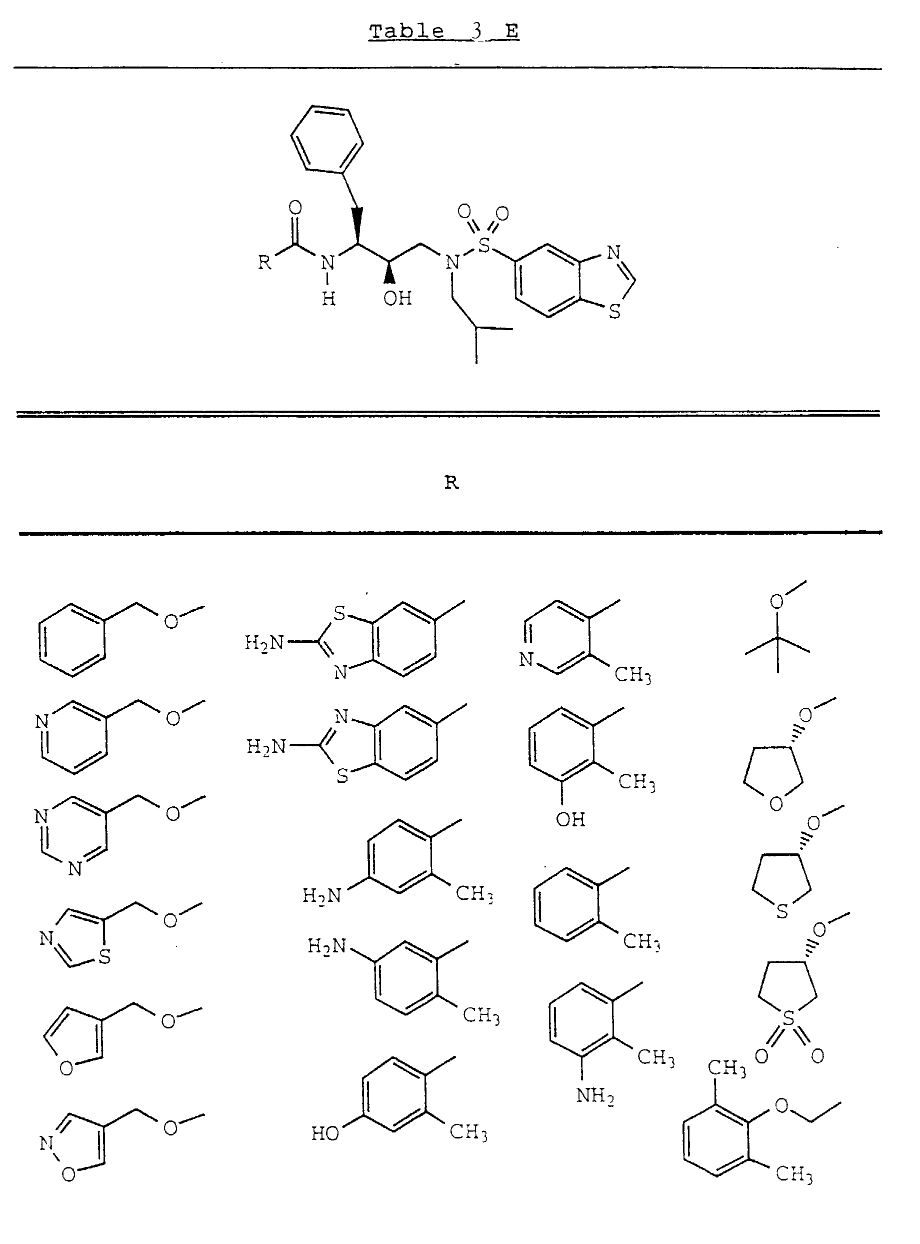

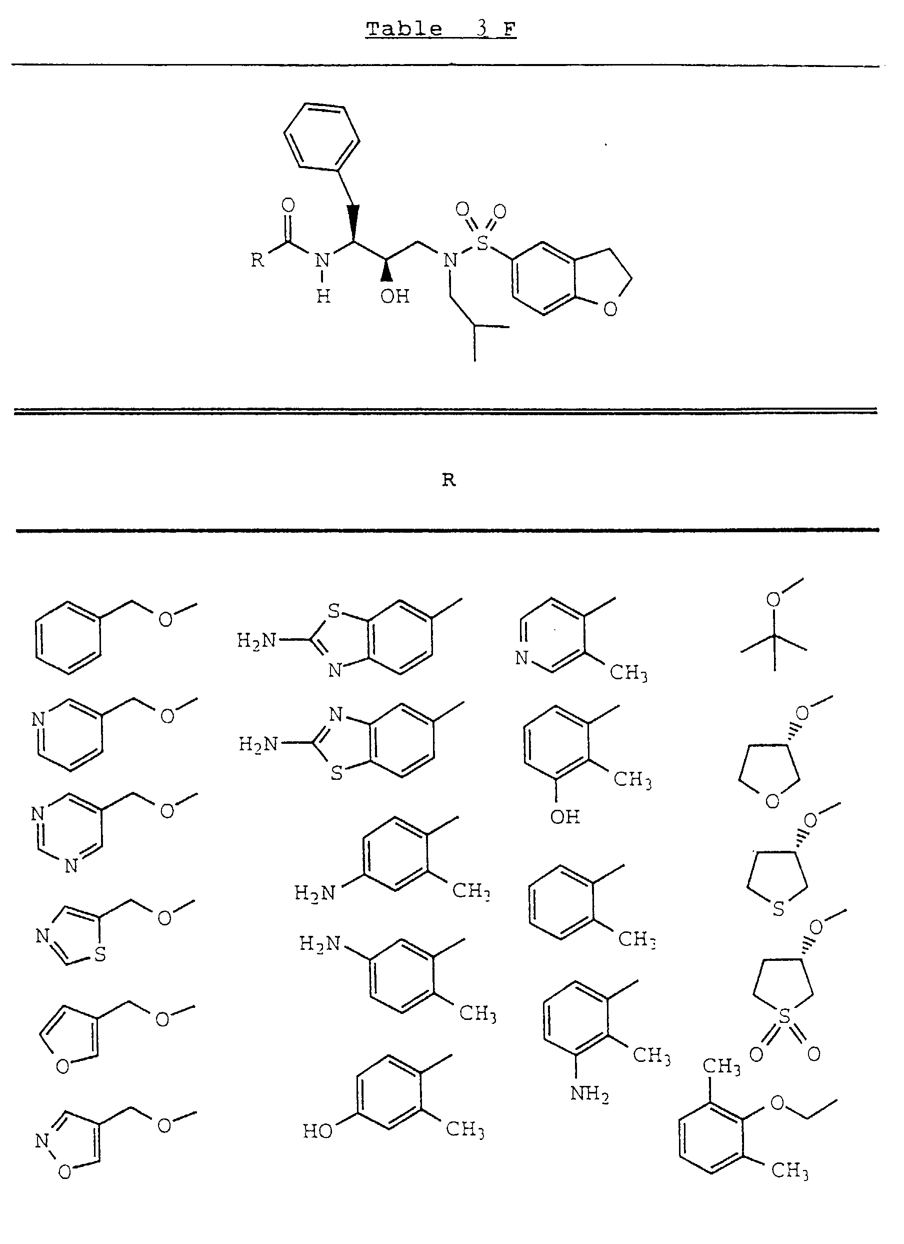

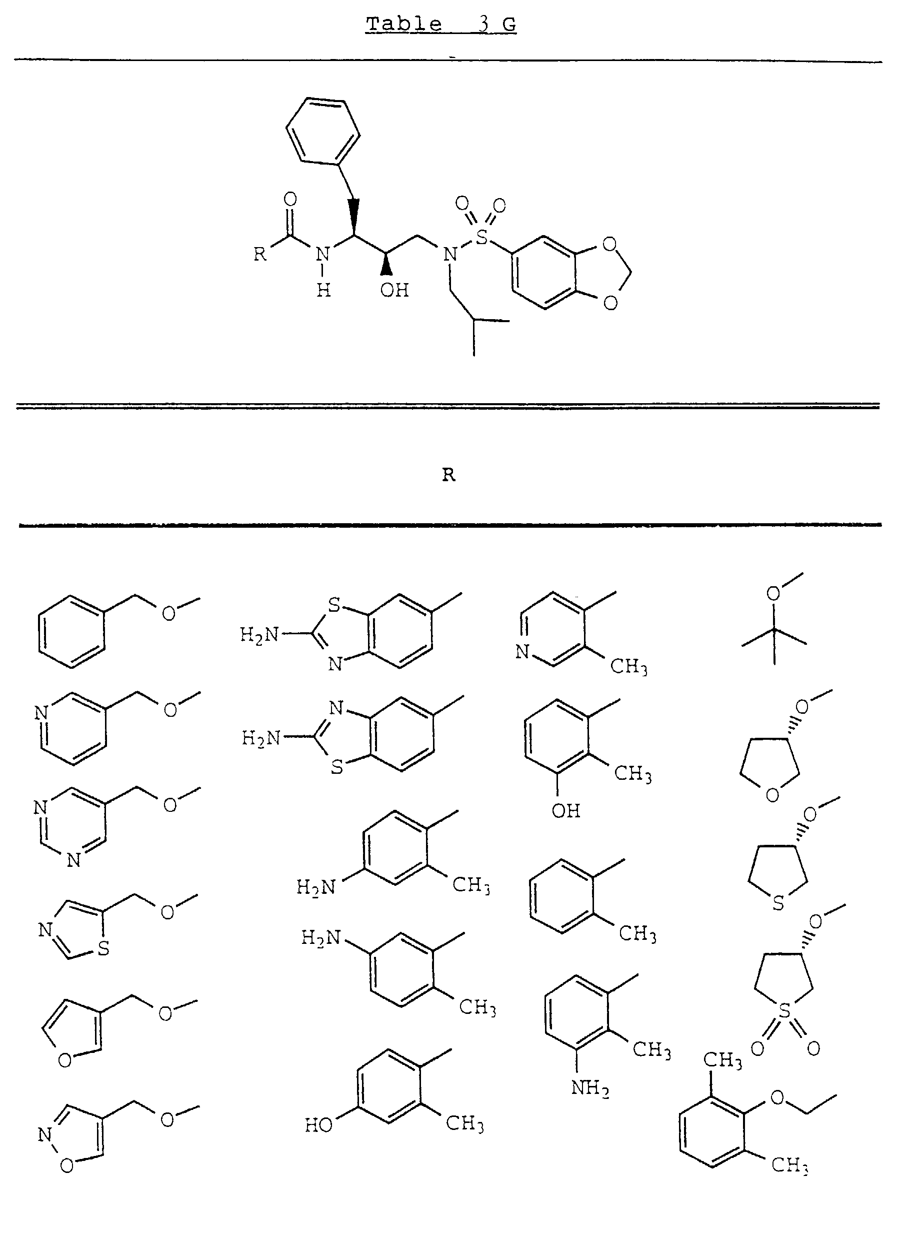

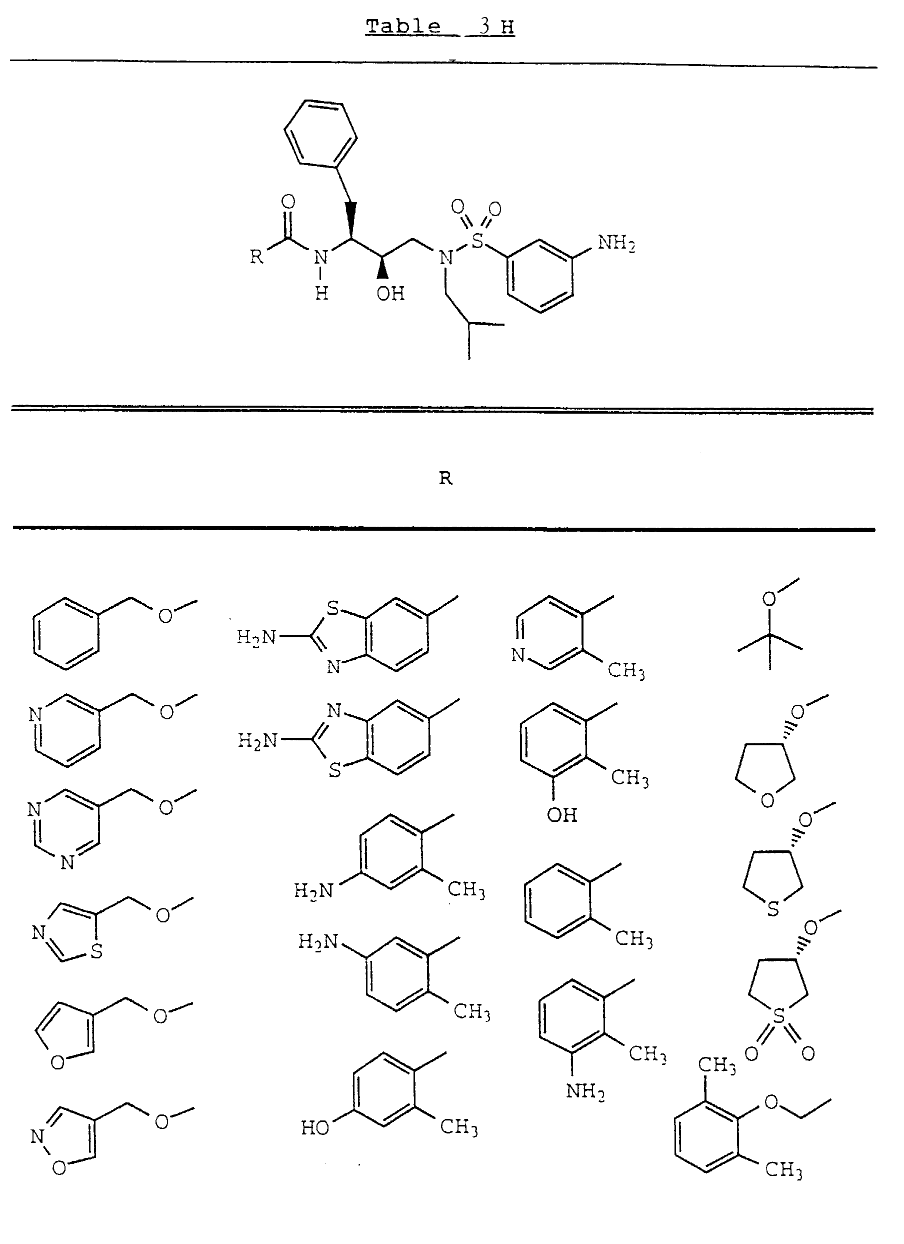

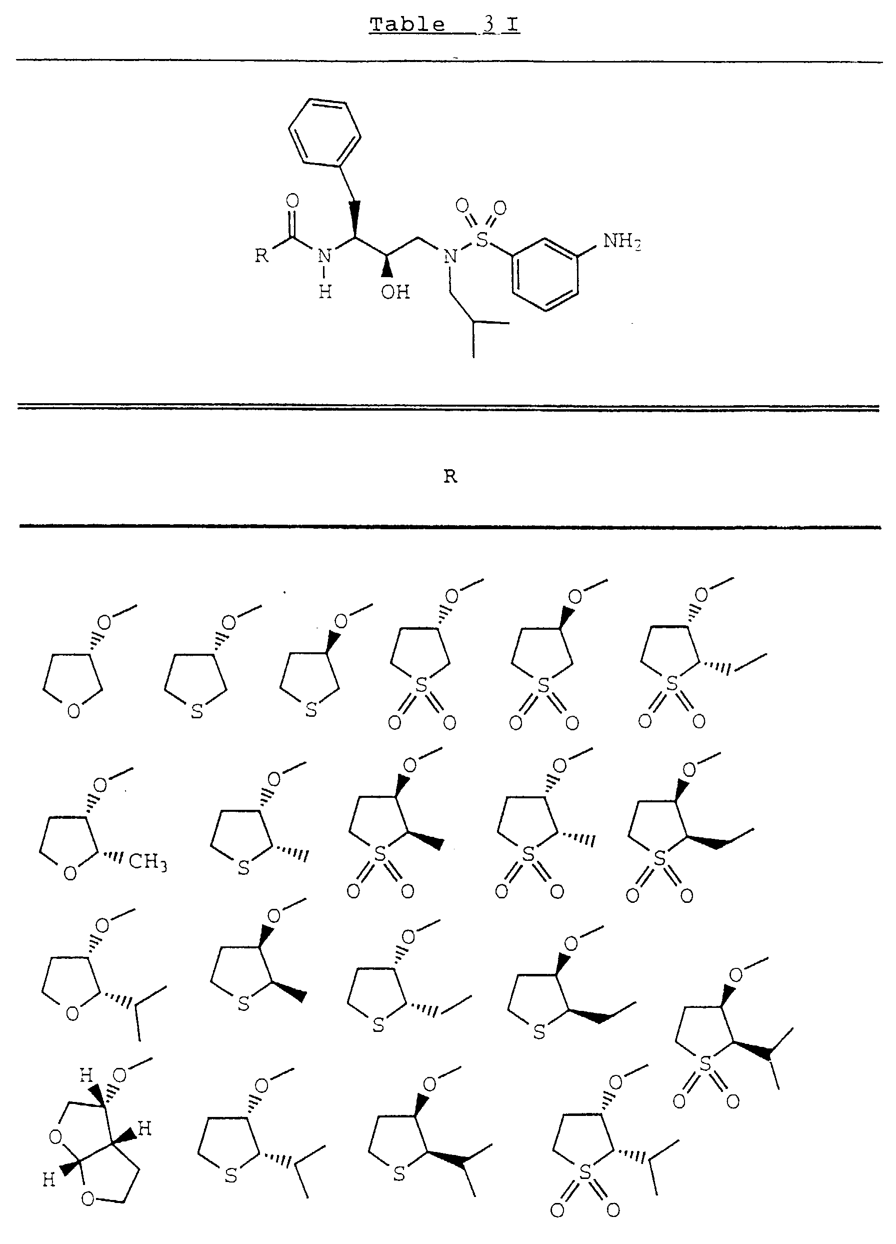

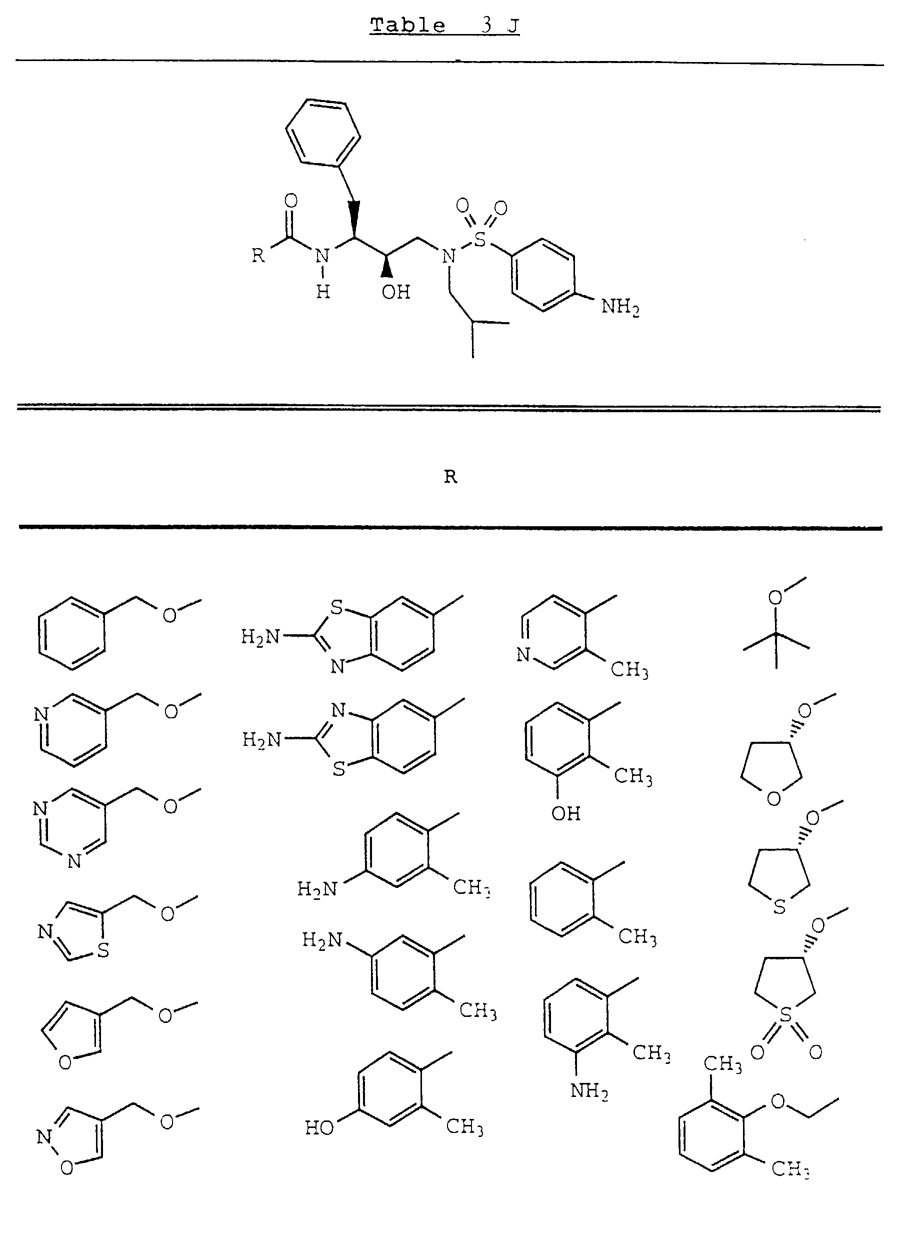

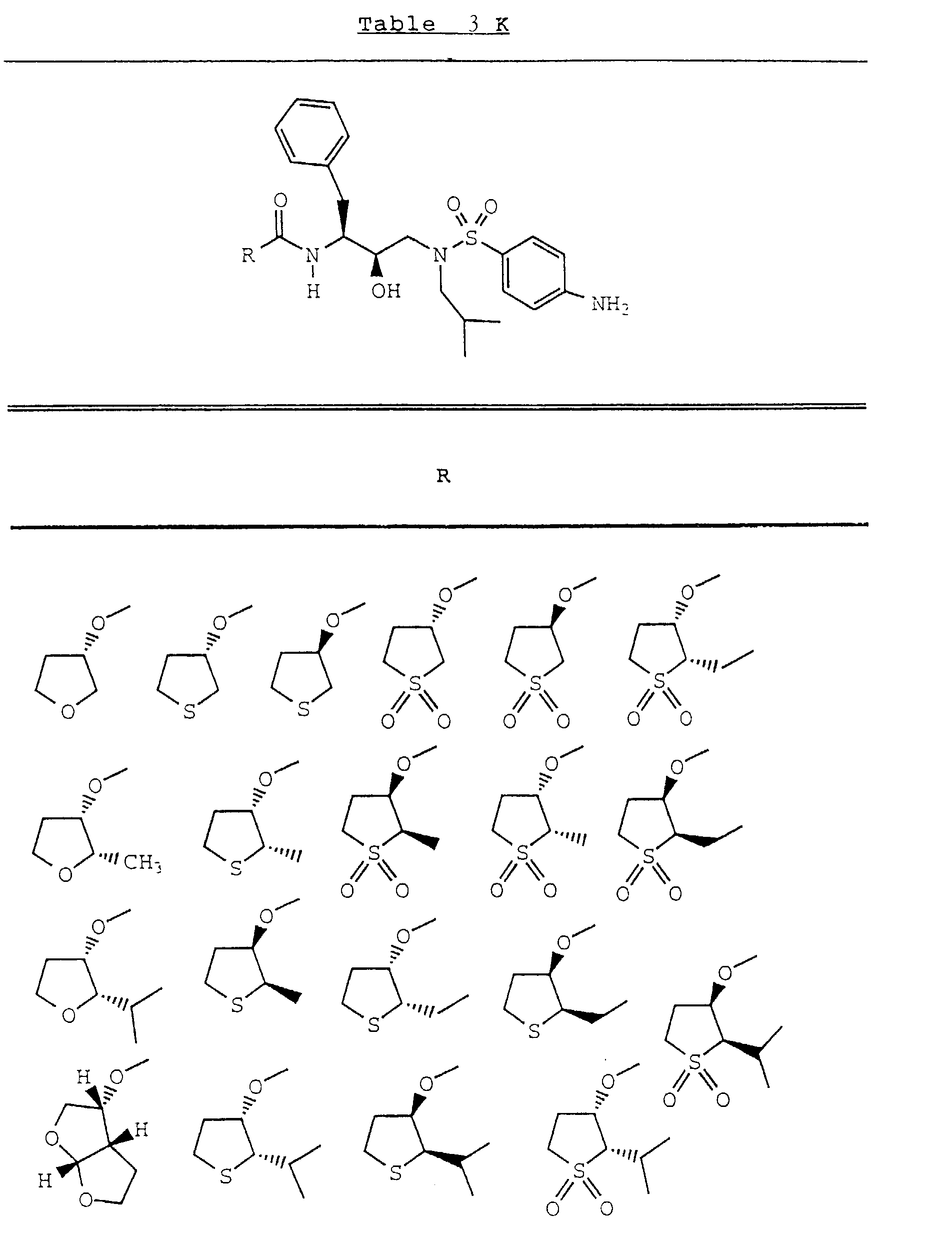

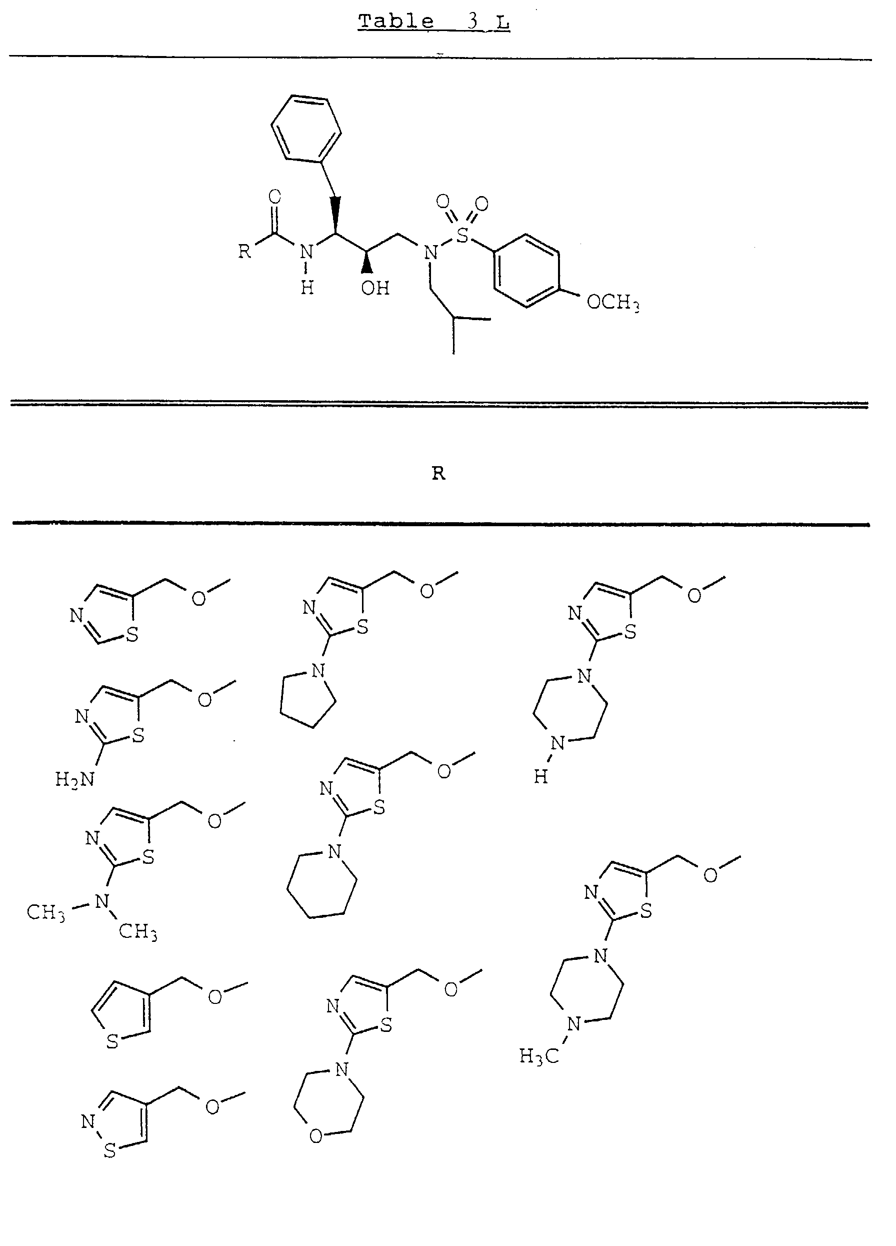

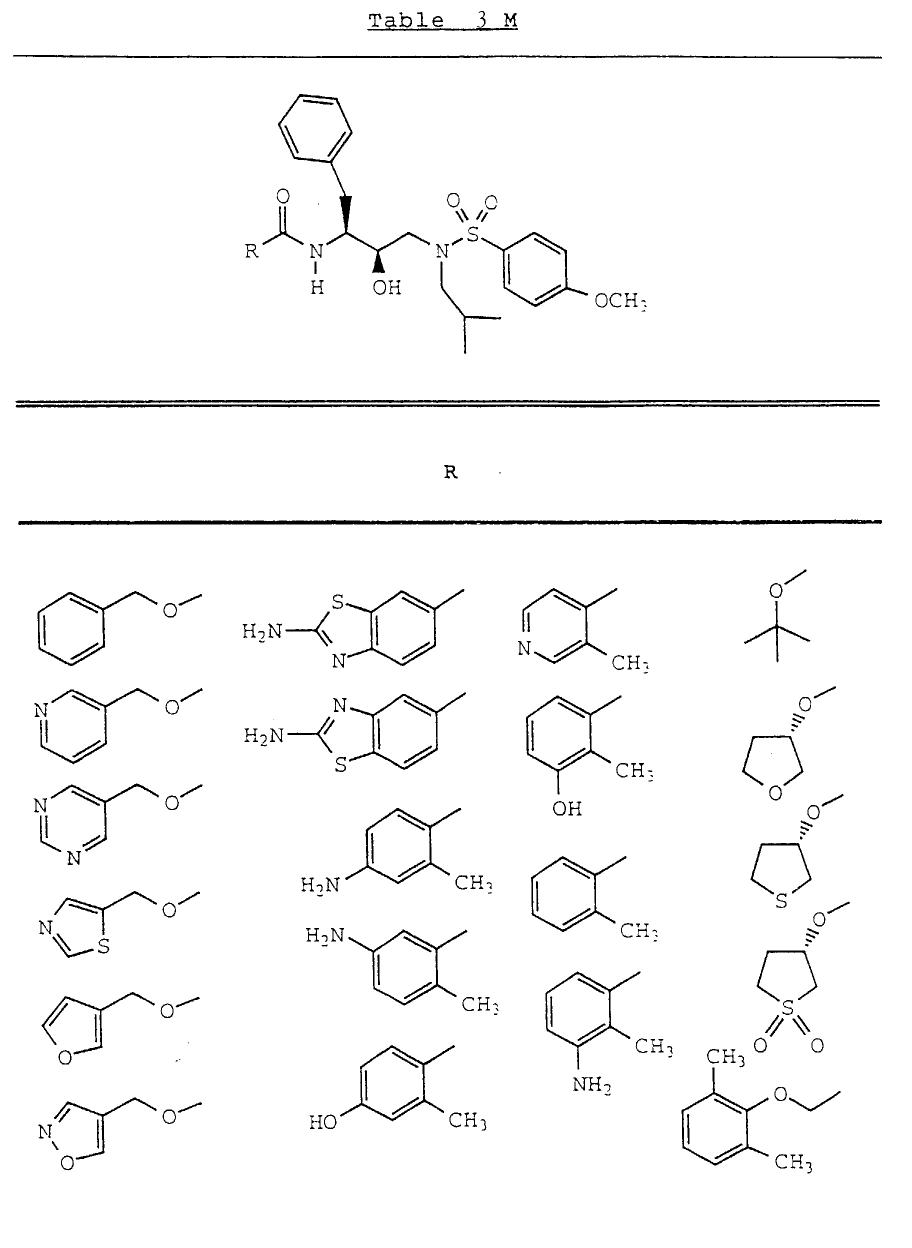

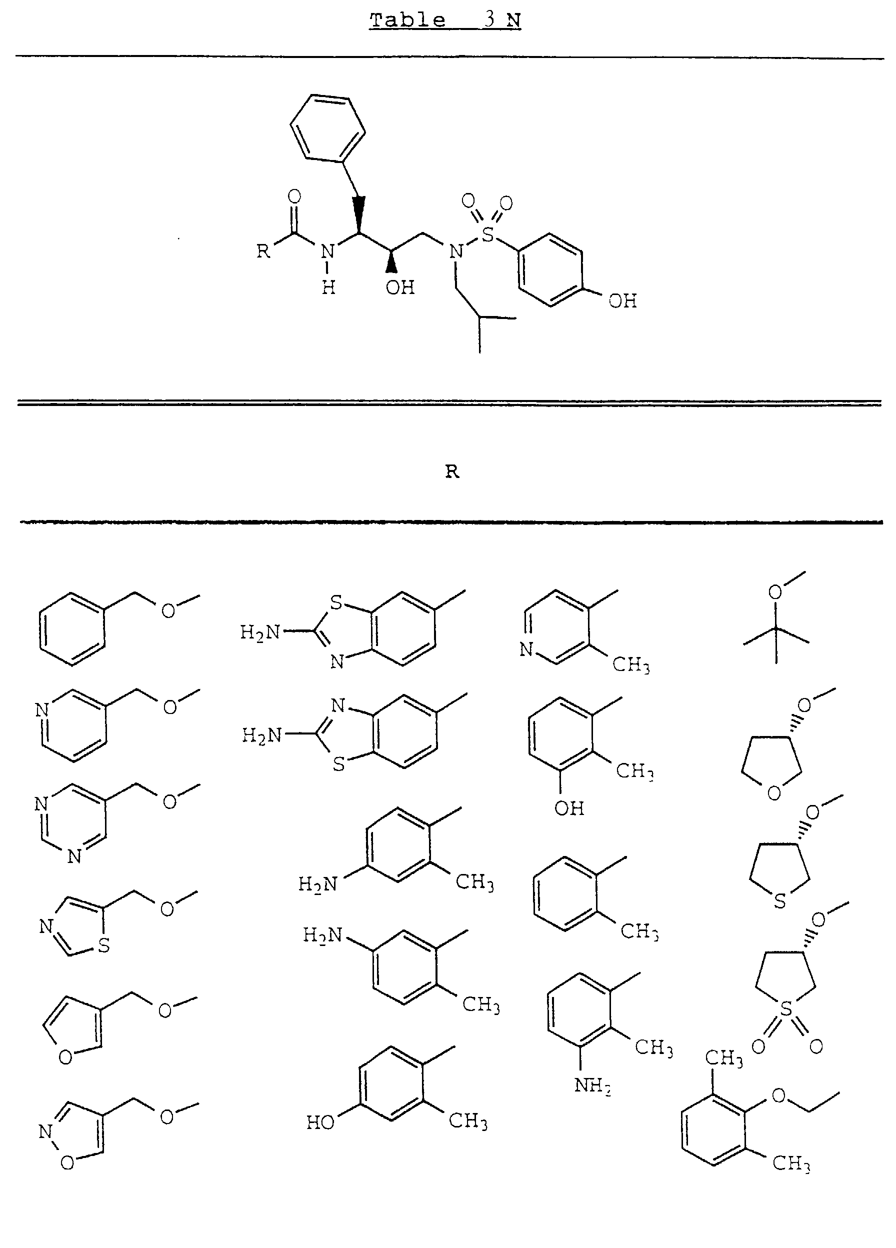

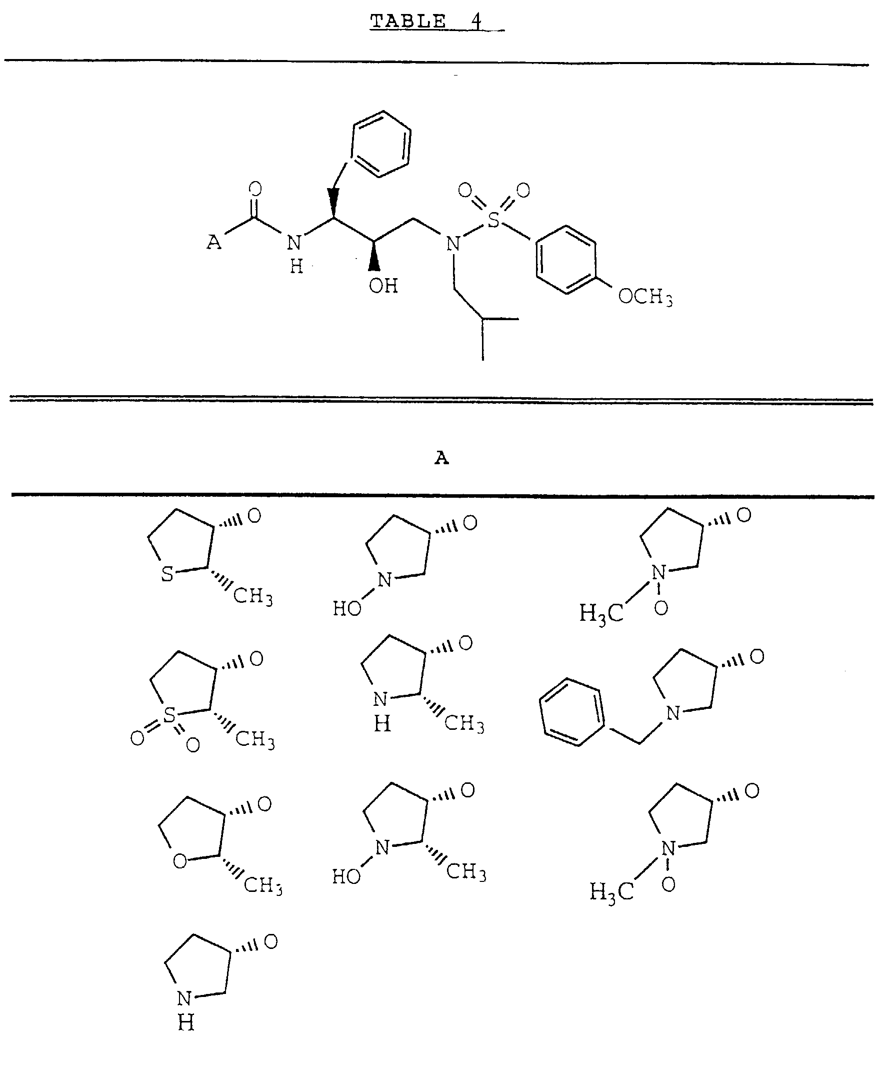

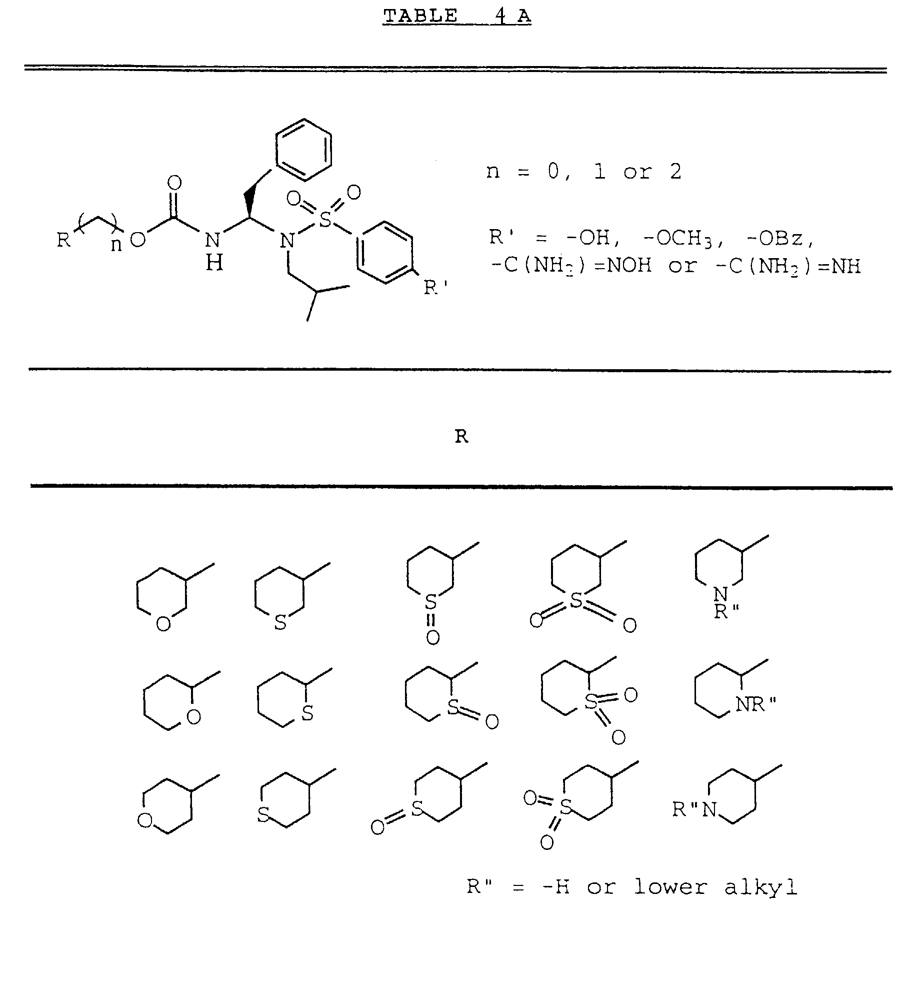

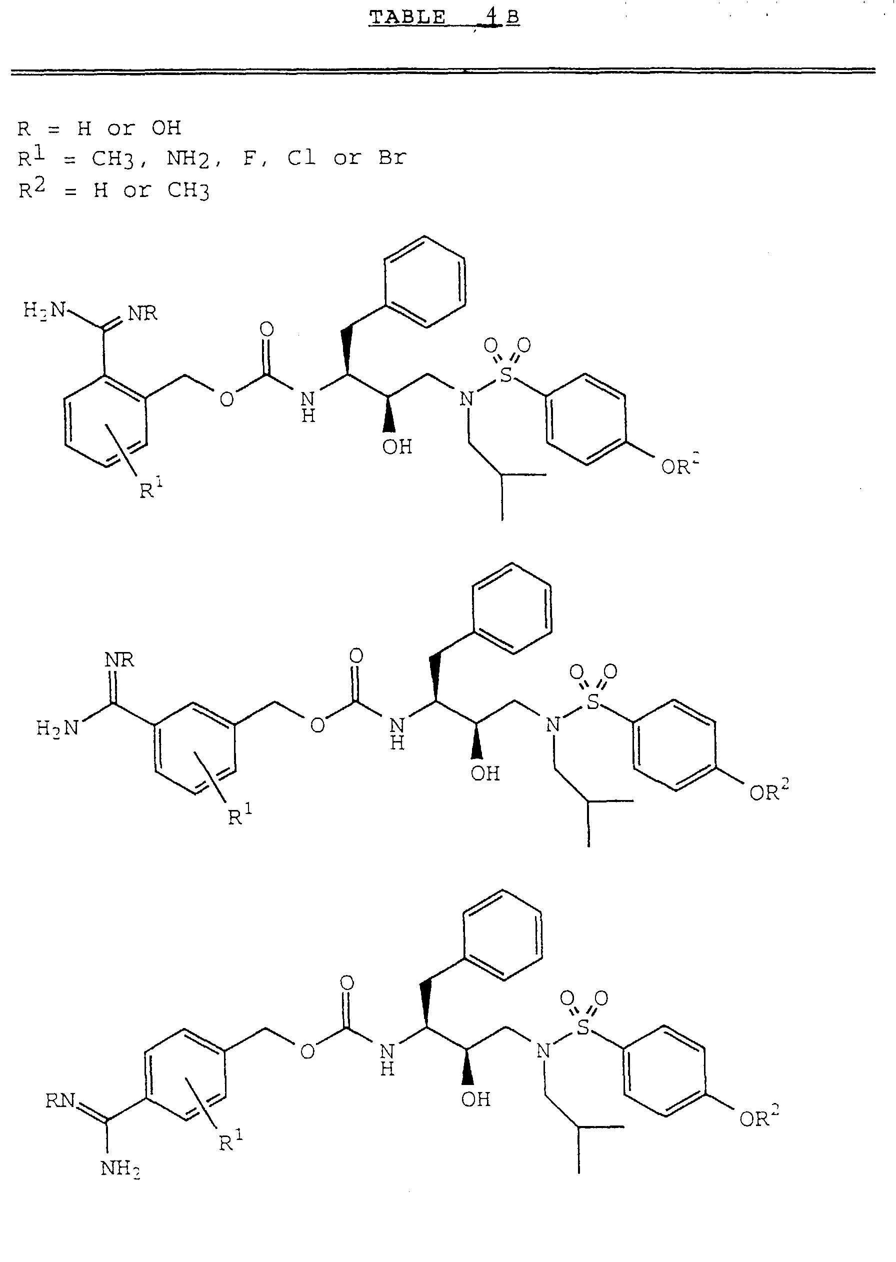

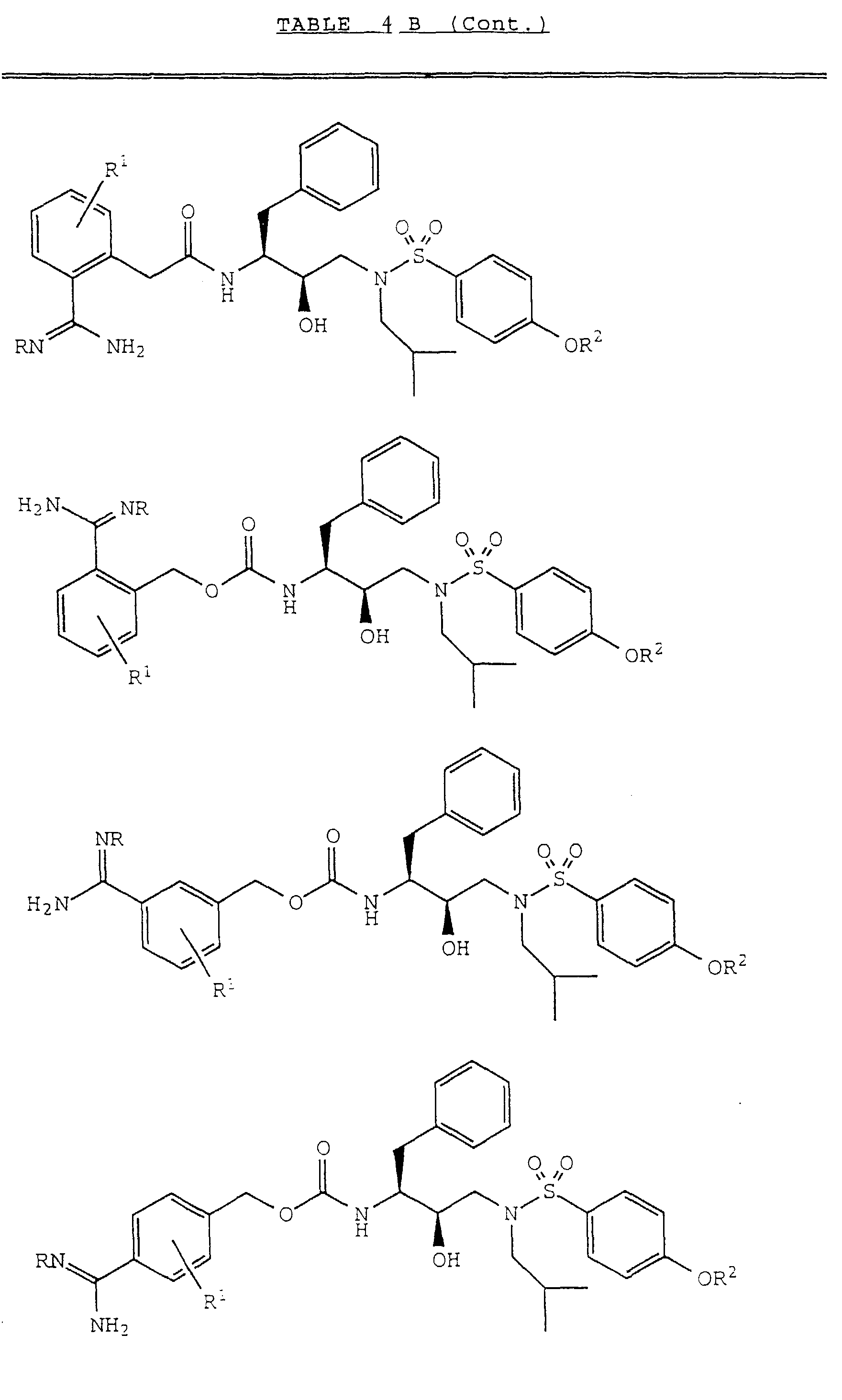

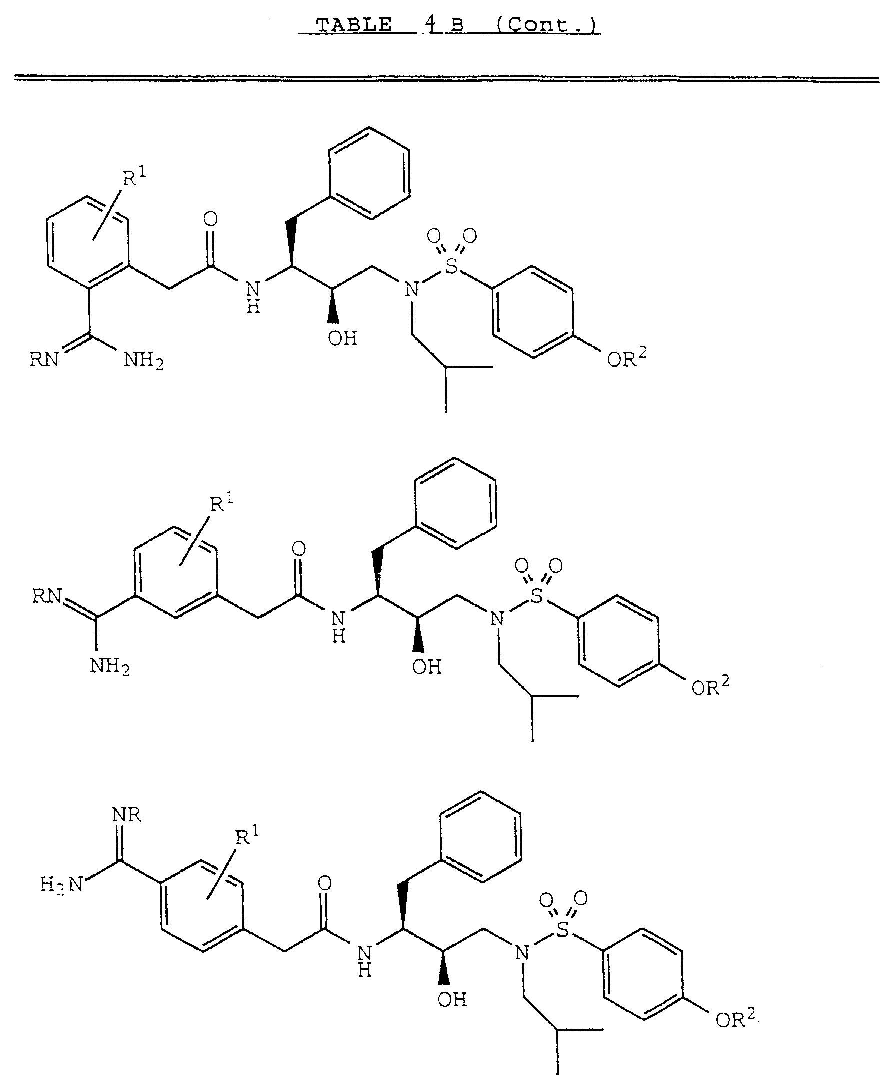

### Example 9

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Tables 18 through 21. The enzyme method is described below. The substrate is 2-Ile-Nle-Phe(p-NO₂) -Gln-ArgNH₂. The positive control is MVT-101 (Miller, M. et al, Science, 246, 1149 (1989)] The assay conditions are as follows:
Assay buffer: 20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

The preceding examples can be repeated with similar success by substituting the generically or specifically described reactants and/or operating conditions of this invention for those used in the preceding examples.

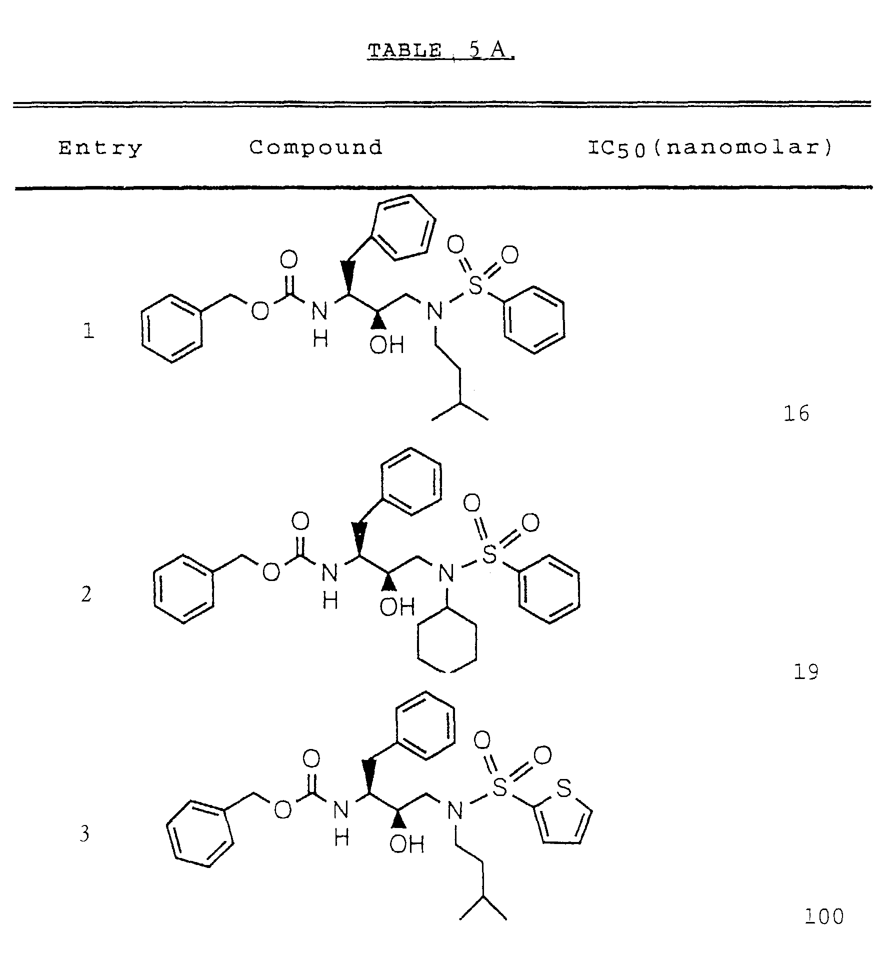

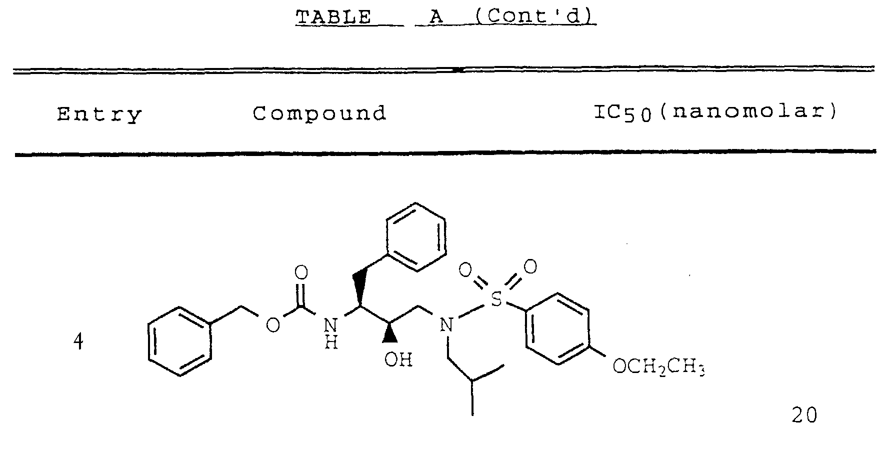

### Example 10

The effectiveness of various compounds were determined in the above-described enzyme assay and in a CEM cell assay.

The HIV inhibition assay method of acutely infected cells is an automated tetrazolium based colorimetric assay essentially that reported by Pauwles et al, J. Virol. Methods, 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene 12µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

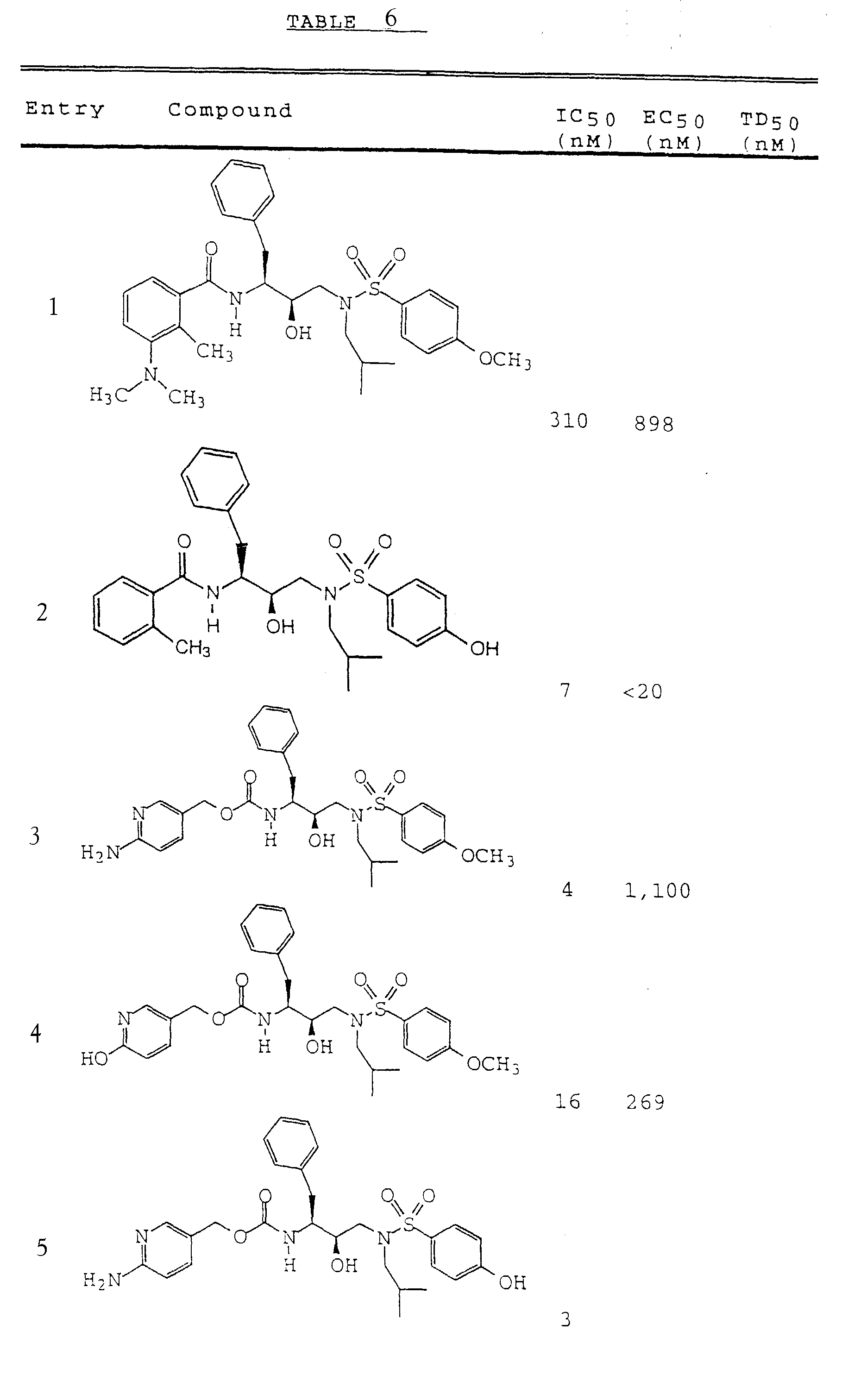

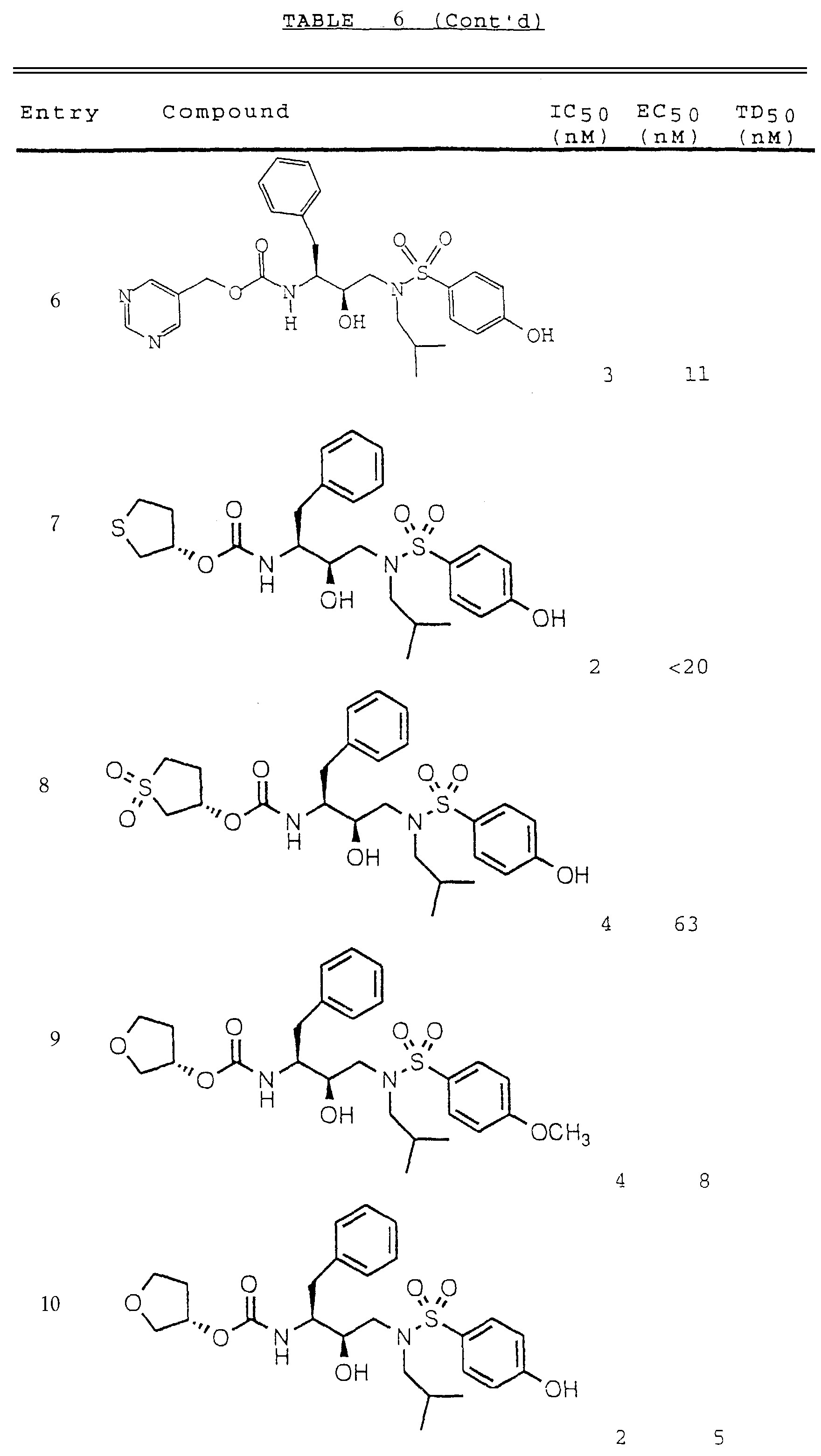

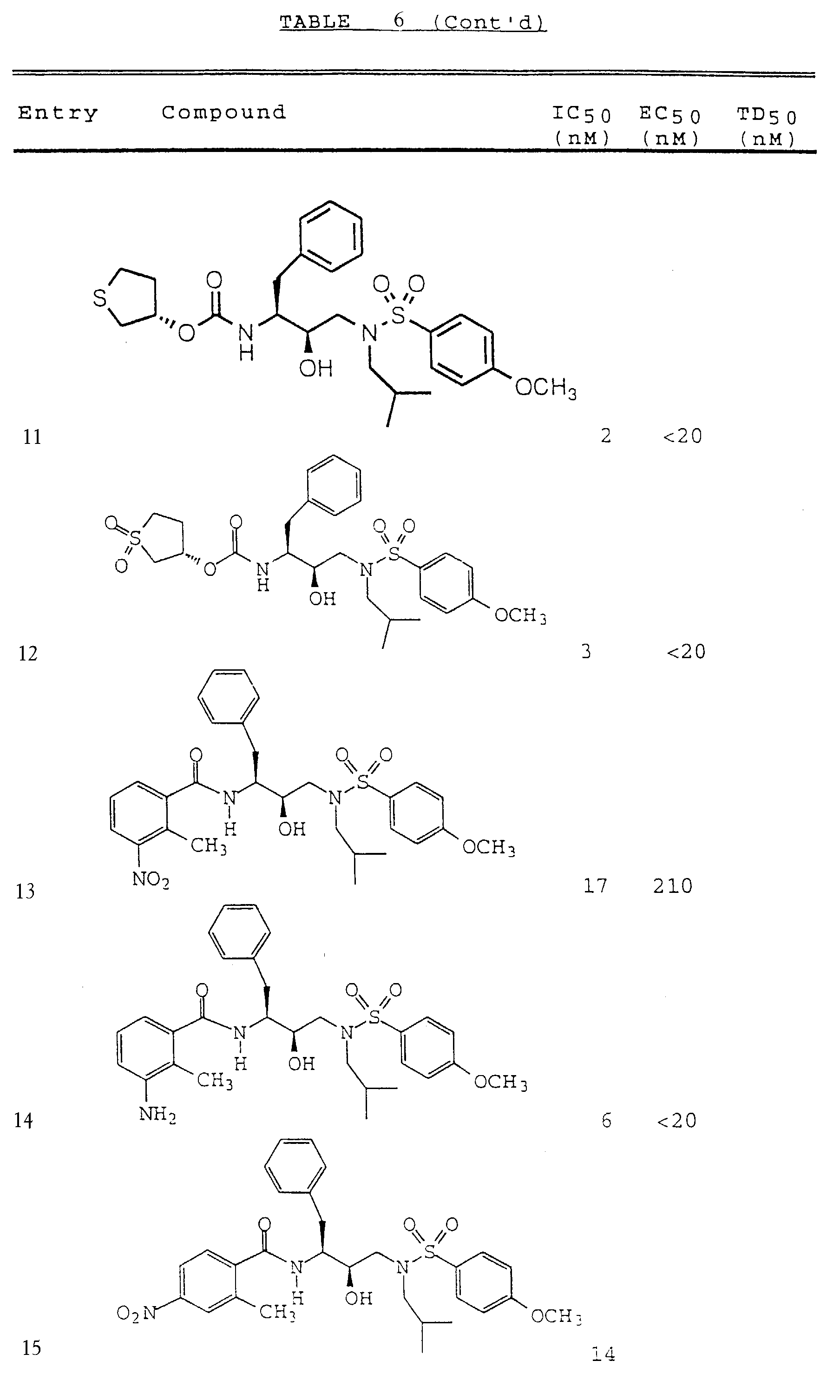

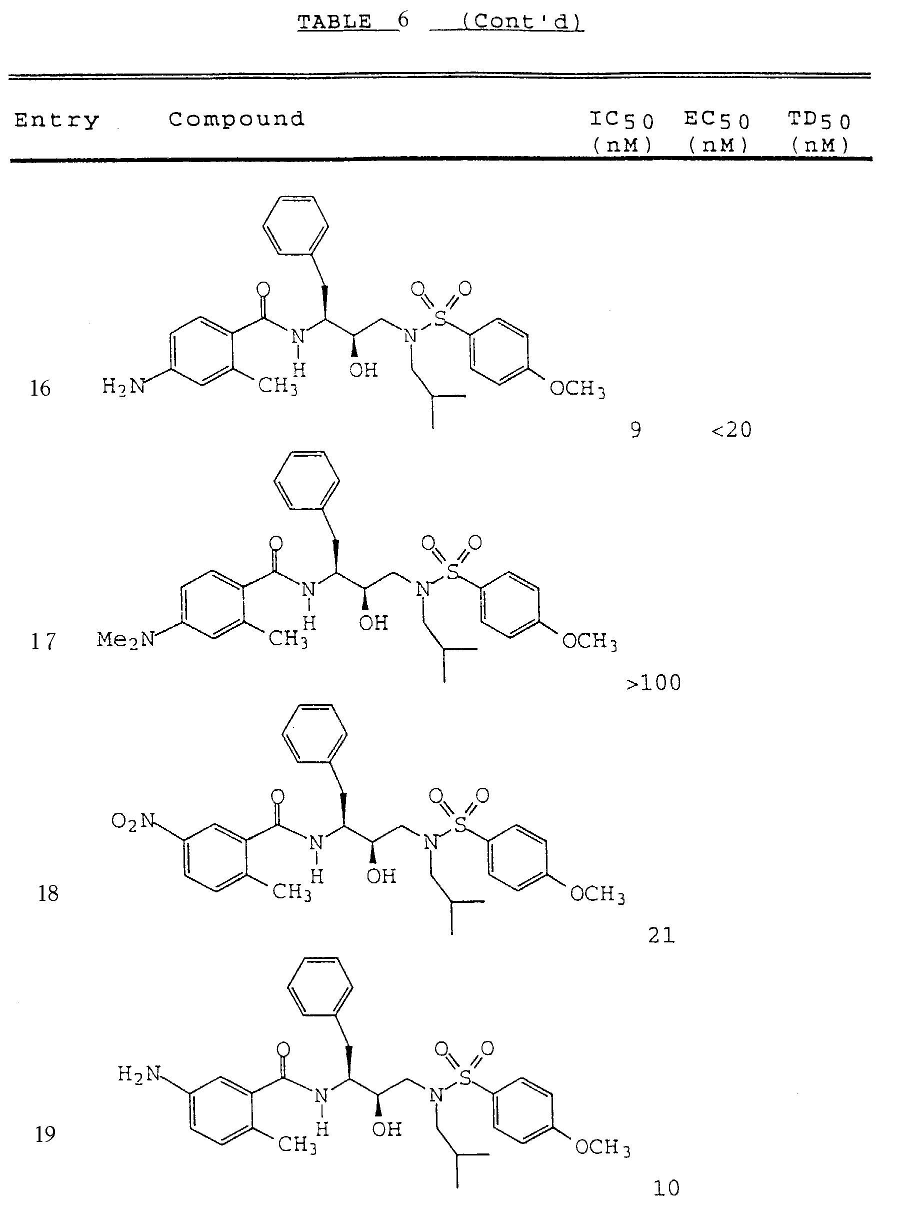

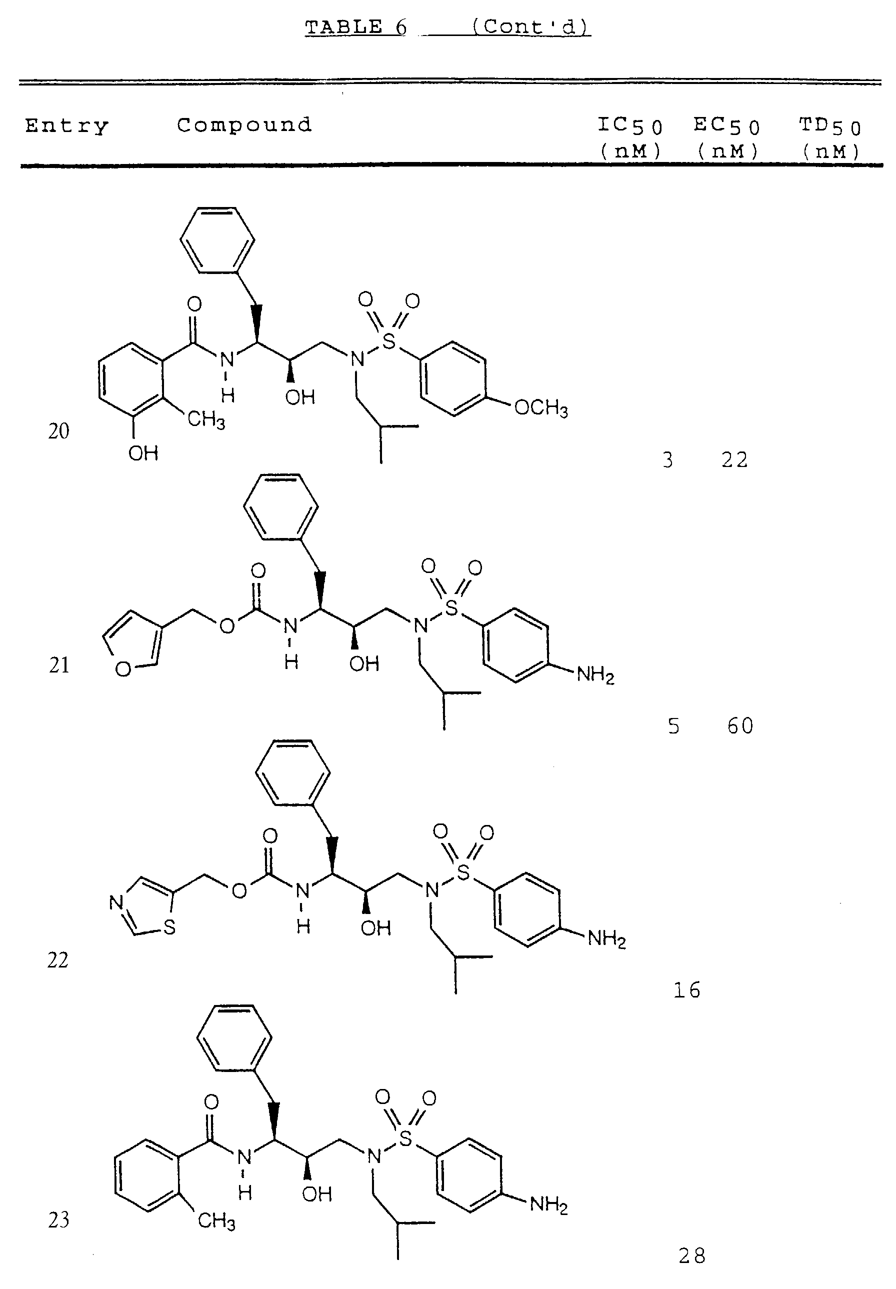

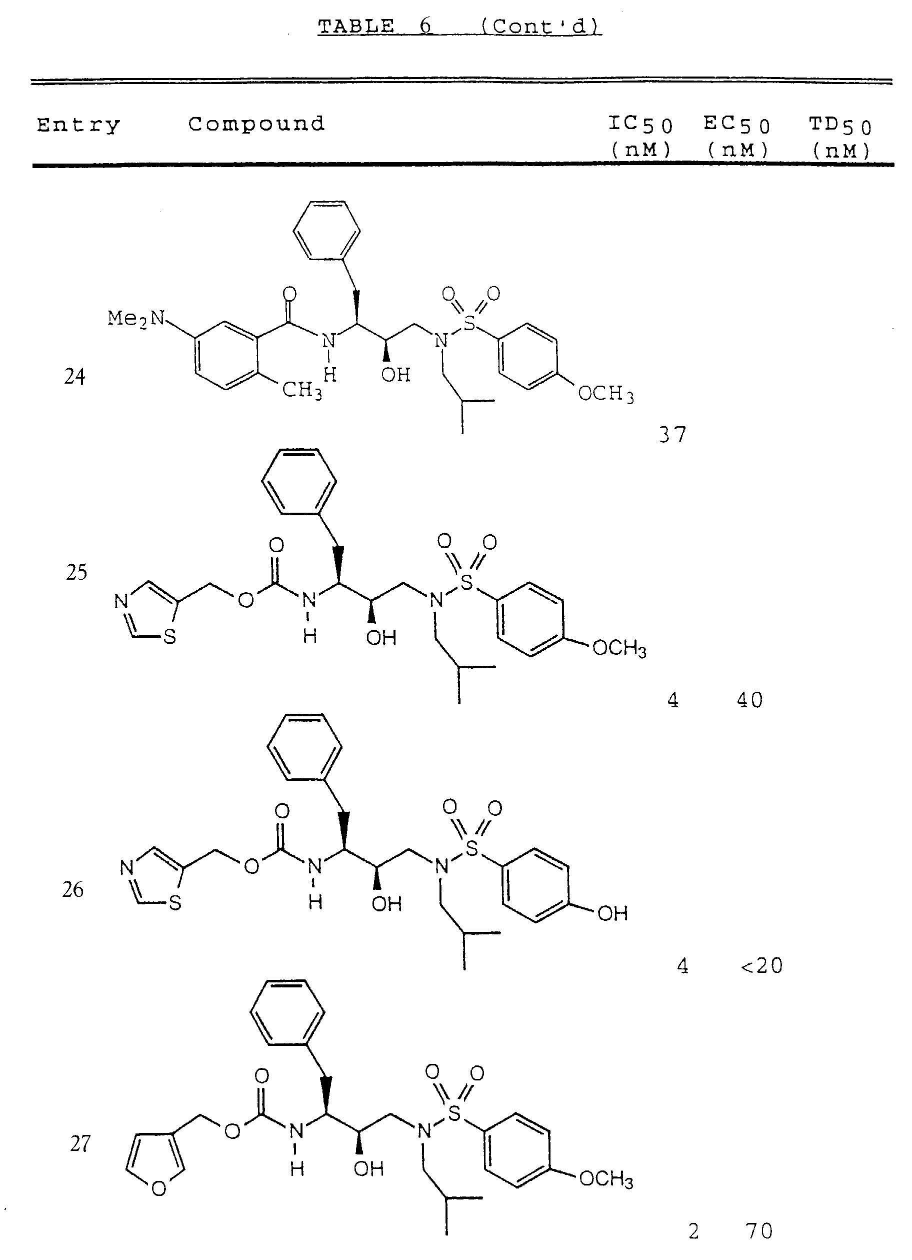

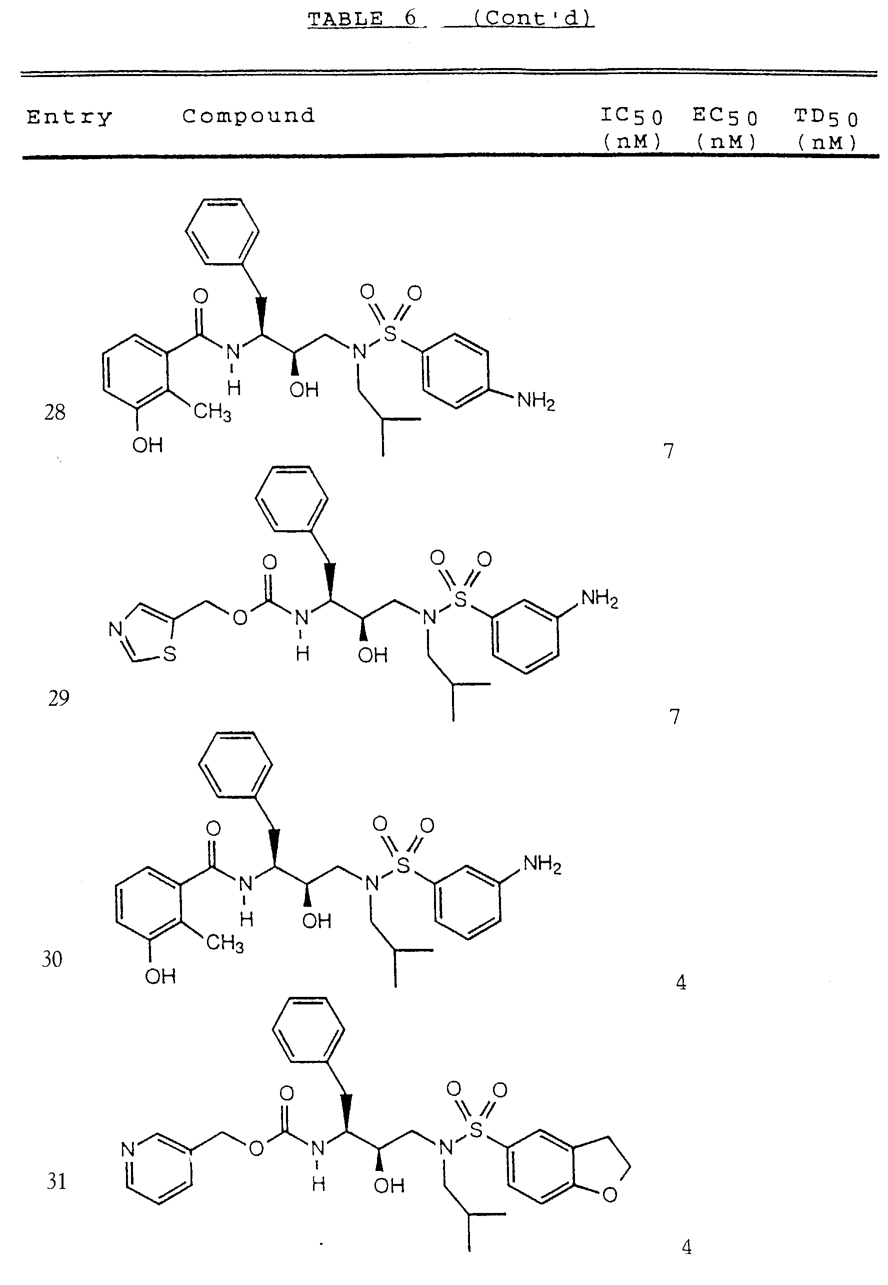

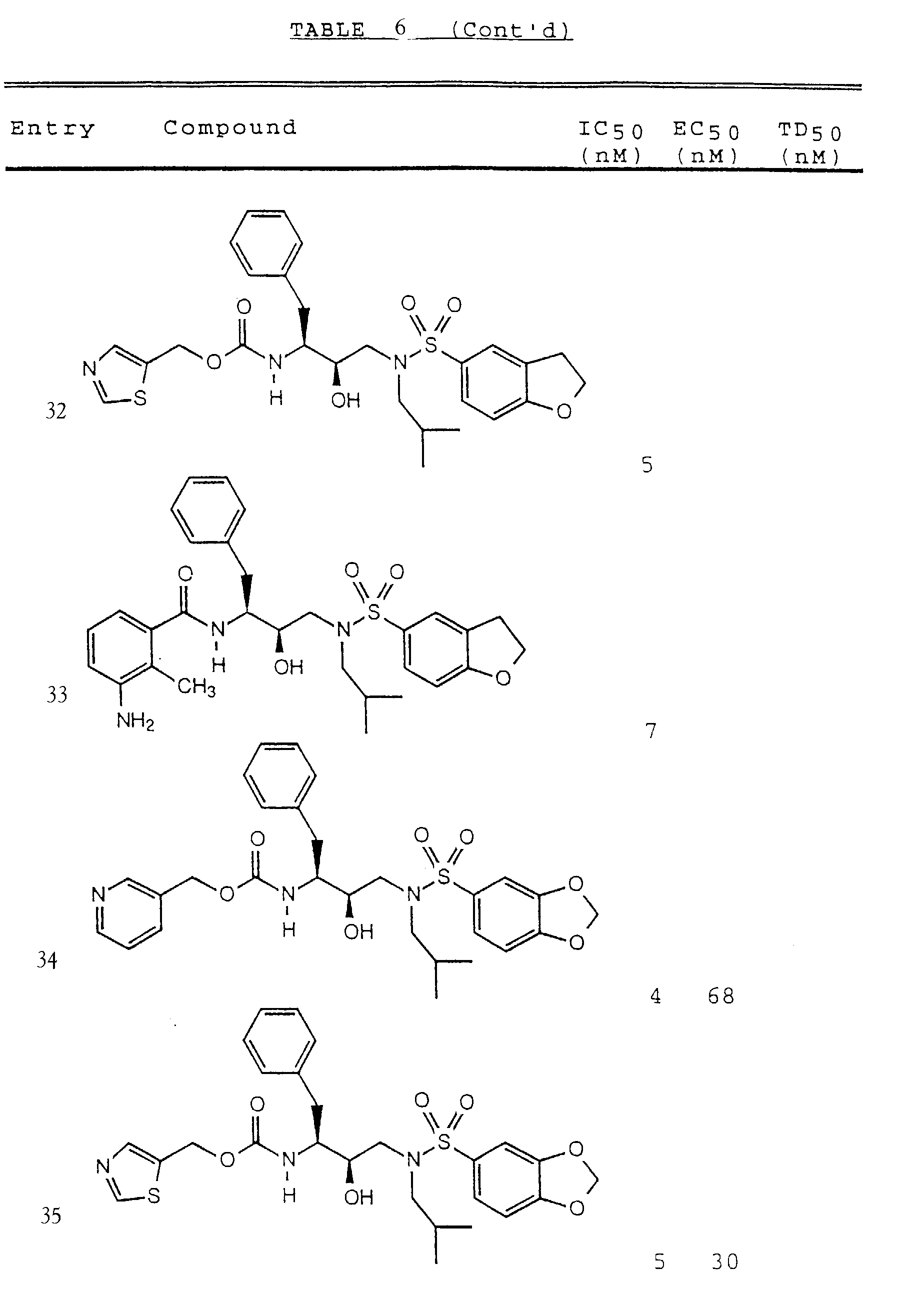

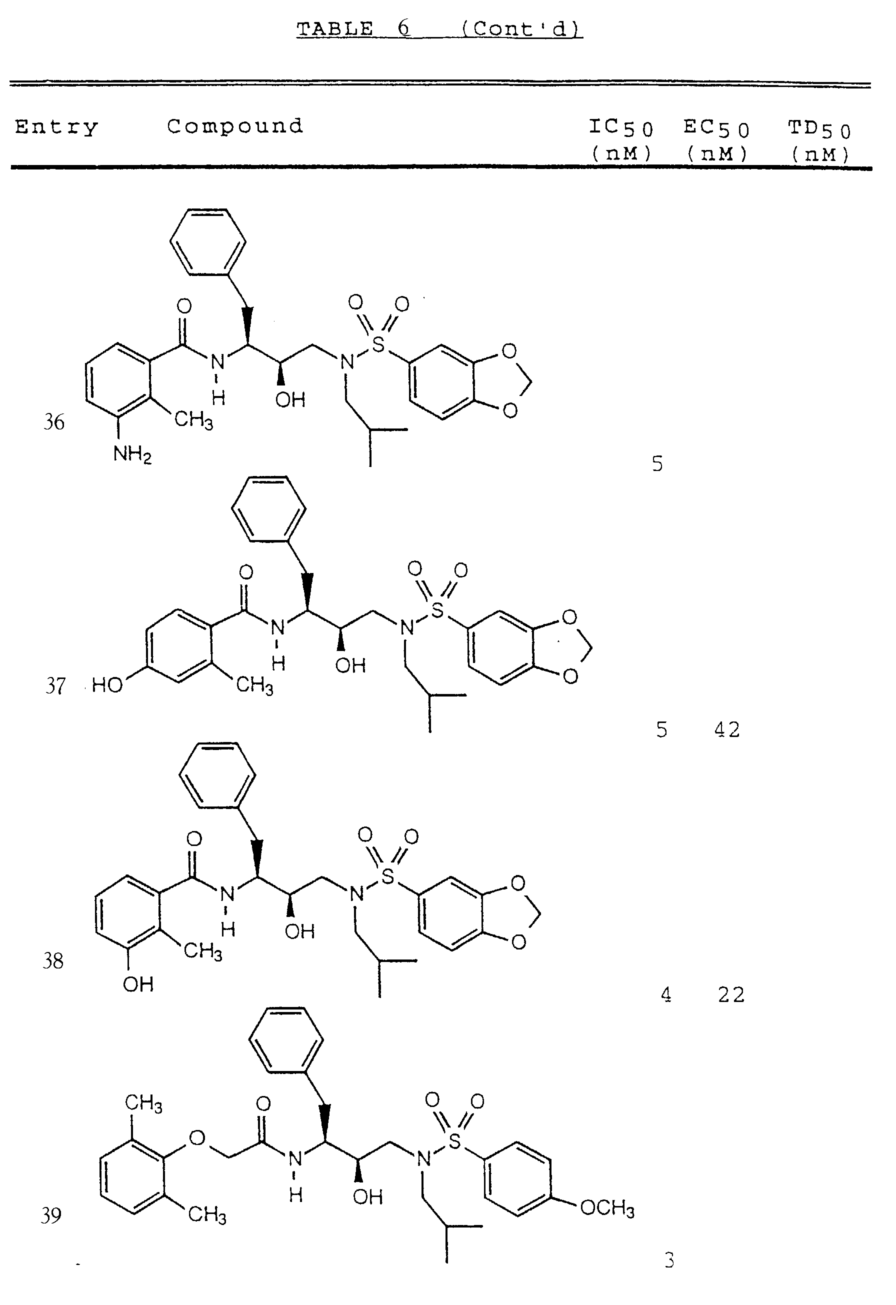

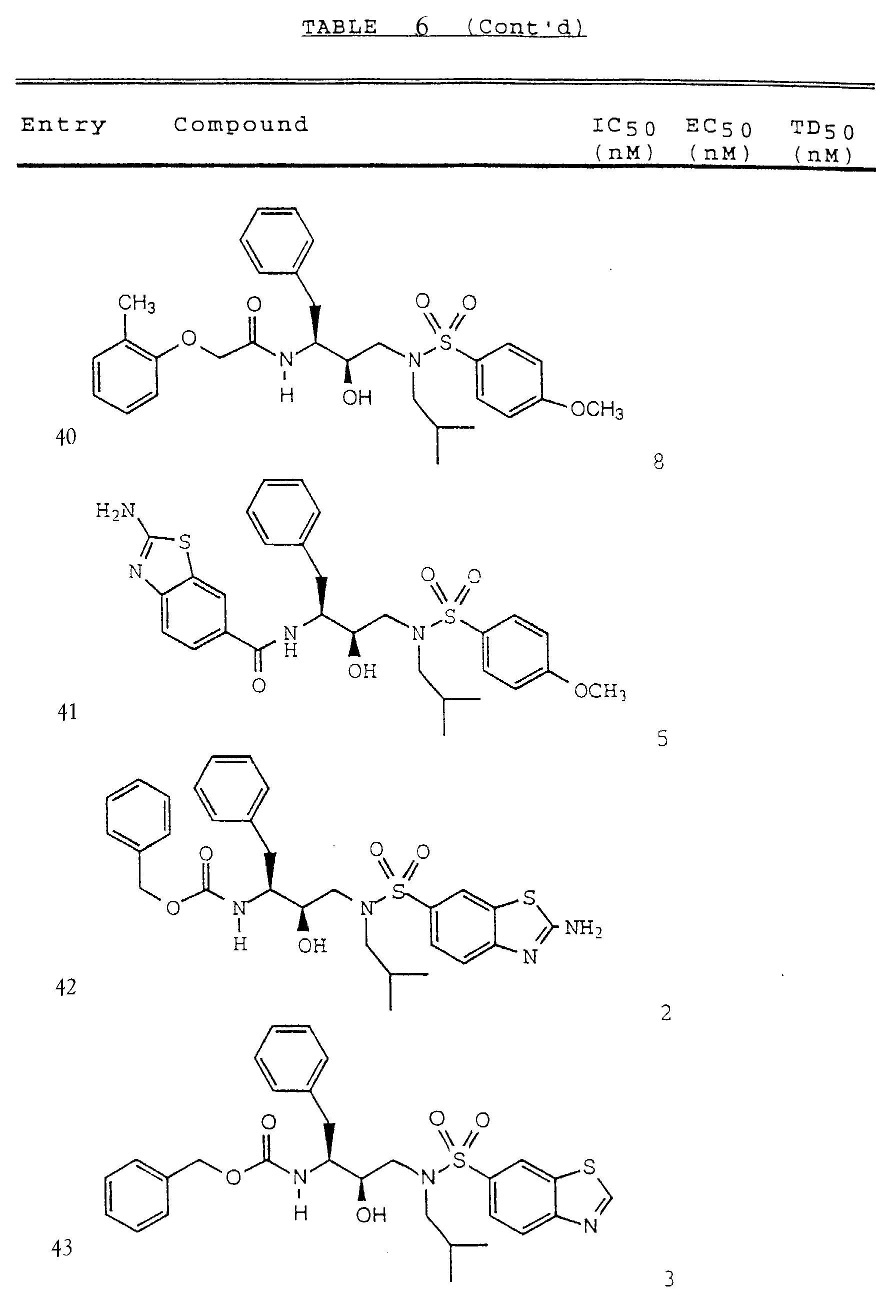

The compounds of the present invention are effective antiviral compounds and, in particular, are effective retroviral inhibitors as shown above. Thus, the subject compounds are effective HIV protease inhibitors. It is contemplated that the subject compounds will also inhibit other retroviruses such as other lentiviruses in particular other strains of HIV, e.g. HIV-2, human T-cell leukemia virus, respiratory syncitial virus, simia immunodeficiency virus, feline leukemia virus, feline immuno-deficiency virus, hepadnavirus, cytomegalovirus and picornavirus. Thus, the subject compounds are effective in the treatment and/or proplylaxis of retroviral infections.

The subject compounds are also effective in preventing the growth of retroviruses in a solution. Both human and animal cell cultures, such as T-lymphocyte cultures, are utilized for a variety of well known purposes, such as research and diagnostic procedures including calibrators and controls. Prior to and during the growth and storage of a cell culture, the subject compounds may be added to the cell culture medium at an effective concentration to prevent the unexpected or undesired replication of a retrovirus that may inadvertently or unknowingly be present in the cell culture. The virus may be present originally in the cell culture, for example HIV is known to be present in human T-lymphocytes long before it is detectable in blood, or through exposure to the virus. This use of the subject compounds prevents the unknowing or inadvertent exposure of a potentially lethal retrovirus to a researcher or clinician.

Compounds of the present invention can possess one or more asymmetric carbon atoms and are thus capable of existing in the form of optical isomers as well as in the form of racemic or nonracemic mixtures thereof. The optical isomers can be obtained by resolution of the racemic mixtures according to conventional processes, for example by formation of diastereoisomeric salts by treatment with an optically active acid or base. Examples of appropriate acids are tartaric, diacetyltartaric, dibenzoyltartaric, ditoluoyltartaric and camphorsulfonic acid and then separation of the mixture of diastereoisomers by crystallization followed by liberation of the optically active bases from these salts. A different process for separation of optical isomers involves the use of a chiral chromatography column optimally chosen to maximize the separation of the enantiomers. Still another available method involves synthesis of covalent diastereoisomeric molecules by reacting compounds of Formula I with an optically pure acid in an activated form or an optically pure isocyanate. The synthesized diastereoisomers can be separated by conventional means such as chromatography, distillation, crystallization or sublimation, and then hydrolyzed to deliver the enantiomerically pure compound. The optically active compounds of Formula I can likewise be obtained by utilizing optically active starting materials. These isomers may be in the form of a free acid, a free base, an ester or a salt.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

The dosage regimen for treating a disease condition with the compounds and/or compositions of this invention is selected in accordance with a variety of factors, including the type, age, weight, sex, diet and medical condition of the patient, the severity of the disease, the route of administration, pharmacological considerations such as the activity, efficacy, pharmacokinetic and toxicology profiles of the particular compound employed, whether a drug delivery system is utilized and whether the compound is administered as part of a drug combination. Thus, the dosage regimen actually employed may vary widely and therefore may deviate from the preferred dosage regimen set forth above.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. For example, the compounds of the invention can be administered in combination with AZT, DDI, DDC or with glucosidase inhibitors, such as N-butyl-1-deoxynojirimycin or prodrugs thereof, for the prophylaxis and/or treatment of AIDS. When administered as a combination, the therapeutic agents can be formulated as separate compositions which are given at the same time or different times, or the therapeutic agents can be given as a single composition.

## Claims

1. A compound represented by the formula: 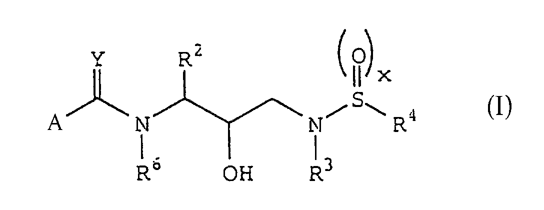 or a pharmaceutically acceptable salt, prodrug or ester thereof, wherein
R² is an alkyl, aryl, cycloalkyl, cycloalkylalkyl or aralkyl radical, which radical is optionally substituted with a radical selected from the group consisting of alkyl, halo, nitro, cyano, CF₃, -OR⁹, and -SR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or disubstituted aminoalkyl radicals, wherein said substituents are selected from the group consisting of alkyl, aryl, aralkyl, cycloalkyl, cycloalkylalkyl, heteroaryl, heteroaralkyl, heterocycloalkyl and heterocycloalkylalkyl radicals; or where said aminoalkyl radical is disubstituted, said substituents along with the nitrogen atom to which they are attached, form a heterocycloalkyl or a heteroaryl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl, heteroaralkyl, aminoalkyl or mono- or disubstituted aminoalkyl radical, wherein said substituents are selected from the group consisting of alkyl, aryl, aralkyl, cycloalkyl, cycloalkylalkyl, heteroaryl, heteroaralkyl, heterocycloalkyl and heterocycloalkylalkyl radicals; or where said aminoalkyl radical is disubstituted, said substituents along with the nitrogen atom to which they are attached, form a heterocycloalkyl or a heteroaryl radical;
R6 is a hydrogen or alkyl radical;
x is 1 or 2;
Y is O or S; and
A is an alkoxy, alkenoxy, aralkoxy, alkyl, cycloalkyl, cycloalkylalkoxy, cycloalkylalkyl, aralkyl, aryl, aryloxy, heterocycloalkyl, heterocycloalkoxy, heterocycloalkylalkyl, heterocycloalkylalkoxy, heteroaralkyl, heteroaralkoxy, heteroaryloxy, heteroaryl, alkenyl, aryloxyalkyl, heteroaryloxyalkyl, hydroxyalkyl, amino, or mono- or disubstituted amino radical, wherein the substituents are selected from the group consisting of alkyl, aryl, aralkyl, cycloalkyl, cycloalkylalkyl, heteroaryl, heteroaralkyl, heterocycloalkyl and heterocycloalkyalkyl radicals; or where said amino radical is disubstituted, said substituents along with the nitrogen atom to which they are attached form a heterocycloalkyl or heteroaryl radical,
except the compounds of the formula 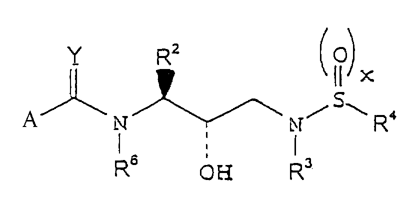 wherein
R³ and R⁶ are both hydrogen
R² is cyclohexylmethyl
x is 2
Y is oxygen
R⁴ is C₁-C₁₂-alkyl, alkylamino, alkylaminoalkyl, heteroaryl, heterocycloalkyl, C₃-C₁₀-cycloalkyl, aryl, C₂-C₆-alkenyl, C₂-C₆-alkynyl,
A is alkoxy, aralkoxy, aryloxy, cycloalkylalkoxy.

2. The compound of Claim 1 or a pharmaceutically acceptable salt, prodrug or ester thereof, wherein
R² is an alkyl, aryl, cycloalkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or disubstituted aminoalkyl radicals, wherein said substituents are selected from the group consisting of alkyl, aralkyl, cycloalkyl and cycloalkylalkyl radicals; or where said aminoalkyl radical is disubstituted, said substituents along with the nitrogen atom to which they are attached, form a heterocycloalkyl or a heteroaryl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, cycloalkylalkoxy, cycloalkylalkyl, aralkyl, aryl, aryloxy, heterocycloalkyl, heterocycloalkoxy, heterocycloalkylalkyl, heterocycloalkylalkoxy, heteroaralkyl, heteroaralkoxy, heteroaryloxy, heteroaryl, hydroxyalkyl, amino, or mono- or disubstituted amino radical, wherein the substituents are selected from the group consisting of alkyl, aralkyl, heteroaryl, heteroaralkyl, heterocycloalkyl and heterocycloalkyalkyl radicals; or where said amino radical is disubstituted, said substituents along with the nitrogen atom to which they are attached form heterocycloalkyl radical.

3. The compound of Claim 2 or a pharmaceutically acceptable salt, prodrug or ester thereof, wherein
R² is an alkyl, aryl, cycloalkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, haloalkyl, alkenyl, alkynyl, hydroxyalkyl, alkoxyalkyl, alkylthioalkyl, alkylsulfonylalkyl, cycloalkyl, cycloalkylalkyl, heterocycloalkyl, heteroaryl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals.

4. The compound of Claim 3 or a pharmaceutically acceptable salt, prodrug or ester thereof, wherein
R² is an alkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, alkenyl, alkynyl, hydroxyalkyl, alkoxyalkyl, alkylthioalkyl, alkylsulfonylalkyl, cycloalkyl, cycloalkylalkyl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl cycloalkyl, cycloalkylalkyl heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals;
with the proviso that alkyl, alone or in combination, is a straight-chain or branched-chain hydrocarbon radical containing from one to eight carbon atoms; alkenyl, alone or in combination, is a straight-chain or branched-chain hydrocarbon radical having at least one double bond and containing from two to eight carbon atoms; alkynyl, alone or in combination, is a straight-chain or branched-chain hydrocarbon radical having at least one triple bond and containing from two to ten carbon atoms; and cycloalkyl, alone or in combination, is a hydrocarbon ring containing from three to eight carbon atoms.

5. The compound of Claim 4 or a pharmaceutically acceptable salt, prodrug or ester thereof, wherein
R² is an alkyl, cycloalkylalkyl or aralkyl radical, which radical is optionally substituted with a radical selected from the group consisting of alkyl, halo and -OR⁹, wherein R⁹ is a radical selected from the group consisting of hydrogen and alkyl;
R³ is a hydrogen, alkyl, alkenyl, alkynyl, hydroxyalkyl, alkoxyalkyl, alkylthioalkyl, alkylsulfonylalkyl, cycloalkyl, cycloalkylalkyl, heterocycloalkylalkyl, aryl, aralkyl, heteroaralkyl, aminoalkyl or mono- or dialkyl substituted aminoalkyl radical;
R⁴ is an alkyl, haloalkyl, alkenyl, alkynyl, hydroxyalkyl, alkoxyalkyl, cycloalkyl, cycloalkylalkyl, heterocycloalkyl, heteroaryl, heterocycloalkylalkyl, aryl, aralkyl, aralkenyl or heteroaralkyl radical;
R⁶ is a hydrogen or alkyl radical;
x is 1 or 2;
and
Y is O or S; and
A is an alkoxy, alkenoxy, aralkoxy, alkyl, cycloalkyl, aryl, heterocycloalkyl, heterocycloalkoxy, heterocycloalkylalkyl, heteroaralkoxy, heteroaryl, amino, or mono- or disubstituted amino radical, wherein the substituents are selected from the group consisting of alkyl and aralkyl radicals;
with the proviso that alkyl, alone or in combination, is a straight-chain or branched-chain hydrocarbon radical containing from one to five carbon atoms; alkenyl, alone or in combination, is a straight-chain or branched-chain hydrocarbon radical having at least one double bond and containing from two to five carbon atoms; alkynyl, alone or in combination, is a straight-chain or branched-chain hydrocarbon radical having at least one triple bond and containing from two to five carbon atoms; and cycloalkyl, alone or in combination, is a hydrocarbon ring containing from three to eight carbon atoms.

6. The compound of Claim 5 or a pharmaceutically acceptable salt, prodrug or ester thereof, wherein
R² is butyl, cyclohexylmethyl, benzyl, 4-fluorobenzyl or naphthylmethyl;
R³ is methyl, ethyl, propyl, butyl, pentyl, hexyl, isobutyl, iso-amyl, 3-methoxypropyl, 3-methylthiopropyl, 4-methylthiobutyl, 4-methylsulfonylbutyl, 2-dimethylaminoethyl, 2-(1-morpholino)ethyl, 4-hydroxybutyl, allyl, propargyl, cyclohexylmethyl, cyclopropylmethyl, phenyl, benzyl, 4-fluorobenzyl, 4-methoxybenzyl, 1-phenylethyl, 2-phenylethyl, naphthylmethyl, 3-pyridylmethyl or 4-pyridylmethyl;
R⁴ is methyl, ethyl, propyl, butyl, ethenyl, chloromethyl, cyclopropyl, cyclobutyl, cyclopentyl, cyclohexyl, phenyl, naphthyl, chlorophenyl, fluorophenyl, hydroxyphenyl, methylphenyl, methoxyphenyl, ethoxyphenyl, methylthiophenyl, methylsulfoxyphenyl, methylsulfonylphenyl, acetamidophenyl, methoxycarbonylphenyl, dimethylaminophenyl, nitrophenyl, trifluoromethylphenyl, benzyl, 2-phenylethenyl or thienyl;
R⁶ is hydrogen;
x is 2; and
Y is O; and
A is methyl, cyclohexyl, cyclopentyl, cycloheptyl, 1,2,3,4-tetrahydronaphthyl, naphthyl, quinolinyl, indolyl, pyridyl, methylpyridyl, furanyl, thiophenyl, oxazolyl, thiazolyl, phenyl, methylphenyl, ethylphenyl, dimethylphenyl, iso-propylphenyl, chlorophenyl, contemplated hydroxyphenyl, methoxyphenyl, methylsulfonylphenyl, methylsulfonylmethylphenyl, carboxyphenyl, aminocarbonylphenyl, methylhydroxyphenyl, methylnitrophenyl, methylaminophenyl, methyl-N,N-dimethylaminophenyl, t-butoxy, benzyloxy, pyridylmethoxy, 3-propenoxy, hydroxypyridylmethoxy, aminopyridylmethoxy, pyrimidinylmethoxy, N-oxo-pyrimidinylmethoxy, thiazolylmethoxy, tetrahydrothiophenoxy, 1,1-dioxotetrahydrothiophenoxy, tetrahydrofuranoxy, methylamino, benzylamino or isopropylamino.

7. The compound of Claim 1 which is:
Phenylmethyl[2R-hydroxy-3-[(3-methylbutyl) (methylsulfonyl)amino]-1S-(phenylmethyl)propyl]carbamate;
Phenylmethyl[2R-hydroxy-3-[(3-methylbutyl) (phenylsulfonyl)amino]-1S-(phenylmethyl)propyl]carbamate;
N1-[2R-hydroxy-3[(3-methylbutyl)(phenylsulfonyl)amino]-1S-(phenylmethyl)propyl]-2S-[(phenylmethyloxycarbonyl) amino]butanediamide;
2S-[[(dimethylamino)acetyl]amino]-N-[2R-hydroxy-3-[(3-methyl-butyl)(phenylsulfonyl)amino]-1S-(phenylmethyl) propyl]-3,3-dimethylbutaneamide;
2S-[[(methylamino)acetyl]amino]-N-[2R-hydroxy-3-[(3-methyl-butyl)(phenylsulfonyl)amino]-1S-(phenylmethyl) propyl]-3,3-dimethylbutaneamide;
N1-[2R-hydroxy-3-[(3-methylbutyl)(phenyl-sulfonyl)amino]-N4-methyl-1S-(phenylmethyl)propyl]-2S-[(2-quinolinylcarbonyl)amino]butanediamide;
[3-[[2-hydroxy-3-[N-(3-methylbutyl)-N-(phenylsufonyl)amino]-1-(phenylmethyl)propyl]amino]-2-methyl-3-oxopropyl]-, (4-methoxyphenyl)methyl ester, [1S-[1R*(S*),2S*]]-;
Carbamic acid, [2R-hydroxy-3-[(4-hydroxyphenylsulfonyl) (2-methylpropyl)amino]-1S-(phenylmethyl)propyl-, 3(S)-1,1-dioxotetrahydrothiophen-3-yl-ester;
Carbamic acid, [2R-hydroxy-3-[(4-methoxyphenylsulfonyl) (2-methylpropyl)amino]-1S-(phenylmethyl)propyl-, 3(S)-1,1-dioxotetrahydrothiophen-3-yl-ester;
Carbamic acid, [2R-hydroxy-3-[(4-methoxyyphenylsulfonyl) (2-methylpropyl)amino]-1S-(phenylmethyl)propyl-, 3-S-tetrahydrothiophen-3-yl-ester;
Carbamic acid, [2R-hydroxy-3-[(4-hydroxyphenylsulfonyl) (2-methylpropyl)amino]-1S-(phenylmethyl)propyl-, 3-S-tetrahydrothiophen-3-yl-ester;
Carbamic acid, [2R-hydroxy-3-[[(4-methoxyphenyl)sulfonyl](2-methylpropyl)amino]-1S-(phenylmethyl)propyl]-, 3-(6-aminopyridyl)methyl ester;
Carbamic acid, [2R-hydroxy-3-[[(4-hydroxyphenyl)sulfonyl](2-methylpropyl)amino]-1S-(phenylmethyl)propyl]-, 3-(6-aminopyridyl)methyl ester;
Carbamic acid, [2R-hydroxy-3-[[(4-methoxyphenyl)sulfonyl] (2-methylpropyl)amino]-1S-(phenylmethyl)propyl]-, 3-(6-hydroxypyridyl)methyl ester;
Carbamic acid, [2R-hydroxy-3-[[(4-hydroxyphenyl)sulfonyl] (2-methylpropyl)amino]-1S-(phenylmethyl)propyl]-, 5-pyrimidylmethyl ester; or
Benzamide, N-[2R-hydroxy-3-[[(4-methoxyphenyl)sulfonyl] (2-methylpropyl)amino]-1S-(phenylmethyl)propyl]-2-methyl.

8. A pharmaceutical composition comprising a compound according to any of claims 1-7 and a pharmaceutically acceptable carrier.

9. Use of a compound to any of claims 1-7, including those compounds of the formula 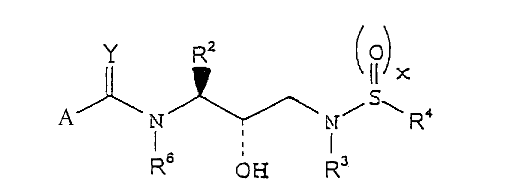 wherein
R³ and R⁶ are both hydrogen
R² is cyclohexylmethyl
x is 2
Y is oxygen
R⁴ is C₁-C₁₂-alkyl, alkylamino, alkylaminoalkyl, heteroaryl, heterocycloalkyl, C₃-C₁₀-cydoalkyl, aryl, C₂-C₆-alkenyl, C₂-C₆-alkynyl,
A is alkoxy, aralkoxy, aryloxy, cycloalkylalkoxy,
for preparing a medicament for inhibiting a retroviral protease.

10. Use of a composition of claim 8 for preparing a medicament for treating a retroviral infection.

11. Use of a compound according to any of claims 1-7 including those compounds of the formula 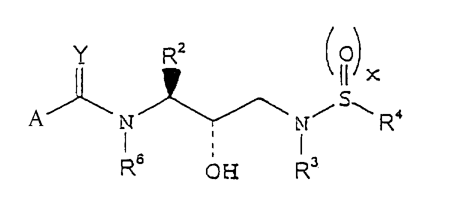 wherein
R³ and R⁶ are both hydrogen
R² is cyclohexylmethyl
x is 2
Y is oxygen
R⁴ is C₁-C₁₂-alkyl, alkylamino, alkylaminoalkyl, heteroaryl, heterocycloalkyl, C₃-C₁₀-cydoalkyl, aryl, C₂-C₆-alkenyl, C₂-C₆-alkynyl,
A is alkoxy, aralkoxy, aryloxy, cycloalkylalkoxy,
for preparing a medicament for preventing relication of a retrovirus.

## Patentansprüche

1. Verbindung, dargestellt durch die Formel: 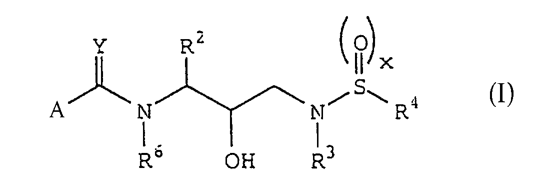 oder ein pharmazeutisch akzeptables Salz, Prodrug oder Ester hiervon, wobei
R² ist ein Alkyl-, Aryl-, Cycloalkyl-, Cycloalkylalkyl- oder Aralkylradikal, welches Radikal optional durch ein Radikal ersetzt wird, das aus der Gruppe bestehend aus Alkyl, Halo, Nitro, Cyano, CF₃, -OR⁹ und SR⁹ ausgewählt wird, wobei R⁹ ist ein Radikal ausgewählt aus der Gruppe bestehend aus Wasserstoff und Alkyl;
R³ ist ein Wasserstoff-, Alkyl-, Haloalkyl-, Alkenyl-, Alkynyl-, Hydroxyalkyl-, Alkoxyalkyl-, Alkylthioalkyl-, Alkylsulfonylalkyl-, Cycloalkyl-, Cykloalkylalkyl- Heterocykloalkyl-, Heteroaryl-, Heterocykloalkylalkyl-, Aryl-, Aralkyl-, Heteroaralkyl-, Aminoalkyl- oder mono- oder disubstituiertes Aminoalkylradikal, wobei die Substituenten ausgewählt werden aus der Gruppe bestehend aus Alkyl-, Aryl-, Aralkyl-, Cycloalkyl-, Cykloalkylalkyl-, Heteroaryl-, Heteroaralkyl-, Heterocycloalkyl- und Heterocycloalkylalkylradikalen; oder wobei das Aminoalkylradikal disubstituiert wird und die Substituenten gemeinsam mit dem Stickstoffatom, an das sie gebunden sind, ein Heterocycloalkyl- oder ein Heteroarylradikal bilden;
R⁴ ist ein Alkyl-, Haloalkyl-, Alkenyl-, Alkynyl-, Hydroxyalkyl-, Alkoxyalkyl-, Cycloalkyl-, Cykloalkylalkyl-Heterocykloalkyl-, Heteroaryl-, Heterocykloalkylalkyl-, Aryl-, Aralkyl-, Aralkenyl-, Heteroaralkyl-, Aminoalkyl- oder mono- oder disubstituiertes Aminoalkylradikal, wobei die Substituenten ausgewählt werden aus der Gruppe bestehend aus Alkyl-, Aryl-, Aralkyl-, Cycloalkyl-, Cykloalkylalkyl-, Heteroaryl-, Heteroaralkyl-, Heterocycloalkyl- und Heterocycloalkylalkylradikalen; oder wobei das Aminoalkylradikal disubstituiert wird und die Substituenten gemeinsam mit dem Stickstoffatom, an das sie gebunden sind, ein Heterocycloalkyl- oder ein Heteroarylradikal bilden;
R⁶ ist ein Wasserstoff- oder Alkylradikal;
x ist 1 oder 2;
Y ist O oder S; und
A ist ein Alkoxy-, Alkenoxy-, Aralkoxy-, Alkyl-, Cycloalkyl-, Cycloalkylalkoxy-, Cycloalkylalkyl-, Aralkyl-, Aryl-, Aryloxy-, Heterocycloalkyl-, Heterocyckloalkoxy-, Heterocycloalkylalkyl-, Heterocycloalkylalkoxy-, Heteroaralkyl-, Heteroaralkoxy-, Heteroaryloxy-, Heteroaryl-, Alkenyl-, Aryloxyalkyl-, Heteroaryloxyalkyl-, Hydroxyalkyl-, Amino- oder mono- oder disubstituiertes Aminoradikal, wobei die Substituenten ausgewählt werden aus der Gruppe bestehend aus Alkyl-, Aryl-, Aralkyl-, Cycloalkyl-, Cykloalkylalkyl-, Heteroaryl-, Heteroaralkyl-, Heterocycloalkyl- und Heterocycloalkylalkylradikalen; oder wobei das Aminoradikal disubstituiert wird und die Substituenten gemeinsam mit dem Stickstoffatom, an das sie gebunden sind, ein Heterocycloalkyl, oder ein Heteroarylradikal bilden,
ausgenommen die Verbindungen der Formel 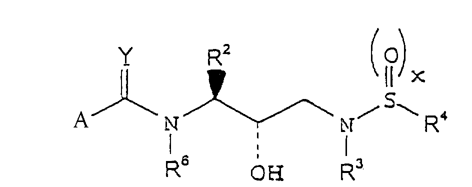 wobei
R³ und R⁶ beide Wasserstoff sind
R² ist Cyclohexylmethyl
x ist 2
Y ist Sauerstoff
R⁴ ist C₁-C₁₂-Alkyl, Alkylamino, Alkylaminoalkyl, Heteroaryl, Heterocycloalkyl, C₃-C₁₀-Cycloalkyl, Aryl, C₂-C₆Alkenyl, C₂-C₆Alkynyl,
A ist Alkoxy, Aralkoxy, Aryloxy, Cykloalkylalkoxy.

2. Verbindung von Anspruch 1 oder ein pharmazeutisch akzeptables Salz, Prodrug oder Ester hiervon, wobei
R² ist ein Alkyl-, Aryl-, Cycloalkyl-, Cycloalkylalkyl- oder Aralkylradikal, welches Radikal optional durch ein Radikal ersetzt wird, das aus der Gruppe bestehend aus Alkyl, Halo und -OR⁹ ausgewählt wird, wobei R⁹ ist ein Radikal ausgewählt aus der Gruppe bestehend aus Wasserstoff und Alkyl;
R³ ist ein Wasserstoff-, Alkyl-, Haloalkyl-, Alkenyl-, Alkynyl-, Hydroxyalkyl-, Alkoxyalkyl-, Alkylthioalkyl-, Alkylsulfonylalkyl-, Cycloalkyl-, Cykloalkylalkyl-, Heterocykloalkyl-, Heteroaryl-, Heterocykloalkylalkyl-, Aryl-, Aralkyl-, Heteroaralkyl-, Aminoalkyl- oder mono- oder disubstituiertes Aminoalkylradikal, wobei die Substituenten ausgewählt werden aus der Gruppe bestehend aus Alkyl-, Aralkyl-, Cycloalkyl- und Cykloalkylalkylradikalen; oder wobei das Aminoalkylradikal disubstituiert wird und die Substituenten gemeinsam mit dem Stickstoffatom, an das sie gebunden sind, ein Heterocycloalkyl- oder ein Heteroarylradikal bilden;
R⁴ ist ein Alkyl-, Haloalkyl-, Alkenyl-, Alkynyl-, Hydroxyalkyl-, Alkoxyalkyl-, Cycloalkyl-, Cykloalkylalkyl-Heterocykloalkyl-, Heteroaryl-, Heterocykloalkylalkyl-, Aryl-, Aralkyl-, Aralkenyl- oder Heteroaralkylradikal;
R⁶ ist ein Wasserstoff- oder Alkylradikal;
x ist 1 oder 2;
und
Y ist O oder S; und
A ist ein Alkoxy-, Alkenoxy-, Aralkoxy-, Alkyl-, Cycloalkyl-, Cycloalkylalkoxy-, Cycloalkylalkyl-, Aralkyl-, Aryl-, Aryloxy-, Heterocycloalkyl-, Heterocycloalkoxy-, Heterocycloalkylalkyl-, Heterocycloalkylalkoxy-, Heteroaralkyl-, Heteroaralkoxy-, Heteroaryloxy-, Heteroaryl-, Hydroxyalkyl-, Amino- oder mono- oder disubstituiertes Aminoradikal, wobei die Substituenten ausgewählt werden aus der Gruppe bestehend aus Alkyl-, Aralkyl-, Heteroaryl-, Heteroaralkyl-, Heterocycloalkyl- und Heterocycloalkylalkylradikalen; oder wobei das Aminoradikal disubstituiert wird und die Substituenten gemeinsam mit dem Stickstoffatom, an das sie gebunden sind, ein Heterocycloalkyl- bilden.

3. Verbindung von Anspruch 2 oder ein pharmazeutisch akzeptables Salz, Prodrug oder Ester hiervon, wobei
R² ist ein Alkyl-, Aryl-, Cycloalkyl-, Cycloalkylalkyl- oder Aralkylradikal, welches Radikal optional durch ein Radikal substituiert wird, das aus der Gruppe bestehend aus Alkyl, Halo und -OR⁹ ausgewählt wird, wobei R⁹ ist ein Radikal ausgewählt aus der Gruppe bestehend aus Wasserstoff und Alkyl;
R³ ist ein Wasserstoff-, Alkyl-, Haloalkyl-, Alkenyl-, Alkynyl-, Hydroxyalkyl-, Alkoxyalkyl-, Alkylthioalkyl-, Alkylsulfonylalkyl-, Cycloalkyl-, Cykloalkylalkyl- Heterocykloalkyl-, Heteroaryl-, Heterocykloalkylalkyl-, Aryl-, Aralkyl-, Heteroaralkyl-, Aminoalkyl- oder mono- oder dialkylsubstituiertes Aminoalkylradikal;
R⁴ ist ein Alkyl-, Haloalkyl-, Alkenyl-, Alkynyl-, Hydroxyalkyl-, Alkoxyalkyl-, Cycloalkyl-, Cykloalkylalkyl-Heterocykloalkyl-, Heteroaryl-, Heterocykloalkylalkyl-, Aryl-, Aralkyl-, Aralkenyl- oder Heteroaralkylradikal;
R⁶ ist ein Wasserstoff- oder Alkylradikal;
x ist 1 oder 2;
und
Y ist O oder S; und
A ist ein Alkoxy-, Alkenoxy-, Aralkoxy-, Alkyl-, Cycloalkyl-, Aryl-, Heterocycloalkyl-, Heterocycloalkoxy-, Heterocycloalkylalkyl-, Heteroaralkoxy-, Heteroaryl-, Amino- oder mono- oder disubstituiertes Aminoradikal, wobei die Substituenten ausgewählt werden aus der Gruppe bestehend aus Alkyl- und Aralkylradikalen.

4. Verbindung von Anspruch 3 oder ein pharmazeutisch akzeptables Salz, Prodrug oder Ester hiervon, wobei
R² ist ein Alkyl-, Cycloalkylalkyl- oder Aralkylradikal, welches Radikal optional durch ein Radikal ersetzt wird, das aus der Gruppe bestehend aus Alkyl, Halo und -OR⁹ ausgewählt wird, wobei R⁹ ist ein Radikal ausgewählt aus der Gruppe bestehend aus Wasserstoff und Alkyl;
R³ ist ein Wasserstoff-, Alkyl-, Alkenyl-, Alkynyl-, Hydroxyalkyl-, Alkoxyalkyl-, Alkylthioalkyl-, Alkylsulfonylalkyl-, Cycloalkyl-, Cykloalkylalkyl- Heterocykloalkylalkyl-, Aryl-, Aralkyl-, Heteroaralkyl-, Aminoalkyl- oder mono- oder dialkylsubstituiertes Aminoalkylradikal;
R⁴ ist ein Alkyl-, Haloalkyl-, Alkenyl-, Alkynyl-, Hydroxyalkyl-, Alkoxyalkyl-, Cycloalkyl-, Cykloalkylalkyl-Heterocykloalkyl-, Heteroaryl-, Heterocykloalkylalkyl-, Aryl-, Aralkyl-, Aralkenyl- oder Heteroaralkylradikal;
R⁶ ist ein Wasserstoff- oder Alkylradikal;
x ist 1 oder 2;
und
Y ist O oder S; und
A ist ein Alkoxy-, Alkenoxy-, Aralkoxy-, Alkyl-, Cycloalkyl-, Aryl-, Heterocycloalkyl-, Heterocycloalkoxy-, Heterocycloalkylalkyl-, Heteroaralkoxy-, Heteroaryl-, Amino- oder mono- oder disubstituiertes Aminoradikal, wobei die Substituenten ausgewählt werden aus der Gruppe bestehend aus Alkyl- und Aralkylradikalen.
unter der Bedingung, daß Alkyl alleine oder in Kombination ein geradkettiges oder verzweigtkettiges Kohlenwasserstoffradikal mit ein bis acht Kohlenstoffatomen ist; Alkenyl alleine oder in Kombination ein geradkettiges oder verzweigtkettiges Kohlenwasserstoflradikal mit zumindest einer Doppelbindung und mit zwei bis acht Kohlenstoffatomen ist; Alkynyl alleine oder in Kombination ein geradkettiges oder verzweigtkettiges Kohlenwasserstoffradikal mit zumindest einer Dreifachbindung und mit zwei bis zehn Kohlenstoffatomen ist; und Cycloalkyl alleine oder in Kombination ein Kohlenwasserstoffring mit drei bis acht Kohlenstoffatomen ist.

5. Verbindung von Anspruch 4 oder ein pharmazeutisch akzeptables Salz, Prodrug oder Ester hiervon, wobei
R² ist ein Alkyl-, Cycloalkylalkyl- oder Aralkylradikal, welches Radikal optional durch ein Radikal ersetzt wird, das aus der Gruppe bestehend aus Alkyl, Halo und -OR⁹ ausgewählt wird, wobei R⁹ ist ein Radikal ausgewählt aus der Gruppe bestehend aus Wasserstoff und Alkyl;
R³ ist ein Wasserstoff-, Alkyl-, Alkenyl-, Alkynyl-, Hydroxyalkyl-, Alkoxyalkyl-, Alkylthioalkyl-, Alkylsulfonylalkyl-, Cycloalkyl-, Cykloalkylalkyl- Heterocykloalkylalkyl-, Aryl-, Aralkyl-, Heteroaralkyl-, Aminoalkyl- oder mono- oder dialkylsubstituiertes Aminoalkylradikal;
R⁴ ist ein Alkyl-, Haloalkyl-, Alkenyl-, Alkynyl-, Hydroxyalkyl-, Alkoxyalkyl-, Cycloalkyl-, Cykloalkylalkyl-Heterocykloalkyl-, Heteroaryl-, Heterocykloalkylalkyl-, Aryl-, Aralkyl-, Aralkenyl- oder Heteroaralkylradikal;
R⁶ ist ein Wasserstoff- oder Alkylradikal;
x ist 1 oder 2;
und
Y ist O oder S; und
A ist ein Alkoxy-, Alkenoxy-, Aralkoxy-, Alkyl-, Cycloalkyl-, Aryl-, Heterocycloalkyl-, Heterocycloalkoxy-, Heterocycloalkylalkyl-, Heteroaralkoxy-, Heteroaryl-, Amino- oder mono- oder disubstituiertes Aminoradikal, wobei die Substituenten ausgewählt werden aus der Gruppe bestehend aus Alkyl- und Aralkylradikalen.
unter der Bedingung, daß Alkyl alleine oder in Kombination ein geradkettiges oder verzweigtkettiges Kohlenwasserstoffradikal mit ein bis fünf Kohlenstoffatomen ist; Alkenyl alleine oder in Kombination ein geradkettiges oder verzweigtkettiges Kohlenwasserstoffradikal mit zumindest einer Doppelbindung und mit zwei bis fünf Kohlenstoffatomen ist; Alkynyl alleine oder in Kombination ein geradkettiges oder verzweigtkettiges Kohlenwasserstoffradikal mit zumindest einer Dreifachbindung und mit zwei bis fünf Kohlenstoffatomen ist; und Cycloalkyl alleine oder in Kombination ein Kohlenwasserstoffring mit drei bis acht Kohlenstoffatomen ist.

6. Die Verbindung von Anspruch 5 oder ein pharmazeutisch akzeptables Salz, Prodrug oder Ester hiervon, wobei
R² ist Butyl, Cyclohexylmethyl, Benzyl, 4-Fluorobenzyl oder Naphthylmethyl;
R³ ist Methyl, Ethyl, Propyl, Butyl, Pentyl, Hexyl, Isobutyl, Iso-Amyl, 3-Methoxypropyl, 3-Methylthiopropyl, 4-Methylthiobutyl, 4-Methylsulfonylbutyl, 2-Dimethylaminoethyl, 2-(1-Morpholino)ethyl, 4-Hydroxybutyl, Allyl, Propargyl, Cyclohexylmethyl, Cyclopropylmethyl, Phenyl, Benzyl, 4-Fluorobenzyl, 4-Methoxybenzyl, 1-Phenylethyl, 2-Phenylethyl, Naphthylmethyl, 3-Pyridylmethyl oder 4-Pyridylmethyl;
R⁴ ist Methyl, Ethyl, Propyl, Butyl, Ethenyl, Chloromethyl, Cyclopropyl, Cyclobutyl, Cyclopentyl, Cyclohexyl, Phenyl, Naphthyl, Chlorophenyl, Fluorophenyl, Hydroxyphenyl, Methylphenyl, Methoxyphenyl, Ethoxyphenyl, Methylthiophenyl, Methylsulfoxyphenyl, Methylsulfonylphenyl, Acetamidophenyl, Methoxycarbonylphenyl, Dimethylaminophenyl, Nitrophenyl, Trifluoromethylphenyl, Benzyl, 2-Phenylethenyl oder Thienyl;
R⁶ ist Wasserstoff;
x ist 2; und
Y ist O; und
A ist Methyl, Cyclohexyl, Cyclopentyl, Cycloheptyl, 1,2,3,4-Tetrahydronaphthyl, Naphthyl, Chinolinyl, Indolyl, Pyridyl, Methylpyridyl, Furanyl, Thiophenyl, Oxazolyl, Thiazolyl, Phenyl, Methylphenyl, Ethylphenyl, Dimethylphenyl, Iso-Propylphenyl, Chlorophenyl, Hydroxyphenyl, Methoxyphenyl, Methylsulfonylphenyl, Methylsulfonylmethylphenyl, Carboxyphenyl, Aminocarbonylphenyl, Methylhydroxyphenyl, Methylnitrophenyl, Methylaminophenyl, Methyl-N,N-Dimethylaminophenyl, t-Butoxy, Benzyloxy, Pyridylmethoxy, 3-Propenoxy, Hydroxypyridylmethoxy, Aminopyridylmethoxy, Pyrimidinylmethoxy, N-oxo-pyrimidinylmethoxy, Thiazolylmethoxy, Tetrahydrothiophenoxy, 1,1-Dioxotetrahydrothiophenoxy, Tetrahydrofuranoxy, Methylamino, Benzylamino oder Isopropylamino.

7. Verbindung von Anspruch 1, welche ist:
Phenylmethyl [2R-Hydroxy-3-[(3-Methylbutyl) (Methylsulfonyl)amino]-1S-(Phenylmethyl)propyl]Karbamat;
Phenylmethyl [2R-Hydroxy-3-[(3-Methylbutyl) (Phenylsulfonyl)amino]-1S-(Phenylmethyl)propyl] Karbamat;
N1-[2R-Hydroxy-3-[(3-Methylbutyl)(Phenylsulfonyl)amino]-1S-(Phenylmethyl)propyl]-2S-[(Phenylmethyloxycarbonyl)amino]butandiamid;
2S-[[(Dimethylamino)acetyl]amino]-N-[2R-Hydroxy-3-[(3-Methyl-butyl)(Phenylsulfonyl)amino]-1S-(Phenylmethyl)propyl]-3,3-Dimethylbutanamid;
2S-[[(Methylamino-)acetyl]amino]-N-[2R-Hydroxy-3-[(3-Methyl-butyl)(Phenylsulfonyl)amino]-1S-(Phenylmethyl)propyl]-3,3-Dimethylbutanamid;
N1-[2R-Hydroxy-3-[(3-Methylbutyl)(Phenyl-sulfonyl)amino]-N4-Methyl-1S-(Phenylmethyl)propyl]-2S-[(2-Chinolinylcarbonyl)amino]butandiamid;
[3-[[2-Hydroxy-3-[N-(3-Methylbutyl)-N-(Phenylsulfonyl)amino]-1-(Phenylmethyl)propyl]amino]-2-Methyl-3-Oxopropyl]-, (4-Methoxyphenyl)methylester, [1S-1R* [S*), 2S*]]-;
Karbamidsäure, [2R-Hydroxy-3-[(4-Hydroxyphenylsulfonyl) (2-Methylpropyl)amino]-1S-(Phenylmethyl)propyl-, 3(S)-1,1-Dioxotetrahydrothiophen-3-yl-ester;
Karbamidsäure, [2R-Hydroxy-3-[(4-Methoxyphenylsulfonyl) (2-Methylpropyl)amino]-1S-(Phenylmethyl)propyl-, 3-(S)-1,1-Dioxotetrahydrothiophen-3-yl-ester;
Karbamidsäure, [2R-Hydroxy-3-[(4-Methoxyphenylsulfonyl) (2-Methylpropyl)amino]-1S-(Phenylmethyl)propyl-, 3-S-Tetrahydrothiophen-3-yl-ester;
Karbamidsäure, [2R-Hydroxy-3-[(4-Hydroxyphenylsulfonyl) (2-Methylpropyl)amino]-1S-(Phenylmethyl)propyl-, 3-S-Tetrahydrothiophen-3-yl-ester;
Karbamidsäure, [2R-Hydroxy-3-[[(4-Methoxyphenyl)sulfonyl](2-Methylpropyl)amino]-1S-(Phenylmethyl)propyl]-, 3-(6-Aminopyridyl)methylester;
Karbamidsäure, [2R-Hydroxy-3-[[(4-Hydroxyphenyl)sulfonyl](2-Methylpropyl)amino]-1S-(Phenylmethyl)propyl-], 3-(6-Aminopyridyl)methylester;
Karbamidsäure, [2R-Hydroxy-3-[[(4-Methoxyphenyl)sulfonyl] (2-Methylpropyl)amino]-1S-(Phenylmethyl)propyl]-, 3-(6-Hydroxypyridyl)methylester;
Karbamidsäure, [2R-Hydroxy-3-[[(4-Hydroxyphenyl)sulfonyl] (2-Methylpropyl)amino]-1S-(Phenylmethyl)propyl]-, 5-Pyrimidylmethylester; oder
Benzamid, N-[2R-Hydroxy-3-[[(4-Methoxyphenyl)sulfonyl] (2-Methylpropyl)amino]-1S-(Phenylmethyl)propyl]-2-Methyl.

8. Pharmazeutische Zusammensetzung, die eine Verbindung gemäß einem der Ansprüche 1-7 und einen pharmazeutisch akzeptablen Träger umfaßt.

9. Nutzung einer Verbindung zu einem der Ansprüche 1-7, einschließlich der Verbindungen der Formel 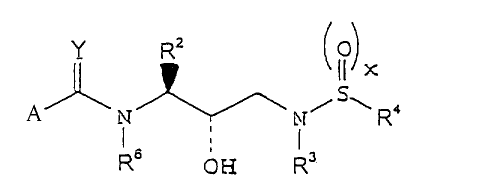 wobei
R³ und R⁶ sind beide Wasserstoff
R² ist Cyclohexylmethyl
x ist 2
Y ist Sauerstoff
R⁴ ist C₁-C₁₂-Alkyl, Alkylamino, Alkylaminoalkyl, Heteroaryl, Heterocycloalkyl, C₃-C₁₀-Cycloalkyl, Aryl, C₂-C₆Alkenyl, C₂-C₆Alkynyl,
A ist Alkoxy, Aralkoxy, Arylkoxy, Cycloalkylalkoxy,
zur Bereitung eines Medikaments zur Hemmung einer retroviralen Protease.

10. Nutzung einer Zusammensetzung nach Anspruch 8 zur Bereitung eines Medikaments zur Behandlung einer retroviralen Infektion.

11. Nutzung einer Verbindung nach Anspruch 1-7, einschließlich der Verbindungen der Formel 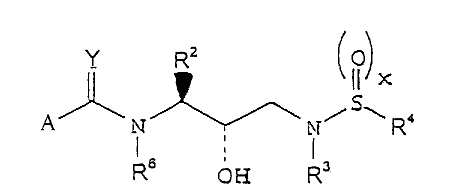 wobei
R³ und R⁶ sind beide Wasserstoff
R² ist Cyclohexylmethyl
x ist 2
Y ist Sauerstoff
R⁴ ist C₁-C₁₂-Alkyl, Alkylamino, Alkylaminoalkyl, Heteroaryl, Heterocycloalkyl, C₃-C₁₀-Cycloalkyl, Aryl, C₂-C₆-Alkenyl, C₂-C₆-Alkynyl,
A ist Alkoxy, Aralkoxy, Aryloxy, Cycloalkylalkoxy,
zur Bereitung eines Medikaments zur Verhinderung einer Umsiedelung eines Retrovirus.

## Revendications

1. Composé représenté par la formule : 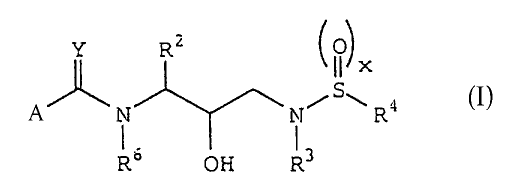 ou un sel, promédicament ou ester acceptable en pharmacie d'un tel composé, dans lequel
R² est un radical alkyle, aryle, cycloalkyle, cycloalkylalkyle ou aralkyle, lequel radical est éventuellement substitué par un radical choisi dans le groupe constitué par les radicaux alkyle, halogéno, nitro, cyano, CF₃, -OR⁹ et -SR⁹, où R⁹ est un radical choisi dans le groupe constitué par l'hydrogène et un radical alkyle ;
R³ est l'hydrogène ou un radical alkyle, halogénoalkyle, alcényle, alcynyle, hydroxyalkyle, alcoxyalkyle, alkylthioalkyle, alkylsulfonylalkyle, cycloalkyle, cycloalkylalkyle, hétérocycloalkyle, hétéroaryle, hétérocycloalkylalkyle, aryle, aralkyle, hétéroaralkyle, aminoalkyle ou aminoalkyle mono- ou di- substitué, dans lequel lesdits substituants sont choisis dans le groupe constitué par les radicaux alkyle, aryle, aralkyle, cycloalkyle, cycloalkylalkyle, hétéroaryle, hétéroaralkyle, hétérocycloalkyle et héterocycloalkylalkyle ; ou dans lequel ledit radical aminoalkyle est disubstitué, lesdits substituants, conjointement avec l'atome d'azote auquel ils sont attachés, formant un radical hétérocycloalkyle ou hétéroaryle ;
R⁴ est un radical alkyle, halogénoalkyle, alcényle, alcynyle, hydroxyalkyle, alcoxyalkyle, cycloalkyle, cycloalkylalkyle, hétérocycloalkyle, hétéroaryle, hétérocycloalkylalkyl, aryle, aralkyle, aralcényle, hétéroaralkyle, aminoalkyle ou aminoalkyle mono- ou di-substitué, dans lequel lesdits substituants sont choisis dans le groupe constitué par les radicaux alkyle, aryle, aralkyle, cycloalkyle, cycloalkylalkyle, hétéroaryle, hétéroaralkyle, hétérocycloalkyle et hétérocycloalkylalkyle ; ou dans lequel ledit radical aminoalkyle est disubstitué, lesdits substituants, conjointement avec l'atome d'azote auquel ils sont attachés, formant un radical hétérocycloalkyle ou hétéroaryle ;
R⁶ est l'hydrogène ou un radical alkyle ;
x vaut 1 ou 2 ;
Y est O ou S ; et
A est un radical alcoxy, alcénoxy, aralcoxy, alkyle, cycloalkyle, cycloalkylalcoxy, cycloalkylalkyle, aralkyle, aryle, aryloxy, hétérocycloalkyle, hétérocycloalcoxy, hétérocycloalkylalkyle, hétérocycloalkylalcoxy, hétéroaralkyle, hétéroaralcoxy, hétéroaryloxy, hétéroaryle, alcényle, aryloxyalkyle, hétéroaryloxyalkyle, hydroxyalkyle, amino, ou amino mono- ou di-substitué, dans lequel les substituants sont choisis dans le groupe constitué par les radicaux alkyle, aryle, aralkyle, cycloalkyle, cycloalkylalkyle, héréroaryle, hétéroaralkyle, hétérocycloalkyle et héterocycloalkylalkyle ; ou dans lequel ledit radical amino est disubstitué, lesdits substituants, conjointement avec l'atome d'azote auquel ils sont attachés, formant un radical hétérocycloalkyle ou hétéroaryle,
à l'exception des composés de formule 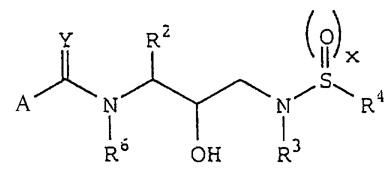 dans laquelle R³ et R⁶ sont tous deux l'hydrogène
R² est un radical cyclohexylméthyle
x vaut 2
Y est l'oxygène
R⁴ est un radical alkyle en C₁ à C₁₂, alkylamino, alkylaminoalkyle, hétéroaryle, hétérocycloalkyle, cycloalkyle en C₃ à C₁₀, aryle, alcényle en C₂ à C₆, alcynyle en C₂ à C₆,
A est un radical alcoxy, aralcoxy, aryloxy, cycloalkylalcoxy.

2. Composé selon la revendication 1 ou un sel, promédicament ou ester acceptable en pharmacie d'un tel composé, dans lequel
R² est un radical alkyle, aryle, cycloalkyle, cycloalkylalkyle ou aralkyle, lequel radical est éventuellement substitué par un radical choisi dans le groupe constitué par les radicaux alkyle, halogéno et -OR⁹ où R⁹ est un radical choisi dans le groupe constitué par l'hydrogène et un radical alkyle ;
R³ est l'hydrogène ou un radical alkyle, halogénoalkyle, alcényle, alcynyle, hydroxyalkyle, alcoxyalkyle, alkylthioalkyle, alkylsulfonylalkyle, cycloalkyle, cycloalkylalkyle, hétrocycloalkyle, hétéroaryle, hétérocycloalkylalkyle, aryle, aralkyle, hétéroaralkyle, aminoalkyle ou aminoalkyle mono- ou di-substitué, dans lequel lesdits substituants sont choisis dans le groupe constitué par les radicaux alkyle, aralkyle, cycloalkyle et cycloalkylalkyle ; ou dans lequel ledit radical aminoalkyle est disubstitué, lesdits substituants, conjointement avec l'atome d'azote auquel ils sont attachés, forment un radical hétérocycloalkyle ou hétéroaryle ;
R⁴ est un radical alkyle, halogénoalkyle, alcényle, alcynyle, hydroxyalkyle, alcoxyalkyle, cycloalkyle, cycloalkylalkyle, hétérocycloalkyle, hétéroaryle, hétérocycloalkylalkyle, aryle, aralkyle, aralcényle ou hétéroaralkyle ;
R⁶ est l'hydrogène ou un radical alkyle ;
x vaut 1 ou 2 ;
et
Y est O ou S ; et
A est un radical alcoxy, alcénoxy, aralcoxy, alkyle, cycloalkyle, cycloalkylalcoxy, cycloalkylalkyle, aralkyle, aryle, aryloxy, hétérocycloalkyle, hétérocycloalcoxy, hétérocycloalkylalkyle, hétérocycloalkylalcoxy, hétéroaralkyle, hétéroaralcoxy, hétéroaryloxy, hétéroaryle, hydroxyalkyle, amino, ou amino mono- ou di-substitué, dans lequel les substituants sont choisis dans le groupe constitué par les radicaux alkyle, aralkyle, hétéroaryle, hétéroaralkyle, hétérocycloalkyle et hétérocycloalkylalkyle ; ou dans lequel ledit radical amino est disubstitué, lesdits substituants, conjointement avec l'atome d'azote auquel ils sont attachés, formant un radical hétérocycloalkyle.

3. Composé selon la revendication 2 ou un sel, promédicament ou ester acceptable en pharmacie d'un tel composé, dans lequel
R² est un radical alkyle, aryle, cycloalkyle, cycloalkylalkyle ou aralkyle, lequel radical est éventuellement substitué par un radical choisi dans le groupe constitué par les radicaux alkyle, halogéno et -OR⁹, où R⁹ est un radical choisi dans le groupe constitué par l'hydrogène et un radical alkyle ;
R³ est l'hydrogène ou un radical alkyle, halogénoalkyle, alcényle, alcynyle, hydroalkyle, alcoxyalkyle, alkylthioalkyle, alkylsulfonylalkyle, cycloalkyle, cycloalkylalkyle, hétérocycloalkyle, hétéroaryle, hétérocycloalkylalkyle, aryle, aralkyle, hétéroaralkyle, aminoalkyle ou aminoalkyle à substitution mono- ou di-alkyle ;
R⁴ est un radical alkyle, halogénoalkyle, alcényle, alcynyle, hydroxyalkyle, alcoxyalkyle, cycloalkyle, cycloalkylalkyle, hétérocycloalkyle, hétéroaryle, hétérocycloalkylalkyle, aryle, aralkyle, aralcényle ou hétéroaralkyle ;
R⁶ est l'hydrogène ou un radical alkyle ;
x vaut 1 ou 2 ; et
Y est O ou S ; et
A est un radical alcoxy, alcénoxy, aralcoxy, alkyle, cycloalkyle, aryle, hétérocycloalkyle, hétérocycloalcoxy, hétérocycloalkylalkyle, hétéroaralcoxy, hétéroaryle, amino, ou amino mono- ou di-substitué, dans lequel les substituants sont choisis dans le groupe constitué par les radicaux alkyle et aralkyle.

4. Composé selon la revendication 3 ou un sel, promédicament ou ester acceptable en pharmacie d'un tel composé, dans lequel
R² est un radical alkyle, cycloalkylalkyle ou aralkyle, lequel radical est éventuellement substitué par un radical choisi dans le groupe constitué par les radicaux alkyle, halogéno et -OR⁹, où R⁹ est un radical choisi dans le groupe constitué par l'hydrogène et un radical alkyle ;
R³ est l'hydrogène ou un radical alkyle, alcényle, alcynyle, hydroxyalkyle, alcoxyalkyle, alkylthioalkyle, alkylsulfonylalkyle, cycloalkyle, cycloalkylalkyle, hétérocycloalkylalkyle, aryle, aralkyle, hétéroaralkyle, aminoalkyle ou aminoalkyle à substitution mono- ou di-alkyle ;
R⁴ est un radical alkyle, halogénoalkyle, alcényle, alcynyle, hydroxyalkyle, alcoxyalkyle, cycloalkyle, cycloalkylalkyle, hétérocycloalkyle, hétéroaryle, hétérocycloalkylalkyle, aryle, aralkyle, aralcényle ou hétéroaralkyle ;
R⁶ est l'hydrogène ou un radical alkyle ;
x vaut 1 ou 2 ;
et
Y vaut O ou S ; et
A est un radical alcoxy, alcénoxy, aralcoxy, alkyle, cycloalkyle, aryle, hétérocycloalkyle, hétérocycloalcoxy, hétérocycloalkylalkyle, hétéroaralcoxy, hétéroaryle, amino, ou amine mono- ou di- substitué, dans lequel les substituants sont choisis dans le groupe constitué par les radicaux alkyle et aralkyle ;
avec pour conditions que le radical alkyle, seul ou en combinaison, est un radical hydrocarboné à chaîne droite ou à chaîne ramifiée contenant de 1 à 8 atomes de carbone ; le radical alcényle, seul ou en combinaison, est un radical hydrocarboné à chaîne droite ou à chaîne ramifiée ayant au moins une double liaison et contenant de 2 à 8 atomes de carbone ; le radical alcynyle, seul ou en combinaison, est un radical hydrocarboné à chaîne droite ou à chaîne ramifiée ayant au moins une triple liaison et contenant de 2 à 10 atomes de carbone ; et le radical cycloalkyle, seul ou en combinaison, est un cycle hydrocarboné contenant de 3 à 8 atomes de carbone.

5. Composé selon la revendication 4 ou un sel, promédicament ou ester acceptable en pharmacie d'un tel composé, dans lequel
R² est un radical alkyle, cycloalkyle ou aralkyle, lequel radical est éventuellement substitué par un radical choisi dans le groupe constitué par les radicaux alkyle, halogéno et -OR⁹, où R⁹ est un radical choisi dans le groupe constitué par l'hydrogène en un radical alkyle ;
R³ est l'hydrogène ou un radical alkyle, alcényle, alcynyle, hydroxyalkyle, alcoxyalkyle, alkylthioalkyle, alkylsulfonylalkyle, cycloalkyle, cycloalkylalkyle, hétérocycloalkylalkyle, aryle, aralkyle, hétéroaralkyle, aminoalkyle ou aminoalkyle à substitution mono- ou di-alkyle ;
R⁴ est un radical alkyle, halogénoalkyle, alcényle, alcynyle, hydroxyalkyle, alcoxyalkyle, cycloalkyle, cycloalkylalkyle, hétérocycloalkyle, hétéroaryle, hétérocycloalkylalkyle, aryle, aralkyle, aralcényle ou hétéroaralkyle ;
R⁶ est l'hydrogène ou un radical alkyle ;
x vaut 1 ou 2 ;
et
Y est O ou S ; et
A est un radical alcoxy, alcénoxy, aralcoxy, alkyle, cycloalkyle, aryle, hétérocycloalkyle, hétérocycloalcoxy, hétérocycloalkylalkyle, hétéroaralcoxy, hétéroaryle, amino, ou amino mono- ou disubstitué, dans lequel les substituants sont choisis dans le groupe constitué par les radicaux alkyle et aralkyle ;
avec pour conditions que le radical alkyle, seul ou en combinaison, est un radical hydrocarboné à chaîne droite ou à chaîne ramifiée contenant de 1 à 5 atomes de carbone ; le radical alcényle, seul ou en combinaison, est un radical hydrocarboné à chaîne droite ou à chaîne ramifiée ayant au moins une double liaison et contenant de 2 à 5 atomes de carbone ; le radical alcynyle, seul ou en combinaison, est un radical hydrocarboné à chaîne droite ou à chaîne ramifiée ayant au moins une triple liaison et contenant de 2 à 5 atomes de carbone ; et le radical cycloalkyle, seul ou en combinaison, est un cycle hydrocarboné contenant de 3 à 8 atomes de carbone.

6. Composé selon la revendication 5 ou un sel, promédicament ou ester acceptable en pharmacie d'un tel composé, dans lequel
R² est un radical butyle, cyclohexylméthyle, benzyle, 4-fluorobenzyle ou naphtylméthyle ;
R³ est un radical méthyle, éthyle, propyle, butyle, pentyle, hexyle, isobutyle, isoamyle, 3-méthoxypropyle, 3-méthylthiopropyle, 4-méthylthiobutyle, 4-méthyl-sulfonylbutyle, 2-diméthylamimoéthyle, 2-(1-morpholino)-éthyle, 4-hydroxybutyle, allyle, propargyle, cyclohexylméthyle, cyclopropylméthyle, phényle, benzyle, 4-fluorobenzyle, 4-méthoxybenzyle, 1-phényléthyle, 2-phényléthyle, naphthylméthyle, 3-pyridylméthyle ou 4-pyridylméthyle ;
R⁴ est un radical méthyle, éthyle, propyle, butyle, éthényle, chlorométhyle, cyclopropyle, cyclobutyle, cyclopentyle, cyclohexyle, phényle, naphtyle, chlorophényle, fluorophényle, hydroxyphényle, méthylphényle, méthoxyphényle, éthoxyphényle, méthylthiophényle, méthylsulfoxyphényle, méthylsulfonylphényle, acétamidophényle, méthoxycarbonylphényle, diméthylamimophényle, nitrophényle, trifluorométhylphényle, benzyle, 2-phényléthényle ou thiényle ;
R⁶ est l'hydrogène ;
x vaut 2 ; et
Y est O ; et
A est un radical méthyle, cyclohexyle, cyclopentyle, cycloheptyle, 1,2,3,4-tétrahydronaphtyle, naphtyle, quinolinyle, indolyle, pyridyle, méthylpyridyle, furanyle, thiophényle, oxazolyle, thiazolyle, phényle, méthylphényle, éthylphényle, diméthylphényle, isopropylphényle, chlorophényle, hydroxyphényle, méthoxyphényle, méthylsulfonylphényle, méthylsulfonylméthylphényle, carboxyphényle, aminocarbonylphényle, méthylhydroxyphényle, méthylnitrophényle, méthylaminophényle, méthyl-N,N-diméthylaminophényle, t-butoxy, benzyloxy, pyridylméthoxy, 3-propénoxy, hydroxypyridylméthoxy, aminopyridylméthoxy, pyrimidinylméthoxy, N-oxo-pyrimidinylméthoxy, thiazolylméthoxy, tétrahydrothiophénoxy, 1,1-dioxotétrahydrothiophénoxy, tétrahydrofuranoxy, méthylamino, benzylamimo ou isopropylamino.

7. Composé selon la revendication 1, qui est :
le [2R-hydroxy-3-[(3-méthylbutyl)-(méthylsulfonyl)amino]-1S-(phénylméthyl)propyl]carbamate de phénylméthyle ;
le [2R-hydroxy-3-[(3-méthylbutyl)-(phénylsulfonyl)amino]-1S-(phénylméthyl)propyl]carbamate de phénylméthyle ;
le N1-[2R-hydroxy-3-[(3-méthylbutyl)-(phénylsulfonyl)amino]-1S-(phénylméthyl)propyl]-2S-[(phénylméthyloxycarbonyl)amino]butanediamide ;
le 2S-[[(diméthylamino)acétyl]amino]-N-(2R-hydroxy-3-[(3-méthylbutyl)-(phénylsulfonyl)amino]-1S-(phénylméthyl)propyl]-3,3-diméthylbutanamide ;
le 2S-[[(méthylamino)acétyl]amino]-N-(2R-hydroxy-3-[(3-méthylbutyl)-(phénylsulfonyl)amino]-1S-(phénylméthyl)propyl]-3,3-diméthylbutanamide ;
le N1-[2R-hydroxy-3-[(3-méthylbutyl)-(phénylsulfonyl)amino]-N4-méthyl-1S-(phénylméthyl)propyl]-2S-[(2-quinolinylcarbonyl)amino]butanediamide ;
l'isomère [1S-[1R*(S*),2S*]] de l'ester (4-méthoxyphényl)méthylique de 3-[[2-hydroxy-3-[N-(3-méthylbutyl)-N-(phénylsulfonyl)amino]-1-(phénylméthyl)propyl]amino]-2-méthyl-3-oxopropyle ;
l'ester 3(S)-1,1-dioxotétrahydrothiophène-3-ylique de l'acide [2R-hydroxy-3-[(4-hydroxyphénylsulfonyl)-(2-méthylpropyl)amino]-1S-(phénylméthyl)propyl-carbamique ;
l'ester 3(S)-1,1-dioxotétrahydrothiophène-3-ylique de l'acide [2R-hydroxy-3-[(4-méthoxyphénylsulfonyl)-(2-méthylpropyl)amino]-1S-(phénylméthyl) propyl-carbamique ;
l'ester 3-S-tétrahydrothiophène-3-ylique de l'acide [2R-hydroxy-3-[(4-méthoxyphénlylsulfonyl)-(2-méthylpropyl)amino]-1S-(phénylméthyl)propyl-carbamique ;
l'ester 3-S-tétrahydrothiophène-3-ylique de l'acide [2R-hydroxy-3-[(4-hydroxyphénylsulfonyl)-(2-méthylpropyl)amino]-1S-(phénylméthyl)propyl-carbamique ;
l'ester 3-(6-aminopyridyl)méthylique de l'acide [2R-hydroxy-3-[[(4-méthoxyphényl)sulfonyl]-(2-méthylpropyl)-amino]-1S-(phénylméthyl)propyl-carbamique ;
l'ester 3-(6-aminopyridyl)méthylique de l'acide [2R-hydroxy-3-[[(4-hydroxyphényl)sulfonyl]-(2-méthylpropyl)-amino]-1S-(phénylméthyl)propyl-carbamique ;
l'ester 3-(6-hydroxypyridyl)méthylique de l'acide [2R-hydroxy-3-[[(4-méthoxyphényl)sulfonyl]-(2-méthylpropyl)amino]-1S-(phénylméthyl)propyl-carbamique ;
l'ester 5-pyrimidylméthylique de l'acide [2R-hydroxy-3-[[(4-hydroxyphényl)sulfonyl]-(2-méthylpropyl)amino]-1S-phénylméthyl)propyl-carbamique ; ou
le N-[2R-hydroxy-3-[[(4-méthoxyphényl)sulfonyl]-(2-méthylpropyl)amino]-1S-(phénylméthyl)propyl-2-méthyl-benzamide.

8. Composition pharmaceutique comprenant un composé selon l'une quelconque des revendications 1 à 7 et un support acceptable en pharmacie.

9. Utilisation d'un composé selon l'une quelconque des revendications 1 à 7, y compris des composés de formule : 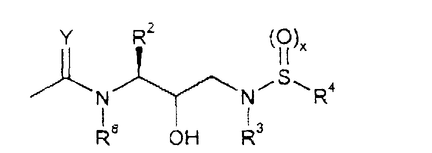 dans laquelle
R³ et R⁶ sont tous deux l'hydrogène
R² est un radical cyclohexylméthyle
x vaut 2
Y est l'oxygène
R⁴ est un radical alkyle en C₁ à C₁₂, alkylamino, alkylaminoalkyle, hétéroaryle, hétérocycloalkyle, cycloalkyle en C3 à C₁₀, aryle, alcényle en C₂ à C₆, alcynyle en C₂ à C₆,
A est un radical alcoxy, aralcoxy, aryloxy, cycloalkylalcoxy,
pour préparer un médicament destiné à inhiber une protéase rétrovirale.

10. Utilisation d'une composition selon la revendication 8, pour préparer un médicament destiné au traitement d'une infection rétrovirale.

11. Utilisation d'un composé selon l'une quelconque des revendications 1 à 7, y compris des composés de formule : 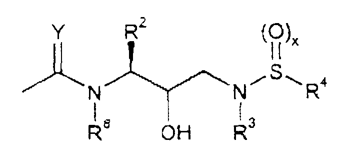 dans laquelle
R³ et R⁶ sont tous deux l'hydrogène
R² est un radical cyclohexylméthyle
x vaut 2
Y est l'oxygène
R⁴ est un radical alkyle en C₁ à C₁₂, alkylamino, alkylaminoalkyle, hétéroaryle, hétérocycloalkyle, cycloalkyle en C₃ à C₁₀, aryle, alcényle en C₂ à C₆, alcynyle en C₂ à C₆,
A est un radical alcoxy, aralcoxy, aryloxy, cycloalkylalcoxy,
pour préparer un médicament destiné à prévenir la réplication d'un rétrovirus.
